(19) Europäisches Patentamt
European Patent Office
Office européen des brevets

(11) EP 0 839 186 B1

(12) EUROPEAN PATENT SPECIFICATION

(45) Date of publication and mention of the grant of the patent:
**10.11.2004 Bulletin 2004/46**

(21) Application number: **96923878.1**

(22) Date of filing: **12.07.1996**

(51) Int Cl.[7]: **C12N 9/20**, C12N 9/18, C11D 3/386

(86) International application number:
**PCT/DK1996/000322**

(87) International publication number:
**WO 1997/004079 (06.02.1997 Gazette 1997/07)**

# (54) A MODIFIED ENZYME WITH LIPOLYTIC ACTIVITY

MODIFIZIERTES ENZYM MIT LIPOLYTISCHER AKTIVITÄT

ENZYME MODIFIEE A ACTIVITE LIPOLYTIQUE

(84) Designated Contracting States:
**AT BE CH DE DK ES FI FR GB GR IE IT LI NL PT SE**

(30) Priority:
**14.07.1995 DK 83295**
**13.09.1995 DK 101395**
**29.09.1995 DK 109695**
**21.11.1995 DK 130695**
**01.04.1996 DK 37296**
**07.05.1996 US 20461 P**

(43) Date of publication of application:
**06.05.1998 Bulletin 1998/19**

(73) Proprietor: **Novozymes A/S**
**2880 Bagsvaerd (DK)**

(72) Inventors:
- **FUGLSANG, Claus, Crone**
  **DK-2880 Bagsvaerd (DK)**
- **OKKELS, Jens, Sigurd**
  **DK-2880 Bagsvaerd (DK)**
- **PETERSEN, Dorte, Aaby**
  **DK-2880 Bagsvaerd (DK)**
- **PATKAR, Shamkant, Anant**
  **DK-2880 Bagsvaerd (DK)**
- **THELLERSEN, Marianne**
  **DK-2880 Bagsvaerd (DK)**
- **VIND, Jesper**
  **Novo Allé DK-2880 Bagsvaerd (DK)**
- **HALKIER, Torben**
  **Novo Allé DK-2880 Bagsvaerd (DK)**
- **JÖRGENSEN, Steen, Troels**
  **DK-2880 Bagsvaerd (DK)**

(56) References cited:
**WO-A-92/05249**
**WO-A-93/01285**

- **DIALOG INFORMATION SERVICES, File 351, WPIL, Dialog Accession No. 009892827, WPI Accession No. 94-172743/21, ASAHI KASEI KOGYO KK, "Modified Lipase Comprising Hydrophobic Peptide at N-Terminal - has Increased Activity and is Useful to Hydrolyse Lipid Into Glycerine and Fatty Acid"; & JP,A,06 113 845 (26-04-94), 9421 (Basic).**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 839 186 B1

## Description

## FIELD OF THE INVENTION

The present invention relates to a modified enzyme with lipolytic activity, a DNA sequence encoding said modified enzyme, a vector comprising said DNA sequence, a host cell harbouring said DNA sequence or said vector, and a process for producing said modified enzyme with lipolytic activity.

Further the invention relates to a method for applying a peptide addition to a parent enzyme with lipolytic activity, a composition comprising the modified enzyme with lipolytic activity of the invention, the advantageous use of the modified enzyme of the invention in detergent compositions, and further a method for improving the washing performance of detergent compositions.

## BACKGROUND OF THE INVENTION

Detergent enzymes have been marketed for more than 20 years and are today well established as normal detergent ingredients in both powder and liquid detergent all over the world.

Detergent compositions may comprise many different enzymes, of which proteases, amylases, cellulases, lipases, cutinases are the most important today.

### Lipolytic enzymes

Lipolytic enzymes (*i.e.* enzymes classified under the Enzyme Classification number E.C. 3.1.1 (Carboxylic Ester Hydrolases) in accordance with the Recommendations (1992) of the International Union of Biochemistry and Molecular Biology (IUBMB)) are enzymes which can be used for removing lipid or fatty stains from clothes and other textiles.

Various microbial lipases have been suggested as detergent enzymes. Examples of such lipases include a *Humicola lanuginosa* lipase, *e.g.* described in EP 258 068 and EP 305 216, *a Rhizomucor miehei* lipase, *e.g.* as described in EP 238 023, *Absidia* sp. lipolytic enzymes (WO 96/13578), a *Candida* lipase, such as a *C. anrarctica* lipase, *e.g.* the *C. aruarctica* lipase A or B described in EP 214 761, a *Pseudomonac* lipase such as a *P. alcaligenes* and *P. pseudoalcaligenes* lipase, *e.g.* as described in EP 218 272, a *P. cepacia* lipase, *e.g.* as described in EP 331 376, a *Pseudomonas* sp. lipase as disclosed in WO95/14783, a *Bacillus* lipase, *e.g.* a *B.* subtilis lipase (Dartois et al., (1993) biochemica et Biophysica acta 1131, 253-260), a *B. stearothermophilus* lipase (JP 64/744992) and a B. *pumilus* lipase (WO 91/16422).

Furthermore, a number of cloned lipases have been described, including the *Penicillium camembertii* lipase described by Yamaguchi et al., (1991), Gene 103, 61-67), the *Geotricum candidum* lipase (Schimada, Y. et al., (1989), J. Biochem., 106, 383-388), and various *Rhizopus* lipases such as a *R. delemar* lipase (Hass, M.J et al., (1991), Gene 109, 117-113) a *R. niveus* lipase (Kugimiya et al., (1992), Biosci. Biotech. Biochem. 56, 716-719) and a *R. oryzae* lipase.

Other types of lipolytic enzymes having been suggested as detergent enzymes include cutinases, *e.g.* derived from *Pseudomonas mendocina* as described in WO 88/09367, or a cutinase derived from *Fusarium solani pisi* (*e.g*, described in WO 90/09446).

In recent years attempts have been made to prepare modified lipolytic enzymes, such as variants and mutants having improved properties for detergent purposes.

In most cases lipolytic enzymes with improved washing performance have been constructed by site-directed mutagenesis resulting in substitution of specific amino acid residues which have been chosen either on the basis of their type or on the basis of their location in the secondary or tertiary structure of the mature enzyme.

An alternative general approach for modifying proteins and enzymes have been based on random mutagenesis, for instance, as disclosed in US 4,894,331, WO 93/01285, and WO 95/22615.

### Comments to prior art

It is known from prior art to modify lipolytic enzymes by site-directed mutagenesis to obtain an improved performance, inparticular washing performance of lipolytic enzymes. The generally used concept has been to insert, delete or substitute amino acids within the structural part of the amino acid chain of the parent lipolytic enzyme in question. Lipolytic enzymes with a significantly improved washing performance have been achieved this way.

However, there is a need for providing lipolytic enzymes with an even further improved performance, such as washing performance and/or even further improved dishwashing properties than the lipolytic enzymes prepared by these prior art methods.

## SUMMARY OF THE INVENTION

Thus, the object of the present invention is to improve properties of enzymes with lipolytic activity, in particular to improve the washing performance of such enzymes.

It has surprisingly been found that it is possible to significantly enhance the washing performance of a lipolytic enzyme by applying a peptide addition to the N- and/or C-terminal of the enzyme.

Consequently, in a first aspect the invention relates to a modified enzyme with lipolytic activity which as compared to its parent enzyme has one or more peptide additions in its C-terminal and/or N-terminal end. In the present context the term "peptide addition" is intended to indicate that a stretch of one or more consecutive amino acid residues has been added to either or both of the N- and/or C-terminal end(s) of the parent enzyme or inserted within the non-structural part of the N-and/or C-terminal end(s) of the parent enzyme. The term "non-strucural part" is intended to indicate the part of the N- and C-terminal end, respectively, which is outside the first or last, respectively, structural element, such as an α-helix or β-sheet structure, of the folded mature enzyme. The non-structural part may easily be identified in a three-dimensional structure or model of the enzyme in question. Typically, the non-structural part comprises the first or the last about 1-20 amino acid residues of the amino acid sequence constituting the enzyme.

The term "mature enzyme" is used in its conventional meaning, i.e. to indicate the active form of the enzyme resulting after expression and posttranslational processing (to remove pro and/or pre-sequences) by the producer organism in question. When the enzyme is a secreted enzyme, the mature enzyme will normally be the form of the enzyme resulting after secretion. More specifically this means that the pre- and pro-peptide sequences, if present, have been removed from the initially translated enzyme, *i.e.* the unprocessed enzyme.

The term "parent" enzyme in intended to indicate the enzyme to be modified according to the invention. The parent enzyme may be a naturally-occuring (or wild type) enzyme or may be a variant thereof prepared by any suitable means. For instance, the parent enzyme may be a variant of a naturally-occurring enzyme which has been modified by substitution, deletion or truncation of one or more amino acid residues or by addition or insertion of one or more amino acid residues to the amino acid sequence of a naturally-occurring enzyme, typically in the structural part of the enzyme.

In other aspects the invention relates to a DNA sequence encoding a modified lipolytic enzyme as defined above, a recombinant vector or transformation vehicle comprising a DNA sequence of the invention, a host cell harbouring a DNA sequence of the invention or a vector of the invention, and a process for preparing a modified lipolytic enzyme by cultivation of said host cell.

The modified lipolytic enzyme of the invention may conveniently be used as a detergent enzyme and accordingly, in final aspects the invention relates to a detergent additive or a detergent composition comprising a modified lipolytic enzyme of the invention.

## BRIEF DESCRIPTION OF THE DRAWING

Figure 1 shows the nucleotide and amino acid sequence of the coding region of the *Humicola Lanuginosa* lipase gene as present in the yeast expression vector pJSO37. The signal sequence (amino acids 1 to 17) is the original signal sequence from *Humicola lanuginosa.* The SPIRR peptide addition is located at amino acid residue 18 to 22. Amino acid residue 23 (E) is the first amino acid residue of the parent lipase expressed in *Aspergillus oryzae.*
Figure 2 shows the nucleotide and amino acid sequence of the coding region of the *Humicola lanuginosa* lipase gene as present in the *E. coli* expression vector pJSO215. The signal sequence (amino acids 1 to 20) is *the A. lyticus* protease I signal (WO 96/17943). The SPIRR peptide is added after amino acid residue 20. Amino acid residue 26 (E) is the first amino acid residue of the parent lipase expressed in *Aspergillus oryzae.*
Figure 3 shows the nucleotide and amino acid sequence of the coding region of the *Humicola lanuginosa* lipase gene as present in the *E. coli* expression vector pSX581. The signal sequence (amino acids 1 to 20) is the *A. lyticus* protease I signal sequence (WO 96117943). Amino acid residue 21 (E) is the first amino acid residue of the parent lipase.
Figure 4 the construction of pSX164;
Figure 5 the construction of pSX578;
Figure 6 the construction of pSX581;
Figure 7 shows the plasmid pSX581;
Figure 8 shows the plasmid pJSO37;
Figure 9 shows the construction of *Aspergillus* vector pCaHj485

## DETAILED DESCRIPTION OF THE INVENTION

### Peptide addition

As stated above it has surprisingly, been found that a significantly improved wash performance of lipolytic enzymes may be achieved when an appropriate peptide addition is applied to a non-structural part of the enzyme m its mature form or at the C-terrninal and/or N-terminal end of the mature enzyme.

The term "improved wash performance" is intended to indicate that the modified enzyme of the invention has a better lipid soil removing capability than the unmodified parent enzyme when tested under wash like conditions. The improvement is often indicated in terms of "an improvement factor" ($f_{improve)}$ (further reference vide the Materials and Methods section further below). Dependent on the peptide addition and the mature enzyme an improvement factor ($f_{improve)}$ ) in the range of 1-5 , or even up to 10 (such as in the range of 1-10) has been obtained. It is presently believed that even higher improvement factors such as up to 20, even up 30, or even up to 50, such as between 30 and 50, or even higher may be achieved in accordance with the present invention.

The term "an appropriate peptide addition" is used to indicate that the peptide addition to be used is one which is capable of effecting an improved wash performance. The "appropriateness" of the peptide addition may be checked by a comparative analysis of the wash performance of a modified enzyme to which the peptide addition has been applied and of the corresponding parent enzyme, respectively. The wash performance may, e.g., be determined by any suitable technique such as any of the wash performance assays described in the present application.

It is presently contemplated that the improved wash performance effected by the peptide addition is, at least in part, due to an increased affinity of the modified lipolytic enzyme towards its lipid substrate (although this may not be the only reason).

The present invention is not limited to improving the wash performance of a parent lipolytic enzyme. It is contemplated that also other properties of parent lipolytic enzymes may be improved in accordance with the present invention, i.e. by applying an appropriate peptide addition at or within a non-structural part of the C-terminal and/or N-terminal end of the parent enzyme. More specifically, it is contemplated that the activity of a parent lipolytic enzyme, e.g., in removing pitch in the paper and pulp industry, in degreasing hides in the leather industry, in acting as a catalyst in organic syntheses, etc., may be significantly improved by applying an appropriate peptide addition at or within the N-terminal or C-terminal end of a lipolytic enzyme, i.e. a peptide addition which is capable of exerting the desired function. Also in these connections it is believed that the improved activity may be at least partly due to an improved affinity for the substrate in question.

As a consequence of the improved activity it may be possible to reduce the dosage of the enzyme required for a given purpose considerably, as compared to the dosage of needed dosage of the unmodified parent enzyme.

In the context of the present invention the term "modified enzyme" is intended to indicate a derivative or a variant of a parent enzyme, which derivative or variant as compared to the parent enzyme comprises a peptide addition at the C-terminal and/or N-terminal end (fused to the first and/or last amino acid residue of the parent enzyme) and/or within the non-structural part of the C- and/or N-teminal end of the parent enzyme. Thus, for instance, the modified enzyme may comprise a peptide addition at either the N-terminal or the C-terminal end or both in the N- and the C-terminal ends of the parent lipolytic enzyme.

It is presently believed that the capability of the peptide addition of providing the desired effect (such as improved wash performance, improved performance in degreasing of hides, etc, depends on, e.g., the identity of the parent enzyme to be modified, the structure (including length) of the peptide addition, the impact of the peptide addition on the structure of the entire lipolytic enzyme, the nature or functionality of amino acid residues of the peptide addition, etc. A prerequisite for the peptide addition being capable of providing the desired effect is, of course, that the modified enzyme containing the peptide addition is expressible in a suitable host organism. The following general considerations may be of relevance for the design of a suitable peptide addition:

Length of peptide addition: It has been found that peptide additions containing varying numbers of amino acid residues are capable of providing the desired effect and thus, it is not possible to specify an exact number of amino acid residues to be present in the peptide addition to be used in accordance with the present invention. It is contemplated that the upper limit of the number of amino acid residues is determined, inter alia, on the impact of the peptide addition on the expression, the structure and/or the activity of the resulting modified enzyme. It is believed that the peptide addition may comprise a substantial number of amino acid residues, however, without all of these amino acid residues need to contributing to the desired effect (even if the peptide addition contains a substantial number of amino acid residues only a small number of these need to providing the desired function, this small number may be termed the functional part of the peptide addition). The main consideration in relation to the lower limit of the number of amino acid residues of the peptide addition will normally be that the number should be sufficient to provide the desired effect.

The peptide addition may thus comprise a single amino acid residue or an amino acid chain of from 2 and 500 amino acids, such as from 1 to 200, or from 2 to to 100, preferably from 2 to 50, such as 3 to 50, even more

preferably from 7-45 and still more preferably between 1 and 15, such as between 1 and 10 or 1 and 7, especially between 4 and 10, such as 4 and 7 amino acids.

Stability: The peptide addition should preferably be chosen so as to provide a modified lipolytic enzyme with an acceptable stability (e.g. structural stability and/or expression stability) or so as to not significantly reduce the structural stability of the parent enzyme. Although many peptide additions are not believed to confer any substantial structural instability to the resulting modified enzyme, it may in certain instances and with certain parent enzymes be relevant to choose a peptide addition which in itself can confer a structural stability to the modified lipolytic enzyme. For instance, a peptide addition which in itself forms a structural element, such as an $\alpha$-helix or a $\beta$-sheet, may stabilize the resulting modified enzyme and thus be used in the context of the present invention. Peptide sequences capable of forming such structures are known in the art. Alternatively, an improved structural stability may be provided by introduction of cystein bridges in the modified lipolytic enzyme of the invention. For instance; a cystein bridge between the peptide addition and the mature part of the enzyme may be established if at least one of the amino acid residues of the peptide addition is a cystein residue which is located so as to be able to form a covalent binding to a cystein residue in the mature part of the enzyme. The positive effect of introducing a cystein bridge is illustrated in Example 19. If no suitable cystein is present in the mature enzyme, a cystein may be inserted at a suitable location of said parent enzyme, conveniently by replacing an amino acid of the parent enzyme, which is considered unimportant for the activity.

In addition, it may be desirable that at least one of the amino acid residues of the peptide addition is chosen so as to make the peptide addition less susceptibility to proteolytic degradation by proteolytic enzymes of the host cell used for expressing the modified lipolytic enzyme. For instance, the peptide addition may comprise at least one, and preferably at least two proline residues. Preferably, the peptide addition comprises 1-5, such as 1-4 or 1-3 or two or one proline residues. The proline residue(s) is(are) preferably placed at the proteolytic cleavage site or close thereto. Alternatively, the peptide addition may be one which provides a protease stable loop to the modified lipase, e.g. as described in EP 407 225 or WO 93/11254.

Nature of amino acid residues of the peptide addition: As stated above and without being limited to any theory, it is presently believed that the improved performance may at least partly be due to an increased affinity of the modified lipolytic enzyme toward the substrate provided by the peptide addition. In particular in relation to wash performance, it is believed that favourable electrostatic interactions may be obtained between the negatively charged lipid surface and positively charged and/or hydrophobic amino acid residues present in the modified enzyme. Accordingly, it is particularly preferred that the modified enzyme of the invention comprises a peptide addition with at least one positive charge, such as at least 2, 3, 4 or more positive charges or expressed differently, in which a substantial number of the amino acid residues of the peptide addition is positively charged and/or hydrophobic.

Analogously, and in order to reduce the negative charge in a non-structural end of the parent enzyme it is preferred to remove at least one such as two or more negatively charged amino acid residues from a non-structural N-terminal or C-terminal part of the parent enzyme of choice, in particular from the part of the parent lipase being constructed of the 1-5 first or last N-terminal or C-terminal amino acid residues, such as 1-4, or 1-3 or 1-2. The negatively charged amino acid residue may either be removed or replaced by a neutral, a positively charged or a hydrophobic amino acid residue. For instance, the negatively charged amino acid residue to be removed may be an E or D which may be replaced with either of the positively charged amino acid residues R, K or H, the neutral amino acid residues S, T, G or Q, or the hydrophobic amino acid residues A, I, W F or L. Similarly, a neutral amino acid residue of a non-structural N-terminal or C-terminal part of the parent enzyme may be replaced with a positively charged or hydrophobic amino acid residue as defined above.

Accordingly, the modified lipolytic enzyme of the invention in addition or as an alternative to a N-terminal and/or C-terminal extension may comprise a mutation in the non-structural C-terminal and/or N-terminal end of the parent enzyme, which mutation has involved deleting or replacing a negatively charged amino acid residue of said non-structural part with a positively charged or neutral amino acid residue or with a hydrophobic amino acid residue.

If a peptide addition is present in both the N- and the C-terminal of the parent enzyme, the peptide addition at or within each of the terminals may have the same or a different amino acid sequence.

Test of suitability of peptide addition: the effect of using a given peptide addition, e.g., designed on the basis of the above principles may be tested by constructing a modified lipolytic enzyme containing the peptide addition and testing the properties of the resulting enzyme for the desired enzyme application such as wash, pitch removal, degreasing of leather, etc either in a full scale test or in an assay whih correlates well with the enzyme application in question.

The peptide addition can be generalised in the following way.

The first residue (counted from the outer residue) is named "a", the second is named "b", the third "c" etc. Thus, in case of an N-terminal addition the first amino acid residue is termed "a", in case of a C-terminal addition the last amino acid residue is termed "a".

In an important embodiment of the invention the peptide addition consists of from 4 to 7 amino acids. Such peptide addition, which can be applied to both the N- and/or C-terminal of the parent enzyme, can be referred to as:

a-b-c-d (four amino acids peptide addition)
a-b-c-d-e (five amino acids peptide addition)
a-b-c-d-e-f (six amino acids peptide addition)
a-b-c-d-e-f-g (seven amino acids peptide addition)

Each letter defines an amino acid residue.
a, b, c, d, e, f and g may independently be any amino acid including Alanine (A), Valine (V), Leucine (L), Isoleucine (1), Proline (P), Phenylalanine (F), Tryptophan (W), Methionine (M), Glycine (G), Serine (S), Threonine (T), Cysteine (C), Tyrosine (Y), Asparagine (N), Glutamine (Q), Aspartic acid (D), Glutamic acid (E), Lysine (K), Arginine (R), and Histidine (H).

In specific embodiments a, b, c, d, e, f, and g are independently one of the following amino acids:

a: Leu, Ile, Val, Trp, Phe, Ser, Arg, Cys, or Lys,
b: Leu, Ile, Val, Trp, Phe Ser, Pro, Arg, Lys, Cys or His,
c: Leu, Ile, Val, Trp, Phe, Ser, Pro, Arg, Cys, or Lys.
d: Leu, Ile, Val, Trp, Phe Ser, Pro, Arg, Cys, or Lys.
e: Leu, Ile, Val, Trp, Phe, Pro, Arg, Lys, Ala, Glu, Cys, or Asp,
f: Leu, Ile, Val, Trp, Phe, Pro, Arg, Lys, Ala, Glu, Cys, or Asp,
g: Leu, Ile, Val, Trp, Phe, Pro, Arg, Lys, Cys, or Met.

In a preferred embodiment at least one such as one, two, three or four of a, b, c, d, e, f, or g is a positively charged amino acid, *i.e.* Arg(R) or Lys (K) or a hydrophobic amino acid, *i.e.* Leu, Ile, Val, Trp or Phe.

As stated further above, and dependent on the host cell of choice it is generally believed that it is important that the peptide addition comprises at least one proline residue in order to protect the modified lipolytic enzyme against proteolytic degradation during the processing of the enzyme by the host cell of choice. It may be desirable that the proline residue occupies position two (i.e. b) and/or three (i.e. c) of the peptide addition or a position close to the desired cleavage point (i.e. the point where processing by the host cell in question is believed to occur). Accordingly, in one embodiment b and optionally c of the peptide addition is Pro

In another embodiment of the invention a-b is SP (Ser-Pro), A-P or Q-P. If the peptide addition contains more amino acid residues, e.g. between 4 and 7 amino acids the peptide addition has the general formula SPcd, SPcde, SPcdef, SPcdefg or APcd, APcde, APcdef, Apcdefg or QPcd, QPcde, QPcdef, QPcdefg. In each of these formulae c, d, e , f, and g may be any amino acid. However, preferred are the above mentioned group of amino acids.

In another embodiment a-b comprise at least one positive amino acids (*i.e.* Arg and Lys) or hydrophobic amino acid residue (*i.e.* Leu, Ile, Val, Trp and Phe).

Specifically, the peptide addition applied to the parent lipolytic enzyme may advantageously be one of the following amino acid residues or peptides:

Arg-Pro-Val-Ser-Gln-Asp (RPVSQD)

Ser-Pro-Ile-Arg-Met (SPIRM), or

Ser-Pro-Ile-Arg-Ala-Arg (SPIRAR), or

Ser-Pro-Ile-Arg-Pro-Arg (SPIRPR) or

Ser-Pro-Ile-Arg-Glu-Arg (SPIRER), or

Ser-Pro-Ile-Arg-Lys (SPIRK), or

Ser-Pro-Ile-Lys-Lys (SPIKK), or

Ser-Pro-Ile-Arg-Arg-Pro (SPIRRP), or

Ser-Pro-Pro-Arg-Arg (SPPRR), or

Ser-Pro-Iso-Pro-Arg (SPIPR), or

Ser-Pro-Arg-Pro-Arg (SPRPR), or

Ser-Pro-Ile-Arg (SPIR), or

Ser-Pro-Ile-Arg-Arg (SPIRR), or

Ser-Cys-ile-Arg-Arg, (SCIRR), or

Ser-Pro-Ile-Arg-Pro-Arg-Pro (SPIRPRP), or

Ser-Cys-Ile-Arg-Pro-Arg-Pro (SCPIRPRP), or

Ser-Pro-Arg-Arg-Pro-Arg-Thr (SPRRPRT), or

Ser-Pro-Phe-Arg-Pro-Lys-Leu (SPFRPKL), or

Ser-Pro-Pro-Arg-Arg-Pro (SPPRRP), or

Ser-Pro-Ile-Arg-Arg-Glu (SPIRRE), or

Ser-Pro-Pro-Arg-Pro-Pro (SPPRPP), or

Ser-Pro-Pro-Arg-Pro-Arg (SPPRPR), or

Ser-Pro-Pro-Trp-Trp-Pro (SPPWWP), or

Ser-Pro-Pro-Trp-Arg-Pro (SPPWRP), or

Ser-Pro-Pro-Arg-Trp-Pro (SPPRWP), or

Ser-Pro-Pro-Arg-Trp-Pro (SPPRWP), or

Ser-His-Trp-Arg-Arg-Trp (SHWRRW), or

Ser-His-Trp-Arg-Lys (SHWRK), or

Ser-His-Trp-Arg-Arg (SHWRR), or

Thr-Ala-Ile-Arg-Pro-Arg-Lys (TAIRPRK),

Ser-Thr-Arg-Arg-Pro-Arg-Pro (STRRPRP),

Gly-Pro-Ile-Arg-Pro-Arg-Pro (GPIRPRP), or

Leu-Pro-Phe-Arg-Glu-Arg-Pro (LPFRQRP), or

Ser-Arg-Ser-Arg-His-Asp-Ala (SRSRHNA), or

Ile-Pro-Ile-Arg-Pro-Arg-Arg (IPIRPRR), or

Ser-Thr-Arg-Arg-Pro-Arg-Pro (STRRPRP), or

Thr-Ala-Ile-Arg-Pro-Arg-Lys (TAIRPRK), or

Trp-Arg-Trp-Arg-Trp-Arg (WRWRWR), or

Glu-Pro-Ile-Arg-Arg (QPIRR), or

Ser-His-Trp-Glu-Glu (SHWQQ), or

Ser- Ala-Leu-Arg-Pro-Arg-Lys (SALRPRK).

Also contemplated according to the invention is additions comprising more than 7 amino acids, such as from 8 to 15 amino acids.

Such peptides can be generalised as:

a-b-c-d-e-f-g-h (8 amino acid peptide)
a-b-c-d-e-f-g-h-i (9 amino acid peptide)
a-b-c-d-e-f-g-h-i-j (10 amino acid peptide)
a-b-c-d-e-f-g-h-i-j-k (11 amino acid peptide)
a-b-c-d-e-f-g-h-i-j-k-l (12 amino acid peptide)
a-b-c-d-e-f-g-h-i-j-k-l-m-(13 amino acid peptide)
a-b-c-d-e-f-g-h-i-j-k-l-m-n(14 amino acid peptide)
a-b-c-d-e-f-g-h-i-j-k-l-m-n-o (15 amino acid peptide).

a to o may be any of the twenty amino acids mentioned above.

The a-g stretch may be as defined above in relation to a peptide addition comprising 1 to 7 amino acid residues.

h, i, j, k, l, m, n, o may as mentioned above be any amino acid, preferably any of the following amino acids: Arg, Lys, Ala, Val, Trp, Ile, Phe, Ser or Pro.

Specific examples of such additions are listed below:

**Arg-Pro-Arg-Pro-Arg-Pro-Arg-Pro (RPRPRPRP), or**
**Ser-Ser-Thr-Arg-Arg-Ala-Ser-Pro-Ile-Lys-Lys (SSTRRASPIKK), or**
**Ala-Trp-Trp-Pro-Ser-Pro-Ile-Arg-Pro-Arg-Pro (AWWPSPIRPRP), or**
**Ala-Pro-Pro-Pro-Arg-Pro-Arg-Pro-Arg-Pro-Arg-Pro (APPPRPRPRPRP), or**
**Ala-Pro-Pro-Pro-Arg-Thr-Arg-Pro-Arg-Pro-Arg-Ser (APPPRTRPRPRS), or**
**Ser-Pro-Lys-Arg-Lys-Pro-Arg-Pro (SPKRKPRP), or**
**Ser-Gln-Arg-Ile-Lys-Gln-Arg-Ile-Lys (SQRIKQRIK), or**
**Ser-Pro-Pro-Pro-Arg-Pro-Arg-Pro (SPPPRPRP), or**
**Ser-Pro-Ile-Arg-Pro-Arg-Pro-Arg-Pro-Arg SPIRPRPRPR, or**
**Ser-Pro-Ile-Arg-Lys-Ala-Trp-Trp-Pro (SPIRKAWWP), or**
**Ala-Pro-Pro-Pro-Lys-Ala-Ser-Pro-Arg-Gln-Arg-Pro (APPPKASPRQRP), or**
**Ser-Pro-Ile-Arg-Pro-Arg-Pro-Ser-Pro-Ile-Arg-Pro-Arg-Pro-Arg (SPIRPRPSPIRPRP), or**
**Ser-Pro-Pro-Arg-Trp-Pro-Arg-Arg (SPPRWPRR), or**
**Ser-Pro-Pro-Arg-Trp-Pro-Arg-Trp (SPPRWPRW), or**
**Ser-Pro-Pro-Arg-Trp-Pro-Trp-Arg (SPPRWPWR), or**
**Ser-Pro-Pro-Trp-Arg-Pro-Arg-Arg (SPPWRPRR), or**
**Ser-Pro-Pro-Trp-Trp-Pro-Arg-Trp (SPPWWPRW, or**

Ser-Pro-Pro-Trp-Trp-Pro-Trp-Arg (SPPWWPWR), or
Ser-Pro-Pro-Trp-Trp-Pro-Trp-Trp (SPPWWPWW), or
Ser-Pro-Pro-Trp-Pro-Arg-pro-Arg-Pro (SPPWPRPRP), or
Ala-Pro-Pro-Pro-Arg-Pro-Arg-Leu-Leu-Pro-Ile-Ser (APPPRPRLLPIS), or
Ala-Pro-Pro-Pro-Thr-Arg-Gln-Arg-Gln-Ser-Pro (APPPTRQRQSP), or
Ala-Pro-Pro-Pro-Arg-Thr-Ile-Pro-Arg-Ser-Ser-Pro (APPPRTIPRSSP).

In any of the above specified peptide additions (whether comprising 1 to 7 or 1 to 15 amino acid residues) in which the position "a" is a Ser, Ala, Arg, Lys or Pro, the Ser may be replaced with an Ala, Arg, Lys or Pro, the Ala with a Ser, Arg, Lys or Pro and the Arg, Lys or Pro with a Ala or Ser.

It is to be emphasised that the above peptide addition may be at either the N-terminal and/or the C-terminal. Examples of modified lipolytic enzymes with both a N- and a C-terminal peptide addition include all combinations of the peptide additions specifically mentioned above. Two specific examples of such are the N-terminal addition SPIRPRP together with the C-terminal addition RRP or RR.

If the peptide addition is inserted into the non-structural part of the parent enzyme, it may replace one or more of the amino acid residues of said non-structural part. For instance, the peptide addition may replace one or more amino acid residues occupying the first, e.g. 1-5, amino acid residues of the N-terminal end and/or the last, e.g. 1-5, amino acids of the enzyme (i.e. the 1-5 amino acid residues of the C-terminal end). For instance, the peptide addition may replace amino acid residue(s) 1 and/or 2 and/or 3 and/or 4, and/or 5, etc. from either end of the parent enzyme. When the parent enzyme is *H. lanuginosa* lipase it has been of particular interest to combine any of the above peptide additions (applied in the N-terminal) with a deletion of the parent first (1E).

In accordance with the invention, it is also contemplated to apply, to the modified enzyme, one or more charged amino acids which permit effective purification of the modified enzyme. Techniques for doing this is well known by a person skilled in the art of molecular biology.

Methods of applying a peptide addition to a parent lipolytic enzyme

Although a modified enzyme of the invention may be obtained by adding (fusing or inserting) a synthetically produced peptide addition into the parent lipolytic enzyme in question, it is presently preferred that the modified enzyme of the invention is prepared by i) modifying the nucleotide, preferably DNA, sequence encoding the parent enzyme so as to encode the desired peptide addition applied to the N- and/or the C-terminal end(s) of the parent enzyme (e.g. by inserting a nucleic acid (preferably DNA) sequence encoding the peptide addition at the relevant location in the nucleic acid (preferably DNA) sequence encoding the parent enzyme), ii) expressing the resulting modified nucleic acid (preferably DNA) sequence in a suitable expression system, and iii) recovering the resulting modified enzyme.

In the present context, the term "applied to" is intended to indicate that the addition is fused to the N- and/or C-terminal end (e.g. to the first or last amino acid residue) of the mature enzyme or inserted into a non-structural part of the N-terminal and/or C-terminal end of the mature enzyme.

Many enzymes are expressed as "prepro-enzymes", i.e, as enzymes consisting of the mature enzyme, a secretory signal peptide (*i.e.* prepeptide) and a pro-peptide. The prepro-enzyme is processed intracellularly to be secreted into the fermentation medium, from which the mature enzyme can be isolated and/or purified. The peptide addition to the parent enzyme can be carried out by applying nucleic acid sequences encoding the desirable peptide additions upstream (for N-terminal peptide additions) and/or downstream (for C-terminal peptide additions) to the DNA sequence encoding the parent enzyme.

The insertion should be performed in such a way that the desired modified enzyme (i.e. having the desired peptide addition(s)) is expressed and secreted by the host cell after transcription, translation, and processing of the enzyme. The term "processing" means in this context removal of pre- and pro-peptides (except, of course, when the pro-peptide is identical to the desired peptide addition. This will be dealt with furtehr below).

Downstream sequences (encoding a C-terminal addition) can be inserted between the DNA sequence encoding the parent enzyme and the terminating codon. However, if the unprocessed DNA sequence comprises a pro-peptide encoding DNA sequence at the C-terminal end the insertion/addition of the DNA sequence encoding the peptide addition can also take place between the DNA sequences encoding the pro-peptide and the mature enzyme, respectively.

In most cases it is possible to extend the parent enzyme upstream by inserting a DNA sequence encoding the peptide addition between the DNA sequence encoding the pro-peptide or the prepeptide (if no prosequence is present) and the DNA sequence encoding the mature enzyme.

The insertion/addition of a DNA sequence encoding the peptide addition can be carried out by any standard techniques known by any skilled person in the field of molecular biology, cf., e.g. Sambrook et al., 1989). This include, e.g., the polymerase chain reaction (PCR) using specific primers, for instance described in US patent 4,683,202 or R. K. Saiki et al., (1988), Science, 239, 487-491. How to provide for the expression and secretion of adjacent DNA sequence(s) will be described below.

In connection with the present invention it has been found that some host cells may be less suited for the production of a desired modified enzyme, in that part or all of the peptide addition(s) may be cut off during the posttrarislational or other processesing performed by the host cell. Accordingly, the term "suitable expression system" is intended to indicate an expression system (host cell and optionally expression vector) which allows for at least a portion of an intact desired modified enzyme to be produced, i.e. an expression system which does not, e.g. as part of the

posttranslational or other processing by the host cell of choice, remove part or all of the peptide addition (and thereby produce the enzyme without the desired peptide addition). Typically, the expression system to be used is devoid of one or more proteolytic activities exerting the undesired posttranslational processing. The choice of expression system and thus host cell will depend on the lipolytic enzyme to be produced as will be discussed in detail further below.

While care must be exerted to select a proper expression system for producing a modified enzyme of the invention (in particular when a modified DNA sequence is used for the production), it has been found that a modified lipolytic enzyme according to the invention (having an improved wash performance) may be obtained by expressing a DNA sequence encoding the parent lipolytic enzyme in question in an expression system which is incapable of processing the translated polypeptide in the normal manner, and thereby results in the production of an enzyme which comprises a part of or the entire propeptide or a similar peptide sequence associated with the mature protein prior to its processing. In this case, the propeptide or similar peptide sequence constitutes the peptide addition. The pro-peptide or similar peptide sequence may be heterologous or homologous to the parent enzyme and can be present in both the N- and C-terminal of the parent enzyme. The production of a modified lipolytic enzyme according to the invention using this latter technique is described further below.

Accordingly, if a suitable stretch of amino acids is already encoded in the prepro form of the parent enzyme and this stretch of amino acids is cut off in the processing of the enzyme by a given expression system, the peptide addition can be applied by changing the expression host system to a system in which said processing of said stretch of amino acids does not occur. In such a case the secretory signal pre-peptide will be cut off during or after the secretion, resulting in a modified enzyme consisting of the parent enzyme comprising the pro-peptide or part thereof or a similar peptide sequence encoded by the corresponding DNA sequence, i.e. a lipolytic enzyme being extended at either its N-terminal or C-terminal end.

In other words, in a further aspect the invention relates to a method for increasing the wash performance or other activity of a parent enzyme, comprising the steps of

a) introducing a DNA sequence encoding the parent lipolytic enzyme into an expression vector,
b) introducing said DNA sequence or expression vector into a host cell incapable or inefficientl in the processing of the expressed pro-enzyme to the mature enzyme,
c) cultivating said host cell under conditions suitable for production of the enzyme comprising a part of the entire pro(pre)-sequence, and
d) recovering and optionally purifying the resulting modified enzyme.

Yeast cells have been found of particular use for applying peptide additions (in the form of the propeptide or a part thereof) to parent fungal lipolytic enzymes, in particular the *H. lanuginosa* lipase enzyme.

In an alternative and highly preferred embodiment the peptide addition is designed and applied by means of random mutagenesis according to the following principle:

a) subjecting a DNA sequence encoding the parent lipolytic enzyme with a peptide addition to localized random mutagenesis in the peptide addition or in a non-structural part of the C-terminal or N-terminal end of the parent enzyme,
b) expressing the mutated DNA sequence obtained in step a) in a host cell, and
c) screening for host cells expressing a mutated lipolytic enzyme which has an improved performance as compared to the parent lipolytic enzyme.

By this approach a number of highly advantageous peptide additions have been created. The localized random mutagenesis may be performed essentially as described in WO 95/22615. More specifically, the mutagenesis is performed under conditions in which only one or more of the above areas are subjected to mutagenesis. Especially for mutagenizing large peptide additions, it may be relevant to use PCR generated mutagenesis (e.g. as described by Deshler 1992 or Leung et al., 1989), in which one or more suitable oligonucleotide probes are used which flanks the area to be mutagenized. For mutagenesis of shorter peptide additions, it is more preferably perform the localized random mutagenesis by use of doped or spiked oligonucleotides. The doping or spiking is used, e.g., to avoid codons for unwanted amino acid residues or to increase the likelihood that a particular type of amino acid residue, such as a positively charged or hydrophobic amino acid residue, is introduced at a desired position.

Subsequent to the mutagenesis the mutated DNA is expressed by culturing a suitable host cell carrying the DNA sequence under conditions allowing expression to take place.The host cell used for this purpose may be one which has been transformed with the mutated DNA sequence, optionaly present on a vector, or one which carried the DNA sequence encoding the parent enzyme during the mutagenesis treatment. Examples of suitable host cells are given below, and is preferably a host cell which is capable of secreting the mutated enzyme (enabling an easy screening). Yeast cells, such as cells of *S. cereviciae,* have been found to be suitable host cells.

The screening criteria of step c) will have to be chosen in dependence of the desired properties of the modified lipolytic enzyme. If it is desirable to construct a modified lipolytic enzyme with an improved wash performance the screening is conveniently conducted for a reduced dependency to calcium and/or an improved tolerance towards a detergent or a detergent component. The detergent or detergent component may be any of the specific components mentioned further below in the Detergent Composition section. A preferred detergent component is a non-ionic or an anionic surfactant such as an alcohol ethoxylate or LAS, a preferred detergent is the detergent PCS described in the Materials and methods section below. Non-ionic surfactants are of particular interest for screening of *H. lanuginosa* type of lipases (e.g. fungal lipases) whereas an-ionic surfactants are of interest for screening of *Pseudomonas* type lipases.

The screening of step c) is coneveniently performed by use of a filter assay based on the following principle:

A microorganism capable of expressing the modified lipolytic enzyme of interest is incubated on a suitable medium and under suitable conditions for the enzyme to be secreted, the medium, being provided with a double filter comprising a first protein-binding filter and on top of that a second fulter exhibiting a low protein binding capability. The microorganism is located on the second filter. Subsequent to the incubation, the first filter comprising enzymes secreted from the microorganisms is separated from the second filter comprising the microorganisms. The first filter is subjected to screening for the desired enzymatic activity and the corresponding microbial colonies present on the second filter are identified.

The filter used for binding the enzymatic activity may be any protein binding filter e.g. nylon or nitrocellulose. The topfilter carrying the colonies of the expression organism may be any filter that has no or low affinity for binding proteins e.g. cellulose acetate or Durapore™. The filter may be pretreated with any of the conditions to be used for screening or may be treated during the detection of enzymatic activity.

The enzymatic activity may be detected by a dye, flourescence, precipitation, pH indicator, IR-absorbance or any other known technique for detection of enzymatic activity.

The detecting compound may be immobilized by any immobilizing agent e.g. agarose, agar, gelatine, polyacrylamide, starch, filter paper, cloth; or any combination of immobilizing agents.

Lipase activity may be detected by Brilliant green, Rhodamine B or Sudan Black in combination with a lipid e.g. olive oil or lard. The screening criteria for identifying modified lipolytic enzymes having improved washing performance may be e.g. EGTA, EDTA, non-ionic or anionic tensides, alkaline pH, or any detergent composition in combination with one of the above detectors of enzymatic activity.

It will be understood that the screening criteria used in the filter assay of the invention may be chosen so as to comply with the desired properties or uses of the enzymes to be screened. For instance, in a screening for lipolytic enzymes of particular use in the paper and pulp industry, it may be relevant to screen for an acid enzyme having an increased temperature stability. This may be performed by using a buffer with acidic pH (e.g. pH 4) and/or incubate under higher temperature before or under the assay. For detergent enzymes screening is normally conducted at alkaline pH.

Alternatively, the screening may be performed by isolating the mutated lipolytic enzyme resulting from step b) and testing the wash performance (or any other relevant property) thereof. Also, the latter "in vivo" test may be used in addition to the screening assay so as to identify the best of the mutated lipolytic enzymes selected in the screening assay. Finally, amino acid sequencing of the resulting modified lipolytic enzyme may be used to confirm the amino acid sequence of the peptide addition.

**Method of improving properties**

It is also an object of the invention to improve properties, in particular to improve the wash performance, of the parent lipolytic enzymes mentioned above. The improved properties obtained by to the method of the invention is believed to be a result of an increased affinity towards the lipid substrate.

The method of the invention comprises applying a peptide addition to the N-terminus or C-terminus of the parent enzyme in its mature form. In an embodiment of the invention this is done by applying a peptide addition to the parent enzyme by cultivating a host cell comprising a DNA sequence encoding the pre, pro or prepro-form of the parent lipolytic enzyme. Optionally the DNA sequence is present on a vector. The recovering of the resulting modified lipolytic enzyme, the host cell, cultivation conditions and/or recovery conditions are selected so that at the most a partial processing of the pre, pro or prepro-form of the parent enzyme occur resulting in that at least 5%, such as at least 10%, such as at least 15%, such as at least 20% , such as at least 25%, such as at least 50% , such as at least 75% of the produced modified enzyme molecules comprise the desired peptide addition, e.g. the entire pro-sequence or a substantial part thereof.

The host cell may be of a different origin than the parent enzyme, e.g. of another genus than the one from

which the parent enzyme is derived, or may have another posttranslational processing machinery than the source of the parent enzyme.

The parent lipolytic enzyme is preferably derived from a filamentous fungus, such as a strain of a *Humicola sp.,* in particular *H. lanuginosa,* or a bacterium, such as a strain of a *Pseudomonas sp.,* and the host cell is a yeast celll, such as a strain of *Saccharomyces* sp., in particular *Saccharomyces cerevisiae,* or a strain of *Hansenula* sp..

In an embodiment of the invention the inherent proteolytic enzyme producing capability of the host cell used for applying the peptide addition to the parent lipolytic enzyme has been reduced, e.g. by abolishing the production of one or more proteolytic enzymes by the host cell.

The method of the invention comprises the steps of:

a) subjecting a DNA sequence encoding the parent lipolytic enzyme with a peptide addition, such as a DNA sequence of the invention, to localized random mutagenesis in the part of the DNA sequence encoding the peptide addition or a non-structural part of the C-terminal or N-terminal end of the parent enzyme.
b) expressing the mutated DNA sequence obtained in step a) in a host cell, and
c) screening for host cells expressing a mutated lipolytic enzyme which has an improved performance as compared to the parent lipolytic enzyme. In an embodiment the DNA sequence is the gene or cDNA sequence encoding the parent enzyme in its pro or prepro-form.

It is also contemplated to according to the invention to introduce a mutation in the non-structural part of the C-terminus or N-terminus of the parent enzyme in its mature form, e.g. by deleting or replacing a negatively charged amino acid residue of the non-structural part with a neutral or positively charged amino acid residue or with a hydrophobic amino acid residue, or replacing a neutral amino acid residue with a positively charged amino acid residue.

**Parent lipolytic enzyme**

The modified lipolytic enzymes of the invention may be constructed from any parent lipolytic enzymes of microbial origin, preferably of bacterial or fungal (i.e. filamentous fungal or yeast) origin.

According to the invention the enzyme of the invention may be any lipolytic enzyme including lipases, phospholipases, esterases and cutinases (according to conventional terminology).

It is to be understood that lipolytic enzymes normally comprising pro- and/or pre-peptides in their unprocessed state as well as enzymes which do not are contemplated to serve as parent enzymes for the modification according to the invention.

Examples of relevant parent lipolytic enzymes include enzymes derived from the following microorganisms:

*Humicola, e. g. H. brevispora, H. lanuginosa, H. brevis var. thermoidea* and *H. insolens* (US 4,810,414) or WO 96/13580;
*Pseudomonas, e.g. Ps. fragi, Ps. stutzeri*, *Ps. cepacia* and *Ps. fluorescens* (WO 89/04361), or *Ps. plarrtarii* or *Ps. gladioli* (US patent no. 4,950,417 (Solvay enzymes)) or *Ps. alcaligenes* and *Ps. pseudoalcaligenes* (EP 218 272 or WO 94/25578) or *Ps. mendocina* (WO 88/09367; US 5,389,536);
*Fusarium*, *e.g. F. oxysporum* (EP 130,064) or *F. solani pisi* (WO 90/09446);
*Mucor* (also called *Rhizomucor), e.g. M. miehei* (EP 238 023);
*Absidia* sp. (WO 96/13578);
*Chromobacterium* especially *C. viscosum*);
*Aspergillus* (especially *A. niger*);
*Candida, e.g. C. cylindracea* (also called *C. rugosa)* or *C. antarctica* (WO 88/02775) or
*C. antarctica* lipase A or B (WO 94/01541 and WO 89/02916);
*Geotricum, e.g. G. candidum* (Schimada et al., (1989), J.Biochem., 106, 383-388);
*Penicillium, e.g. P. camembertii* (Yamaguchi et al.,(1991), Gene 103, 61-67);
*Rhizopus, e.g. R. delemar* (Hass et al., (1991), Gene 109,107-113) or *R.* niveus (Kugimiya et al., (1992) Biosci. Biotech. Biochem 56, 716-719) or *R. oryzae;* and
*Bacillus, e.g. B. subrilis* (Dartois et al., (1993) Biochemica et Biophysica acta 1131, 253-260) or *B. stearothermophilus* (JP 64/7744992) or *B. pumilus* (WO91/16422).

The generic name *Thermomyces lanuginosus* was introduced by Tsiklinsky in 1899 for one species, *Thermomyces lanuginosus.* The species is very characteristic morphologically and physiologically and there is little doubt that Tsiklinsky's *Thermomyces lanuginosus* is the same as isolated and described by later workers (such as for instance Bunce (1961) and Cooney & Emerson (1964) as *Humicola lanuginosa.*

According to The International Code of Botanical Nomenclature the correct name is the earliest legitimate

one. Thus *Thermomyces lanuginosus* should be the correct name. However, due to Tsiklinsky's incomplete description of the species, this legitimacy has been questioned by many mycologists. Thus the main reason for the taxonomical confusion relates to different views as to whether Tsiklinsky's description meets the requirements for a valid publication.

However, the name *Humicola lanuginosa* is still seen in literature, probably due to the popularity of the book of Cooney & Emerson (1964) "The Thermophilic Fungi", which recommends the use of the name *Humicola lanuginosa.*

Sequencing of the DNA encoding part of the 18S ribosomal gene from *Thermomyces lanuginosus* have been performed. The 18S sequences was compared to other 18S sequences in the GenBank database and a phylogenetic analysis using parsimony (PAUP, Version3.1.1, Smithsonian Institution, 1993) have also been made. This clearly assigns *Thermomyces lanuginosus* to the class of *Plectomycetes,* probably to the order of *Eurotiales.* According to the Entrez Browser at the NCBI (National Center for Biotechnology Information), this relates *Thermomyces lanuginosus* to families like *Eremascaceae, Monoascaceae, Pseudoeurotiaceae* and *Trichocomaceae,* the latter containing genera like *Emericella, Aspergillus, Penicillium, Eupenicilliun, Paecilomyces, Talaromyces, Thermoascus* and *Sclerocleista.* In connection with the present invention the name *Humicola lanuginosa* will be used.

In connection with the *Pseudomonas sp.* lipases it has been found that lipases from the following organisms have a high degree of homology and thus are contemplated to belong to the same family of lipases: *Ps.* ATCC21808, *Ps. aeruginosa* EF2, *Ps. aeruginosa* PAC1R, *Ps. aeruginosa* PAO1, *Ps. aeruginosa* TE3285, *Ps. sp.* 109, *Ps. pseudoalcaligenes* M1, *Ps. glumae*, *Ps. cepacia* DSM3959, *Ps. cepacia* M-12-33, *Ps. sp.* KWI-56, *Ps. purida* IFO3458, *Ps. parida* IFO12049 (Gilbert, E. J., (1993), Pseudomonas lipases: Biochemical properties and molecular cloning. Enzyme Microb. Technol., 15, 634-645).

Specific examples of readily available commercial lipases which may be modified according to the invention include Lipolase® and Lipolase®Ultra (available from Novo Nordisk A/S).

Examples of other lipolytic enzymes specifically contemplated according to the invention are Lumafast® (a *Ps. mendocina* lipase from Genencor Int. Inc); Lipomax® (a *Ps. pseudoalcaligenes* lipase from Genencor Int. Inc.); a *Fusarium solani* lipase (cutinase) from Unilever; a *Bacillus* sp. lipase from Solvay enzymes; and Liposam®, (a *Ps. mendocina* lipase from Showa Denko) and further the *Peudomonas* sp. lipase described in WO 95/06720 which have been sequenced and found to have the amino acid sequence shown in SEQ ID NO. 93.

It is to be emphasized that the parent lipolytic enzyme from which the modified enzyme of the invention can be constructed includes the above mentioned lipolytic enzymes and any variant, modification, and truncation thereof.

Examples of such parent enzymes which are specifically contemplated include the enzymes described in WO92/05249, WO 94/01541, WO 94/14951, WO 94/25577, WO 95/22615 and a protein engineered lipase variants as described in EP 407 225; a protein engineered *Ps. mendocina* lipase as described in US 5,352,594; a cutinase variant as described in WO 94/14964; a variant of an *Aspergillus* lipolytic enzyme as described in EP patent 167,309; and *Peudomonas sp.* lipase described in WO 95/06720.

In the most preferred embodiment the parent enzyme is derived from a strain of a *Humicola* sp. or or from a strain of a *Pseudomonas* sp. or a genus considered to belong to the *Pseudomonas* family.

In a specific embodiment of the invention the DNA sequence encoding the parent enzyme with lipolytic activity (to be processed into a modified enzyme of the invention) is the DNA sequence encoding the enzyme with lipolytic activity derived from the filamentous fungi *Humicola lanuginosa* described in EP 305 216. The amino acid sequence of the parent enzyme is in this case that of the secreted mature enzyme.

It is presently contemplated that the washing performance and/or thermostability of the modified enzyme of the invention may be further improved if the enzyme is glycosylated. Accordingly, in an embodiment of the invention the modified enzyme may be glycosylated. The amino acid sequence may have any degree of glycosylation.

### DNA sequence of the invention

In another aspect the invention relates to a DNA sequence encoding said modified enzyme with lipolytic activity of the invention.

The DNA sequence encoding a modified enzyme of the invention is normally prepared by modification of the DNA sequence encoding the parent lipolytic enzyme in question by any suitable method known in the art(e.g. by use of PCR as mentioned above), but may also be prepared synthetically by established standard methods, *e.g.* the phosphoamidite method described by Beaucage and Caruthers, (1981), Tetrahedron Letters 22, 1859 - 1869, or the method described by Matthes et al., (1984), EMBO Journal 3, 801-805. According to the phosphoamidite method, oligonucleotides are synthesized, *e.g.* in an automatic DNA synthesizer, purified, annealed, ligated and cloned in suitable vectors.

A DNA sequence encoding the parent lipolytic enzyme of choice may be obtained by use of conventional techniques. For instance, the DNA sequence may be isolated from a genomic or DNA library prepared from the relevant organism or may be obtained by expression cloning, e.g. as described in WO 93/11249, or may be produced synthetically.

In a currently preferred embodiment, the DNA sequence encoding the modified lipolytic enzyme of the invention is prepared from the DNA sequence encoding a lipolytic enzyme derived from *Humicola lanuginosa* and described in EP 305 216. In another preferred embodiment the DNA sequence is derived from a strain of *Pseudomonas sp.,* especially a strain of *Ps. alcaligenes* or *Ps. pseudoalcaligenes.*

An isolated nucleic acid sequence encoding a modified lipolytic enzyme of the invention may be manipulated in a variety of ways to provide for expression of the enzyme. Manipulation of the nucleic acid sequence encoding a modified lipolytic enzyme prior to its insertion into a vector may be desirable or necessary depending on the expression vector. The techniques for modifying nucleic acid sequences utilizing cloning methods are well known in the art.

The term "control sequences" is defined herein to include all components which are necessary or advantageous for expression of the coding sequence of the nucleic acid sequence. Each control sequence may be native or foreign to the nucleic acid sequence encoding the modified lipolytic enzyme. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a modified lipolytic enzyme.

The control sequence may be an appropriate promoter sequence, a nucleic acid sequence which is recognized by a host cell for expression of the nucleic acid sequence. The promoter sequence contains transcription and translation control sequences which mediate the expression of the modified lipolytic enzyme. The promoter may be any nucleic acid sequence which shows transcriptional activity in the host cell of choice and may be obtained from genes encoding extracellular or intracellular polypeptides either homologous or heterologous to the host cell. Examples of suitable promoters for directing the transcription of the nucleic acid constructs of the present invention, especially in a bacterial host cell, are the promoters obtained from the *E. coli lac* operon, the *Streptomyces coelicolor* agarase gene *(dagA),* the *B. subtilis* levansucrase gene *(sacB)* or the alkaline protease gene, the *B. licheniformis* alpha-amylase gene *(amyL),* the *B. stearothermophilus* maltogenic amylase gene *(amyM),* the *B. amyloliquefaciens* alpha-amylase gene *(amyQ),* the *B. licheniformis* penicillinase gene *(penP),* the *B. subtilis xylA* and *xylB* genes, the *B. pumilus* xylosidase gene, and the prokaryotic beta-lactamase or tryptophan gene (Villa-Karnaroff *et al.,* 1978, *Proceedings of the National Academy of Sciences USA* 75:3727-3731), as well as the *tac* gene (DeBoer *et al.,* 1983, *Proceedings of the National Academy of Sciences USA* 80:21-25). Further promoters are described in "Useful proteins from recombinant bacteria" *in Scientific American,* 1980, 242:74-94; and in Sambrook *et al.,* 1989, *supra.* Examples of suitable promoters for directing the transcription of the nucleic acid constructs of the present invention in a filamentous fungal host cell are promoters obtained from the genes encoding A. *oryzae* TAKA amylase, *A. oryzae* triose phosphate isomerase, *Rhizomucor miehei* aspartic proteinase, *A. niger* neutral alpha-amylase. *A. niger* acid stable alpha-amylase, *A. niger* or *A. awamori* glucoamylase (*glaA*), *Rhizomucor miehei* lipase, *A. oryzae* alkaline protease, *A. oryzae* triose phosphate isomerase, *A. nidulans* acetamidase, *Fusarium oxysporum* trypsin-like protease (as described in U.S. Patent No. 4,288,627, which is incorporated herein by reference), or the ADH-3 promoter (McKnight et al., (1985), The EMBO J. 4, 2093-3099) and hybrids thereof Particularly preferred promoters for use in filamentous fungal host cells is the TAKA amylase and the *gluA* promoters. In a yeast host, promoters from yeast glycolytic genes (Hitzeman et al.,(1980), J. Biol. Chem. 255, 12073-12080; Alber and Kawasaki, (1982), J. Mol. Appl. Gen. 1, 419-434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., (1983), Nature 304, 652-654) promoters. useful promoters are obtained from the *S. cerevisiae* enolase (ENO-1) gene, the *S. cerevisiae* galactokinase gene (GAL1), the *S. cerevisiae* alcohol dehydrogenase/glyceraldehyde-3-phosphate dehydrogenase genes (ADH2/GAP), and the *S. cerevisiae* 3-phosphoglycerate kinase gene. Other useful promoters for yeast host cells are described by Romanos *et al,* 1992, *Yeast* 8:423-488.

The control sequence may also be a suitable transcription terminator sequence, a sequence recognized by a host cell to terminate transcription. The terminator sequence is operably linked to the 3' terminus of the nucleic acid sequence encoding the modified lipolytic enzyme. The terminator sequence may be native to the nucleic acid sequence encoding the modified lipolytic enzyme or may be obtained from foreign sources. Any terminator which is functional in the host cell of choice may be used in the present invention. Preferred terminators for filamentous fungal host cells are obtained from the genes encoding *A. oryzae* TAKA amylase, *A. niger* glucoamylase, *A. nidulans* anthranilate synthase, *A. niger* alpha-glucosidase, and *Fusarium oxysporum* trypsin-like protease. Preferred terminators for yeast host cells are obtained from the genes encoding *S. cerevisiae* enolase, S. *cerevisiae* cytochrome C (CYC1), or *S. cerevisiae* glyceraldehyde-3-phosphate dehydrogenase. Other useful terminators for yeast host cells are described by Romanos *et al.,* 1992, *supra*

The control sequence may also be a suitable leader sequence, a nontranslated region of a mRNA which is important for translation by the host cell. The leader sequence is operably linked to the 5' terminus of the nucleic acid sequence encoding the modified lipolytic enzyme. The leader sequence may be native to the nucleic acid sequence encoding the modified lipolytic enzyme or may be obtained from foreign sources. Any leader sequence which is functional in the host cell of choice may be used in the present invention. Preferred leaders for filamentous fungal host cells are obtained from the genes encoding *A. oryzae* TAKA amylase and *A* oryzae triose phosphate isomerase. Suitable leaders for yeast host cells are obtained from the *S. cerevisiae* enolase (ENO-1) gene, the S. *cerevisiae* 3-phosphoglycerate kinase gene, the *S. cerevisiae* alpha-factor, and the *S. cerevisiae* alcohol dehydrogenase/glyceraldehyde-3-phosphate dehydrogenase genes (ADH2/GAP).

The control sequence may also be a polyadenylation sequence, a sequence which is operably linked to the 3' terminus of the nucleic acid sequence and which, when transcribed, is recognized by the host cell as a signal to add polyadenosine residues to transcribed mRNA The polyadenylation sequence may be native to the nucleic acid sequence encoding the modified lipolytic enzyme or may be obtained from foreign sources. Any polyadenylation sequence which is functional in the host cell of choice may be used in the present invention. Preferred polyadenylation sequences for filamentous fungal host cells are obtained from the genes encoding *A. oryzae* TAKA amylase, *A. niger* glucoamylase, *A. nidulans* anthranilate synthase, and *A. niger* alpha-glucosidase. Useful polyadenylation sequences for yeast host cells are described by Guo and Sherman, 1995, *Molecular Cellular Biology* 15:5983-5990. Polyadenylation sequences are well known in the art for mammalian host cells.

The control sequence may also be a signal peptide coding region, which codes for an amino acid sequence linked to the amino terminus of the modified lipolytic enzyme which can direct the expressed modified lipolytic enzyme into the cell's secretory pathway. The signal peptide coding region may be native to the modified lipolytic enzyme of the invention or may be obtained from foreign sources. The 5' end of the coding sequence of the nucleic acid sequence may inherently contain a signal peptide coding region naturally linked in translation reading frame with the segment of the coding region which encodes the secreted modified lipolytic enzyme. Alternatively, the 5' end of the coding sequence may contain a signal peptide coding region which is foreign to that portion of the coding sequence which encodes the secreted modified lipolytic enzyme. The foreign signal peptide coding region may be required where the coding sequence does not normally contain a signal peptide coding region. Alternatively, the foreign signal peptide coding region may simply replace the natural signal peptide coding region in order to obtain enhanced secretion of the enzyme relative to the natural signal peptide coding region normally associated with the coding sequence. The signal peptide coding region may be obtained from a glucoamylase or an amylase gene from an *Aspergillus* species, a lipase or proteinase gene from a *Rhizomucor* species, the gene for the α-factor from *Saccharomyces cerevisiae,* an amylase or a protease gene from a *Bacillus* species, or the calf preprochymosin gene. An effective signal peptide coding region for bacterial host cells is the signal peptide coding region obtained from the maltogenic amylase gene from *Bacillus* NCIB 11837, the *B. stearothermophilus* alpha-amylase gene, the *B. licheniformis* subtilisin gene, the *B. licheniformis* beta-lactamase gene, the *B. stearothennophilus* neutral proteases genes (*nprT, nprS, nprM*), and the *B. subtilis* PrsA gene. Further signal peptides are described by Simonen and Palva, 1993, *Microbiological Reviews* 57:109-137. An effective signal peptide coding region for filamentous fungal host cells is the signal peptide coding region obtained from *A. oryzae* TAKA amylase gene, *A. niger* neutral amylase gene, the *Rhizomucor miehei* aspartic proteinase gene, the *H. lanuginosa* cellulase gene, or the *Rhizomucor miehei* lipase gene. Useful signal peptides for yeast host cells are obtained from the genes for *S. cerevisiae* α-factor and *S. cerevisiae* invertase. Other useful signal peptide coding regions are described by Romanos *et al.,* 1992, *supra.* However, any signal peptide coding region capable of directing the expressed enzyme into the secretory pathway of a host cell of choice may be used in the present invention.

The nucleic acid constructs of the present invention may also comprise one or more nucleic acid sequences which encode one or more factors that are advantageous in the expression of the modified lipolytic enzyme, *e.g.*, an activator (*e.g.*, a trans-acting factor), a chaperone, and a processing protease. The nucleic acids encoding one or more of these factors are not necessarily in tandem with the nucleic acid sequence encoding the modified lipolytic enzyme. An activator is a protein which activates transcription of a nucleic acid sequence encoding a modified lipolytic enzyme (Kudla *et al.,* 1990, *EMBO Journal* 9:1355-1364; Jarai and Buxton, 1994, *Current Genetics* 26:2238-244; Verdier, 1990, *Yeast* 6:271-297). The nucleic acid sequence encoding an activator may be obtained from the genes encoding *B. stearothermophilus* NprA *(nprA), S. cerevisiae* heme activator protein I (*hap1*), *S. cerevisiae* galactose metabolizing protein 4 (*gal4*), and *A. nidulans* ammonia regulation protein (*areA*). For further examples, see Verdier, 1990, *supra* and MacKenzie *et al.*, 1993, *Journal of General Microbiology* 139:2295-2307. A chaperone is a protein which assists another polypeptide in folding properly (Hartl *et al.,* 1994, *TIBS* 19:20-25; Bergeron *et al.,* 1994, *TIBS* 19:124-128; Demolder *et al.,* 1994, *Journal of Biotechnology* 32:179-189; Craig, 1993, *Science* 260:1902-1903; Gething and Sambrook, 1992, *Nature* 355:33-45; Puig and Gilbert, 1994, *Journal of Biological Chemistry* 269:7764-7771; Wang and Tsou, 1993, *The FASEB Journal* 7:1515-11157; Robinson *et al.,* 1994, *Bio/Technology* 1:381-384). The nucleic acid sequence encoding a chaperone may be obtained from the genes encoding *B. subtilis* GroE proteins, *A. oryzae* protein disulphide isomerase, *S. cerevisiae* calnexin, *S. cerevisiae* BiP/GRP78, and *S. cerevisiae* Hsp70. For further examples, See Gething and Sambrook, 1992, *supra*, and Hart1, *et al.*, 1994, *supra*. Any factor that is functional in the host cell of choice may be used in the present invention.

It may also be desirable to add regulatory sequences which allow the regulation of the expression of the modified lipolytic enzyme relative to the growth of the host cell. Examples of regulatory systems are those which cause

the expression of the gene to be turned on or off in response to a chemical or physical stimulus, including the presence of a regulatory compound. Regulatory systems in prokaryotic systems would include the *lac, tac,* and *trp* operator systems. In yeast, the ADH2 system or GAL1 system may be used. In filamentous fungi, the TAKA alpha-amylase promoter, *A. niger* glucoamylase promoter, and the *A. oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleic acid sequence encoding the modified lipolytic enzyme would be placed in tandem with the regulatory sequence.

**Expression Vectors**

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the modified lipolytic enzyme at such sites. Alternatively, the nucleic acid sequence of the present invention may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids. The vector may be an autonomously replicating vector, *i.e.,* a vector which exists as an extrachromosomal entity, the replication of which is independent of chromosomal replication, *e.g.*, a plasmid, an extrachromosomal element, a minichromosome, or an artificial chromosome. The vector may contain any means to assuring self-replication. Alternatively, the vector may be one which, when introduced into the host cell, is integrated into the genome and replicated together with the chromosome(s) into which it has been integrated. The vector system may be a single vector or plasmid or two or more vectors or plasmids which together contain the total DNA to be introduced into the genome of the host cell, or a transposon.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *B. subtilis* or *B. licheniformis,* or markers which confer antibiotic resistance such as ampicillin, kanamycin, chloramphenicol or tetracycline resistance. A frequently used mammalian marker is the dihydrofolate reductase gene. Suitable markers for yeast host cells are ADE2, HIS3, LEU2, LYS2, MET3, TRP1, and URA3. A selectable marker for use in a filamentous fungal host cell may be selected from the group including, but not limited to, *amdS* (acetamidase), *argB* (omithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), and glufosinate resistance markers, as well as equivalents from other species. Preferred for use in an *Aspergillus* cell are the *amdS* and *pyrG* markers of *A. nidulans* or *A. oryzae* and the *bar* marker of *Streptomyces hygroscopicus.* Furthermore, selection may be accomplished by co-transformation, *e.g.*, as described in WO 91/17243, where the selectable marker is on a separate vector.

The vectors of the present invention preferably contain an element(s) that permits stable integration of the vector into the host cell genome or autonomous replication of the vector in the cell independent of the genome of the cell.

The vectors of the present invention may be integrated into the host cell genome when introduced into a host cell. For integration, the vector may rely on the nucleic acid sequence encoding the modified lipolytic enzyme or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleic acid sequences. On the other hand, the vector may be integrated into the genome of the host cell by non-homologous recombination. These nucleic acid sequences may be any sequence that is homologous with a target sequence in the genome

of the host cell, and, furthermore, may be non-encoding or encoding sequences. For autonomous replication, the vector may further comprise an origin of replication enabling the vector to replicate autonomously in the host cell in question. Examples of bacterial origins of replication are the origins of replication of plasmids pBR322, pUC19, pACYC177, pACYC184, pUB110, pE194, pTA1060, and pAMß1. Examples of origin of replications for use in a yeast host cell are the 2 micron origin of replication, the combination of CEN6 and ARS4, and the combination of CEN3 and ARS1. The origin of replication may be one having a mutation which makes its functioning temperature-sensitive in the host cell (see, *e.g.*, Ehrlich, 1978, *Proceedings of the National Academy of Sciences USA* 75:1433).

More than one copy of a nucleic acid sequence encoding a modified lipolytic enzyme of the present invention may be inserted into the host cell to amplify expression of the nucleic acid sequence. Stable amplification of the nucleic acid sequence can be obtained by integrating at least one additional copy of the sequence into the host cell genome using methods well known in the art and selecting for transformants.

The procedures used to ligate the elements described above to construct the recombinant expression vectors of the present invention are well known to one skilled in the art (see, *e.g.*, Sambrook *et al.,* 1989, *supra).*

## Host Cells

The present invention also relates to recombinant host cells, comprising a nucleic acid sequence of the invention, which are advantageously used in the recombinant production of the modified lipolytic enzymes. The cell is preferably transformed with a vector comprising a nucleic acid sequence of the invention followed by integration of the vector into the host chromosome. "Transformation" means introducing a vector comprising a nucleic acid sequence of the present invention into a host cell so that the vector is maintained as a chromosomal integrant or as a self-replicating extra-chromosomal vector. Integration is generally considered to be an advantage as the nucleic acid sequence is more likely to be stably maintained in the cell. Integration of the vector into the host chromosome may occur by homologous or non-homologous recombination as described above.

The choice of a host cell will to a large extent depend upon the gene encoding the modified lipolytic enzyme and its source. In addition, the choice of host cell will often depend on the proteolytic enzyme system of the host cell and its impact on the production of a modified lipolytic enzyme of the invention. Accordingly, it may be desirable to use a host cell which is deficient in one or more proteolytic enzymes or other enzyme processing means. Protease deficient host cells of bacteria as well as fungal (filamentous fungal and yeast) cells are well-known in the art.

The host cell may be a unicellular microorganism or a non-unicellular microorganism. Useful unicellular cells are bacterial cells such as gram positive bacteria including, but not limited to, a *Bacillus* cell, *e.g.*, *B. subtilis*, *B. licheniformis, B. lentus, B. brevis, B. stearothermophilus, B. alkalophilus*, *B. amyloliquefaciens*, *B. coagulans*, *B. circulans*, *B. lautus, B. megaterium,* and *B. thuringiensis*, or a *Streptomyces* cell, *e.g.*, *S. lividans* or *S. murinus,* or gram negative bacteria such as *E coli* and *Pseudomonas* sp. (especially when a bacterial lipolytic enzyme, such as a *Pseudomonas sp.* enzyme is to be produced). The transformation of a bacterial host cell may, for instance, be effected by protoplast transformation (see, *e.g.*, Chang and Cohen, 1979, *Molecular General Genetics* 168:111-115), by using competent cells (see, *e.g.*, Young and Spizizin, 1961, *Journal of Bacteriology* 81:823-829, or Dubnar and Davidoff-Abelson, 1971, *Journal of Molecular Biology* 56:209-221), by electroporation (see, *e.g.,* Shigekawa and Dower, 1988, *Biotechniques* 6:742-751), or by conjugation (see, *e.g.,* Koehler and Thome, 1987, *Journal of Bacteriology* 169:5771-5278).

The host cell may be a eukaryote, and is preferably a fungal, i.e. a yeast cell or a filamentous fungal cell, especially for the production of a modified lipolytic enzyme of eukaryotic origin.

"Yeast" as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae (*e.g.*, genus *Schizosaccharomyces*), Nadsonioideae, Lipomycoideae, and Saccharomycoideae (*e.g.,* genera *Pichia, Kluyveromyces* and *Saccharomyces*). The basidiosporogenous yeasts include the genera *Leucosporidim, Rhodosporidium, Sporidiobolus, Filobasidium,* and *Filobasidiella*. Yeast belonging to the Fungi Imperfecti are divided into two families, Sporobolomycetaceae (*e.g.,* genera *Sorobolomyces* and *Bullera*) and Cryptococcaceae (*e.g.*, genus *Candida*). Since the classification of yeast may change in the future, for the purposes of this invention, yeast shall be defined as described in *Biology and Activities of Yeast* (Skinner, F.A, Passmore, S.M., and Davenport, RR, eds, Soc. App. Bacteriol. Symposium Series No. 9, 1980. The biology of yeast and manipulation of yeast genetics are well known in the art (see, *e.g., Biochemistry and Genetics of Yeast,* Bacil, M., Horecker, B.J., and Stopani, A O.M., editors, 2nd edition, 1987; *The Yeasts*, Rose, AH., and Harrison, J.S., editors, 2nd edition, 1987; and *The Molecular Biology of the Yeast Saccharomyces,* Strathern *et al.,* editors, 1981). In connection with the present invention the use of yeast cells which typically have another proteolytic enzyme processing system that, e.g., bacteria and filamentous fungi, may be of particular use for preparing modified lipolytic enzymes which, as the peptide addition, comprise a part or all of the natural prosequences of the parent lipolytic enzyme in question. When the fungal host cell is a yeast cell (e.g. to be used in applying a peptide addition (in the form of part of or the entire prosequence of the parent enzyme, the yeast

host cell may be a cell of a species of *Candida, Kluyveromyces, Saccharomyces, Schizosaccharomyces, Pichia,* or *Yarrowia such as* a *S. cerevisiae* cell, a *S.s carlsbergensis, a S. diastaticus* cell, a *S. douglasii* cell, a *S. kluyveri* cell, a *S. norbensis* cell, or a *S. oviformis* cell.

"Fungi" as used herein includes the phyla Ascomycota, Basidiomycota, Chytridiomycota, and Zygomycota (as defined by Hawksworth *et al., In, Ainsworth and Bisby's Dictionary of The Fungi,* 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra).* Representative groups of Ascomycota include, *e.g.*, *Neurospora, Eupenicillium* (=*Penicillium*), *Emericella* (=*Aspergillus*), *Eurotium* (=*Aspergillus*), and the true yeasts listed above. Examples of Basidiomycota include mushrooms, rusts, and smuts. Representative groups of Chytridiomycota include, *e. g., Allomyces, Blartocladiella, Coelomomyces,* and aquatic fungi. Representative groups of Oomycota include, *e.g.,* Saprolegniomycetous aquatic fungi (water molds) such as *Achlya.* Examples of mitosporic fungi include *Aspergillus, Penicillium, Candida,* and *Alternaria.* Representative groups of Zygomycota include, *e.g., Rhizopus* and *Mucor.*

"Filamentous fungi" include all filamentous forms of the subdivision Eumycota and Oomycota (as defined by *Hawksworth et al.,* 1995, *supra).* The filamentous fungi are characterized by a vegetative mycelium composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In a preferred embodiment, the fungal host cell is a filamentous fungal cell. In a more preferred embodiment, the filamentous fungal host cell is a cell of a species of, but not limited to, *Acremonium, Aspergillus, Fusarium, Humicola, Afyceliophihora, Mucor, Neurospora, Penicillium, Thielavia, Tolypocladium,* and *Trichoderma.* In an even more preferred embodiment, the filamentous fungal host cell is an *Aspergillus* cell. In another even more preferred embodiment, the filamentous fungal host cell is a *Fusarium* cell. In a most preferred embodiment, the filamentous fungal host cell is an *A. oryzae* cell, an *A. niger* cell, an *A. foetidus* cell, or an *A. japonicus* cell. In another most preferred embodiment, the filamentous fungal host cell is a *Fusarium orysporum* cell or a *F. gramirremum* cell.

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton *et al.,* 1984, *Proceedings of the National Academy of Sciences USA* 81: 1470-1474. A suitable method of transforming *Fusarium* species is described by Malardier *et al.*, 1989, *Gene* 78: 147-156 or in WO 96/00787. Yeast may be transformed using the procedures described by Becker and Guarente, *In* Abelson, J.N. and Simon, M.I., editors, *Guide to Yeast Genetics and Molecular Biology,* Methods in Enzymology, Volume 194, pp 182-187, Academic Press, Inc., New York; Ito *et al.,* 1983, *Journal of Bacteriology* 153:163; and Hinnen *et al.,* 1978, *Proceedings of the National Academy of Sciences USA* 75:1920. Mammalian cells may be transformed by direct uptake using the calcium phosphate precipitation method of Graham and Van der Eb (1978, *Virology* 52:546).

Host cells used according to the invention may advantagously be protease deficient or protease minus strains (or the like), such as strains deficient of proteases, especially exo-proteases, capable of cleaving the modified lipolytic enzyme at a site close to the peptide addition (in certain case being the propeptide). Especially relevante host cells are the host cells deficient of the tripeptidyl-aminopeptidases (TPAP) (see e.g. WO 96/14404 from Novo Nordisk A/S), deficient of dipeptidyl-aminopeptidases (DPAP), or host cells deficient of a Kex2 protease or a Kex2-tike protease and therefore not capable of cleaving at di-basic site such as Arg-Arg (RR).

Other examples of host cells include the aspartic proteinase deficient host cells described in EP 429 490 (from Genencor Inc), host cells deficient of proteolytic enzymes such as the host cells described in WO 93/02925 (Amgen), WO 92/17595 (Salk Inst Biotech), EP 341 215, EP 574 347, and PCT/DK96/00111 (Novo Nordisk A/S).

In the Examples below an *Aspergillus oryzae* host cell, with a deleted alkaline protease gene, have been used.

**Methods of Production**

The present invention also relates to methods for producing a modified lipolytic enzyme of the invention comprising (a) cultivating a host cell transformed with a DNA sequence encoding the enzyme under conditions conducive to expression of the modified lipolytic enzyme; and (b) recovering the modified lipolytic enzyme.

The host cells may be cultivated in a nutrient medium suitable for production of the modified lipolytic enzyme using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the modified lipolytic enzyme to be expressed and/or isolated. The cultivation takes place in a suitable nutrient medium comprising carbon and nitrogen sources and inorganic salts, using procedures known in the art (see, e.g., references for bacteria and yeast; Bennett, J.W. and LaSure, L., editors, *More Gene Martipulations in Fungi,* Academic Press, CA, 1991). Suitable media are available from commercial suppliers or may be prepared according to published compositions (*e.g.*, in catalogues of the American Type Culture Collection). If the modified lipolytic enzyme is secreted into the nutrient medium, the modified lipolytic

enzyme can be recovered directly from the medium. If the modified lipolytic enzyme is not secreted, it is recovered from cell lysates.

The resulting modified lipolytic enzyme may be recovered by methods known in the art. For example, the modified lipolytic enzyme may be recovered from the nutrient medium by conventional procedures including, but not limited to, centrifugation, nitration, extraction, spray-drying, evaporation, or precipitation. The recovered modified lipolytic enzyme may then be further purified by a variety of chromatographic procedures, *e.g.*, ion exchange chromatography, gel filtration chromatography, affinity chromatography, or the like.

The modified lipolytic enzymes of the present invention may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing (IEF), differential solubility (*e.g.*, ammonium sulfate precipitation), or extraction (see *e.g.*, *Protein Purification*, J.-C. Janson and Lars Ryden, editors, VCH Publishers, New York, 1989).

In a preferred embodiment of the invention a peptide addition is applied to the parent enzyme by cultivating a host cell comprising a DNA sequence encoding the pre, pro or prepro-form of the parent lipolytic enzyme, the DNA sequence optionally being present on a vector, and recovering the resulting modified lipolytic enzyme. The host cell, cultivation condition and/or recovery conditions being selected so that at the most a partial processing of the pre, pro or preproform of the parent enzyme has occured resulting in that at least 5%, such as at least 10%, such as at least 15%, such as at least 20%, such as at least 25%, such as at least 50% or at least 75% of the produced modified enzyme molecules comprises the desired , e.g. the entire pre-sequence or a substantial part thereof

## Enzyme composition of the invention

In a further aspect the invention relates to an enzyme composition comprising an enzyme with lipolytic activity of the invention.

As defined herein, a "substantially pure" enzyme is an enzyme which is essentially free of other homologous contaminants (originating from the same source as the modified lipolytic enzyme), e.g., at least about 20% pure, preferably at least about 40% pure, more preferably about 60% pure, even more preferably about 80% pure, most preferably about 90% pure, and even most preferably about 95% pure, as determined by SDS-PAGE.

In certain cases the host cell does not process all of the modified lipolytic enzyme molecules expressed by that host at the same cleavage site, resulting in a modified ehzyme product consisting of a portion having the full length peptide addition and one or more other portions with only a part of the peptide addition. The inventors found that this does not influence the wash performance significantly. Consequently, even though not all of the lipolytic enzyme of the enzyme composition of the invention may have retained the full length peptide addition the enzyme composition is still capable of exerting the desired effect, such as an improved wash performance. Actually, it has been found that as long as at least about 5% of the total amount of modified lipolytic enzyme of the invention to be used for a given purpose has the intact peptide addition as disclosed above, this may be found to be sufficient for providing the desired effect. The remaining part of the modified lipolytic enzyme molecules may then have a peptide addition which is shorter than the one intended (e.g. as a consequence of one or more amino acid residues have been cut off during processing of the enzyme by the host organism) or no peptide addition at all. Therefore, the enzyme composition of the invention need only to comprise at least about 5%, preferably at least about 10%, such as at least about 25%, better at least about 50%, especially at least about 75% of the modified lipolytic enzyme with its full length addition.

Said enzyme composition may further comprise an enzyme selected from the group of proteases, cellulases, peroxidases, cutinases, amylases and/or lipases, and when intended for washing also ingredients normally used in detergent compositions.

Modified lipolytic enzymes of the invention have been found to be of particular interest as components in detergent compositions such as washing powder or dishwashing compositions which will be described in details in the following section. In addition, due to their improved properties the modified lipolytic enzymes of the invention are contemplated to be useful in, for example, the baking industry, as a catalyst in organic syntheses (e.g. esterification, transesterification or ester hydrolysis reactions), in the papermaking industry (e.g. for pitch removal), and in the leather, wool and related industries (e.g. for degreasing of animal hides, sheepskin or wool), and for other applications involving degreasing/defatting.

## DETERGENT DISCLOSURE

### Surfactant system

The detergent compositions according to the present invention comprise a surfactant system wherein the surfactant can be selected from nonionic and/or anionic and/or cationic and/or ampholytic and/or zwitterionic and/or

semi-polar surfactants.

The surfactant is typically present at a level of from 0.1 % to 60% by weight.

The surfactant is preferably formulated to be compatible with enzyme components present in the composition. In liquid or gel compositions the surfactant is most preferably formulated such that it promotes, or at least does not degrade, the stability of any enzyme in these compositions.

Preferred surfactant systems to be used according to the present invention comprise as a surfactant one or more of the nonionic and/or anionic surfactants described herein.

Nonionic detergent surfactants normally consist of a water-solubilizing polyalkoxylene or a mono- or di-alkanolamide group in chemical combination with an organic hydrophobic group derived, for example, from alkylphenols in which the alkyl group contains from about 6 to about 12 carbon atoms, dialkylphenols in which each alkyl group contains from 6 to 12 carbon atoms, primary, sedondary or tertiary aliphatic alcohols (or alkyl-capped derivatives thereof), preferably having from 8 to 20 carbon atoms, monocarboxylic acids having from 10 to about 24 carbon atoms in the alkyl group and polyproxylenes. Also common are fatty acid mono- and dialkanolamides in which the alkyl group of the fatty acid radical contains from 10 to about 20 carbon atoms and the alkyloyl group having from 1 to 3 carbon atoms. Optionally, in any of the mono- and di-alkanolamide derivatives there may be a polyoxyalkylene moiety joining the latter groups and the hydrophobic part of the molecule. In all polyalkoxylene containing surfactants, the polyalkoxylene moiety preferably consists of from 2 to 20 groups of ethylene oxide or of ethylene oxide and propylene oxide groups.

Polyethylene, polypropylene, and polybutylene oxide condensates of alkyl phenols are suitable for use as the nonionic surfactant of the surfactant systems of the present invention, with the polyethylene oxide condensates being preferred. These compounds include the condensation products of alkyl phenols having an alkyl group containing from about 6 to about 14 carbon atoms, preferably form about 8 to about 14 carbon atoms, in either a straightchain or branched-chain configuration with the alkylene oxide. In a preferred embodiment, the ethylene oxide is present in an amount equal to from about 2 to about 25 moles, more preferably from about 3 to about 15 moles, of ethylene oxide per mole of alkyl phenol. Commercially available nonionic surfactants of this type include Igepal™ CO-630, marketed by the GAP Corporation; and Triton™ X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with from about 1 to about 25 moles of ethylene oxide are suitable for use as the nonionic surfactant of the nonionic surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol™ 15-S-9 (The condensation product of $C_{11}$-$C_{15}$ linear alcohol with 9 moles ethylene oxide), Tergitol™ 24-L-6 NMW (the condensation product of $C_{12}$-$C_{14}$ primary alcohol with 6 moles ethylene oxide with a narrow molecular weight distribution), both marketed by Union Carbide Corporation; Neodol™ 45-9 (the condensation product of $C_{14}$-$C_{15}$ linear alcohol with 9 moles of ethylene oxide), Neodol™ 23-3 (the condensation product of $C_{12}$-$C_{13}$ linear alcohol with 3.0 moles of ethylene oxide), Neodol™ 45-7 (the condensation product of $C_{14}$-$C_{15}$ linear alcohol with 7 moles of ethylene oxide), Neodol™ 45-5 (the condensation product of $C_{14}$-$C_{15}$ linear alcohol with 5 moles of ethylene oxide) marketed by Shell Chemical Company, Kyro™ EOB (the condensation product of $C_{13}$-$C_{15}$ alcohol with 9 moles ethylene oxide), marketed by The Procter & Gamble Company, and Genapol LA 050 (the condensation product of $C_{12}$-$C_{14}$ alcohol with 5 moles of ethylene oxide) marketed by Hoechst. Preferred range of HLB in these products is from 8-11 and most preferred from 8-10.

The detergent composition of the invention may comprise a nonionic material which is alkoxylated aliphatic alcohols containing at least 25 alkylene oxide groups, preferably at least 50 alkylene oxide groups, and more preferably at least 80 alkylene oxide groups.

Also useful as the nonionic surfactant of the surfactant systems of the present invention are alkylpolysaccharides disclosed in US 4,565,647, having a hydrophobic group containing from about 6 to about 30 carbon atoms, preferably from about 10 to about 16 carbon atoms and a polysaccharide, e.g. a polyglycoside, hydrophillic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units.

Another useful nonionic surfactant is a glycoside of a uronic acid, a uronic acid salt or a uronic acid lactone or polyuronic acid with a straight-chain or branced saturated or unsaturated aliphatic chain of from 6 to 24 carbon

atoms, optionally containing an aromatic, cycloaliphatic, mixed aromatic-aliphatic or polyalkyloxyalkyl radical, as described in WO 95/10524.

The preferred alkylpolyglycosides have the formula

$$R^2O(C_nH_{2n}O)_t(glycosyl)_x$$

wherein $R^2$ is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4-, and/or 6-position, preferably predominately the 2-position.

The condensation products of ethylene oxide with a hydrophobic base formed by the condensation of propylene oxide with propylene glycol are also suitable for use as the additional nonionic surfactant systems of the present invention. The hydrophobic portion of these compounds will preferably have a molecular weight of from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially available Pluronic™ surfactants, marketed by BASF.

Also suitable for use as the nonionic surfactant of the nonionic surfactant system of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11.000. Examples of this type of nonionic surfactant include certain of the commercially available Tetronic™ compounds, marketed by BASF.

Preferred for use as the nonionic surfactant of the surfactant systems of the present invention are polyethylene oxide condensates of alkyl phenols, condensation products of primary and secondary aliphatic alcohols with from about 1 to about 25 moles of ethyleneoxide, alkylpolysaccharides, and mixtures hereof. Most preferred are $C_8$-$C_{14}$ alkyl phenol ethoxylates having from 3 to 15 ethoxy groups and $C_8$-$C_{18}$ alcohol ethoxylates (preferably $C_{10}$ avg.) having from 2 to 10 ethoxy groups, and mixtures thereof. Highly preferred nonionic surfactants are polyhydroxy fatty acid amide surfactants of the formula

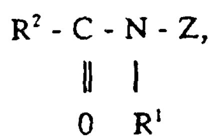

wherein $R^1$ is H, or $R^1$ is $C_{1-4}$ hydrocarbyl, 2-hydroxy ethyl, 2-hydroxy propyl or a mixture thereof, $R^2$ is $C_{5-31}$ hydrocarbyl, and Z is a polyhydroxyhydrocarbyl having a linear hydrocarbyl chain with at least 3 hydroxyls directyl connected to the chain, or an alkoxylated derivative thereof. Preferably, $R^1$ is methyl, $R^2$ is straight $C_{11-15}$ alkyl or $C_{16-18}$ alkyl or alkenyl chain such as coconut alkyl or mixtures thereof, and Z is derived from a reducing sugar such as glucose, fructose, maltose, lactose, in a reductive amination reaction.

A nonionic surfactant system comprising an alkoxylated nonionic surfactant having an average degree of alkoxylation of at least 6 and an aldobionamide of the structure $ANR_1R_2$ wherein A is a sugar moiety which is an aldobionic acid except that it does not contain the OH group normally extending from the carbonyl group on the aldonic acid. $NR_1R_2$ is attached where the hydroxyl group on the aldobionic acid would normally be found. $R_1R_2$, may be the same or different, is a hydrogen atom, an aliphatic hydrocarbon radical, an aromatic radical a cycloaliphatic radical an amino acid ester, or an ether amine. $R_1$ and $R_2$ cannot both be hydrogen atoms as described in WO 95/2770.

Other so-called nonionic detergent compounds include long chain tertiary amine oxides, long chain tertiary phosphine oxides and dialkyl sulphoxides.

Highly preferred anionic surfactants include alkyl alkoxylated sulfate surfactants hereof are water soluble salts

or acids of the formula $RO(A)_mSO_3M$ wherein R is an unsubstituted $C_{10}$-$C_{24}$ alkyl or hydroxyalkyl group having a $C_{10}$-$C_{24}$ alkyl component, preferably a $C_{12}$-$C_{20}$ alkyl or hydroxyalkyl, more preferably $C_{12}$-$C_{18}$ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethylammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are $C_{12}$-$C_{18}$ alkyl polyethoxylate (1.0) sulfate ($C_{12}$-$C_{18}E(1.0)M$), $C_{12}$-$C_{18}$ alkyl polyethoxylate (2.25) sulfate ($C_{12}$-$C_{18}(2.25)M$, and $C_{12}$-$C_{18}$ alkyl polyethoxylate (3.0) sulfate ($C_{12}$-$C_{18}E$ (3.0)M), and $C_{12}$-$C_{18}$ alkyl polyethoxylate (4.0) sulfate ($C_{12}$-$C_{18}E(4.0)M$), wherein M is conveniently selected from sodium and potassium. Suitable anionic surfactants to be used are alkyl ester sulfonate surfactants including linear esters of $C_8$-$C_{20}$ carboxylic acids (i.e., fatty acids) which are sulfonated with gaseous $SO_3$ according to "The Journal of the American, Oil Chemists Society", 52 (1975), pp. 323-329. Suitable starting materials would include natural fatty substances as derived from tallow, palm oil, etc.

The preferred alkyl ester sulfonate surfactant, especially for laundry applications, comprise alkyl ester sulfonate surfactants of the structural formula:

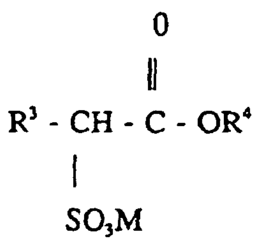

wherein $R^3$ is a $C_8$-$C_{20}$ hydrocarbyl, preferably an alkyl, or combination thereof, $R^4$ is a $C_1$-$C_6$ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethonolamine, and triethanolamine. Preferably, $R^3$ is $C_{10}$-$C_{16}$ alkyl, and $R^4$ is methyl, ethyl or isopropyl. Especially preferred are the methyl ester sulfonates wherein $R^3$ is $C_{10}$-$C_{16}$ alkyl.

Other suitable anionic surfactants include the alkyl sulfate surfactants which are water soluble salts or acids of the formula $ROSO_3M$ wherein R preferably is a $C_{10}$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of $C_{12}$-$C_{16}$ are preferred for lower wash temperatures (e.g. below about 50°C) and $C_{16}$ $C_{18}$ alkyl chains are preferred for higher wash temperatures (e.g. above about 50°C).

Other anionic surfactants useful for detersive purposes can also be included in the laundry detergent compositions of the present invention. Theses can include salts (including, for example, sodium, potassium, ammonium, and substituted ammonium salts such as mono- di- and triethanolamine salts) of soap, $C_8$-$C_{22}$ primary or secondary alkanesulfonates, $C_8$-$C_{24}$ olefinsulfonates, sulfonated polycarboxylic acids prepared by sulfonation of the pyrolyzed product of alkaline earth metal citrates, e.g., as described in GB 1,082,179, $C_8$-$C_{24}$ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, isethionates esters of alkoxy carboxylic acids (as described in WO95/14661), N-acyl taurates, alkyl succinamates and sulfosuccinates, monoesters of sulfosuccinates (especially saturated and unsaturated $C_{12}$-$C_{18}$ monoesters) and diesters of sulfosuccinates (especially saturated and unsaturated $C_6$-$C_{12}$ diesters), acyl sarcosinates, oleoyl sarcosinate, sulfates of alkylpolysaccharides such as the sulfates of alkylpolyglucoside (the nonionic nonsulfated compounds being described below), branched primary alkyl sulfates, and alkyl polyethoxy carboxylates such as those of the formula $RO(CH_2CH_2O)_k$-$CH_2COO$-$M+$ wherein R is a $C_8$-$C_{22}$ alkyl, k is an integer from 1 to 10, and M is a soluble salt forming cation. Resin acids and hydrogenated resin acids are also suitable, such as rosin, hydrogenated rosin, and resin acids and hydrogenated resin acids present in or derived from tall oil. Alkylbenzene sulfonate is highly preferred, in particular linear alkyl benzene sulfonate (LAS) where the alkyl group contains preferably from 10 to 18 carbon atoms.

Further examples are described in "Surface Active Agents and Detergents" (Vol. I and II by Schwartz, Perrry and Berch). A variety of such surfactants are also generally disclosed in US 3,929,678, (Column 23, line 58 through Column 29, line 23).

When included therein, the laundry detergent compositions of the present invention typically comprise from about 1 % to about 40%, preferably from about 3% to about 20% by weight of such anionic surfactants.

The laundry detergent compositions of the present invention may also contain cationic, ampholytic, zwitterionic, and semi-polar surfactants, as well as the nonionic and/or anionic surfactants other than those already described herein.

Cationic detersive surfactants suitable for use in the laundry detergent compositions of the present invention are those having one long-chain hydrocarbyl group. Examples of such cationic surfactants include the ammonium surfactants such as alkyltrimethylammonium halogenides, and those surfactants having the formula:

$$[R^2(OR^3)_y][R^4OR^3)_y]_2R^5N+X-$$

wherein $R^2$ is an alkyl or alkyl benzyl group having from about 8 to about 18 carbon atoms in the alkyl chain, each $R^3$ is selected form the group consisting of $-CH_2CH_2-$, $-CH_2CH(CH_3)-$, $-CH_2CH(CH_2OH)-$, $-CH_2CH_2CH_2-$, and mixtures thereof; each $R^4$ is selected from the group consisting of $C_1$-$C_4$ alkyl, $C_1$-$C_4$ hydroxyalkyl, benzyl ring structures formed by joining the two $R^4$ groups, $-CH_2CHOHCHOHCOR^6CHOHCH_2OH$ wherein $R^6$ is any hexose or hexose polymer having a molecular weight less than about 1000, and hydrogen when y is not 0; $R^5$ is the same as $R^4$ or is an alkyl chain wherein the total number of carbon atoms or $R^2$ plus $R^5$ is not more than about 18; each y is from 0 to about 10 and the sum of the y values is form 0 to about 15; and X is any compatible anion.

Highly preferred cationic surfactants are the water soluble quaternary ammonium compounds useful in the present composition having the formula:

$$R_1R_2R_3R_4N^+X^- \qquad (i)$$

wherein $R_1$ is $C_8$-$C_{16}$ alkyl, each of $R_2$, $R_3$ and $R_4$ is independently $C_1$-$C_4$ alkyl, $C_1$-$C_4$ hydroxy alkyl, benzyl, and $-(C_2H_{40})_xH$ where x has a value from 2 to 5, and X is an anion. Not more than one of $R_2$, $R_3$ or $R_4$ should be benzyl.

The preferred alkyl chain length for $R_1$ is $C_{12}$-$C_{15}$ particularly where the alkyl group is a mixture of chain lengths derived from coconut or palm kernel fat or is derived synthetically by olefin build up or OXO alcohols synthesis.

Preferred groups for $R_2R_3$ and $R_4$ are methyl and hydroxyethyl groups and the anion X may be selected from halide, methosulphate, acetate and phosphate ions. Examples of suitable quaternary ammonium compounds of formulae (i) for use herein are:

coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
$C_{12-15}$ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonim chloride or bromide;
lauryl dimethyl (ethenoxy)$_4$ ammonium chloride or bromide;
choline esters (compounds of formula (i) wherein $R_1$ is

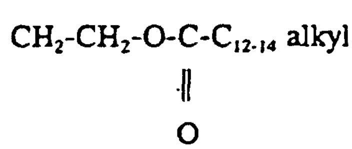

and $R_2R_3R_4$ are methyl).
di-alkyl imidazolines [compounds of formula (i)].

Other cationic surfactants useful herein are also described in US 4,228,044 and in EP 000 224.

When included therein, the laundry detergent compositions of the present invention typically comprise from 0.2 % to about 25%, preferably from about 1% to about 8% by weight of such cationic surfactants.

Ampholytic surfactants are also suitable for use in the laundry detergent compositions of the present invention. These surfactants can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about 8 to about 18 carbon atoms, and at least one contains an anionic water-solubilizing group, e.g. carboxy, sulfonate, sulfate. See US 3,929,678 (column 19, lines 18-35) for examples of ampholytic surfactants.

When included therein, the laundry detergent compositions of the present invention typically comprise from 0.2 % to about 15%, preferably from about 1 % to about 10% by weight of such ampholytic surfactants.

Zwitterionic surfactants are also suitable for use in laundry detergent compositions. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See US 3,929,678 (column 19, line 38 through column 22, line 48) for examples of zwitterionic surfactants.

When included therein, the laundry detergent compositions of the present invention typically comprise from 0.2% to about 15%, preferably from about 1% to about 10% by weight of such zwitterionic surfactants.

Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms; watersoluble phosphine oxides containing one alkyl moiety of form about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety of from about 10 to about 18 carbon atoms and a moiety selected from the group consisting of alkyl and hydroxyalkyl moieties of from about I to about 3 carbon atoms.

Semi-polar nonionic detergent surfactants include the amine oxide surfactants having the formula:

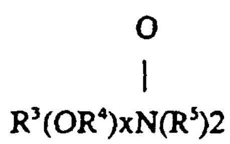

wherein $R^3$ is an alkyl, hydroxyalkyl, or alkyl phenyl group or mixtures thereof containing from about 8 to about 22 carbon atoms; $R^4$ is an alkylene or hydroxyalkylene group containing from about 2 to about 3 carbon atoms or mixtures thereof; x is from 0 to about 3: and each $R^5$ is an alkyl or hydroxyalkyl group containing from about 1 to about 3 carbon atoms or a polyethylene oxide group containing from about 1 to about 3 ethylene oxide groups. The $R^5$ groups can be attached to each other, e.g., through an oxygen or nitrogen atom, to form a ring structure.

These amine oxide surfactants in particular include $C_{10}$-$C_{18}$ alkyl dimethyl amine oxides and $C_8$-$C_{12}$ alkoxy ethyl dihydroxy ethyl amine oxides.

When included therein, the laundry detergent compositions of the present invention typically comprise from 0.2% to about 15%, preferably from about 1% to about 10% by weight of such semi-polar nonionic surfactants.

Builder system

The compositions according to the present invention may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethylenephosphonic acid. Phosphate builders can also be used herein, e.g pyrophosphates, orthophosphates or polyphosphates.

Suitable builders can be an inorganic ion exchange material, commonly an inorganic hydrated aluminosilicate material, more particularly a hydrated synthetic zeolite such as hydrated zeolite A, X, B, HS or MAP. Another suitable inorganic builder material is layered silicate, e.g. SKS-6 (Hoechst). SKS-6 is a crystalline layered silicate consisting of sodium silicate ($Na_2Si_2O_5$).

Suitable polycarboxylates containing one carboxy group include lactic acid, glycolic acid and ether derivatives thereof as disclosed in BE 831,368, BE 821,369 and BE 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in DE 2,446,686, DE 2,446,487,

US 3,935,257 and the sulfinyl carboxylates described in BE 840,623. Polycarboxylates containing three carboxy groups include, in particular, water-soluble citrates, aconitrates and citraconates as well as succinate derivatives such as the carboxymethyloxysuccinates described in GB 1,379,241, lactoxysuccinates described in NL application 7205873, and the oxypolycarboxylate materials such as 2-oxa-1,1,3-propane tricarboxylates described in GB 1,387,447.

Polycarboxylates containing four carboxy groups include oxydisuccinates disclosed in GB 1,261,829, 1, 1,2,2,-ethane tetracarboxylates, 1,1,3,3-propane tetracarboxylates containing sulfo substituents include the sulfosuccinate derivatives disclosed in GB 1,398,421 and GB 1,398,422 and in US 3,936,448, and the sulfonated pyrolysed citrates described in GB 1,082,179, while polycarboxylates containing phosphone substituents are disclosed in GB 1,439,000.

Alicyclic and heterocyclic polycarboxylates include cyclopentane-cis,cis-cis-tetracarboxylates, cyclopentadienide pentacarboxylates, 2,3,4,5-tetrahydro-furan - cis, cis, cis-tetracarboxylates, 2,5-tetrahydro-furan-cis, discarboxylates, 2,2,5,5,-tetrahydrofuran - tetracarboxylates, 1,2,3,4,5,6-hexane - hexacarboxylates and carboxymethyl derivatives of polyhydric alcohols such as sorbitol, mannitol and xylitol. Aromatic polycarboxylates include mellitic acid, pyromellitic acid and the phthalic acid derivatives disclosed in GB 1,425,343. Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

A suitable chelant for inclusion in the detergent compositions in accordance with the invention is ethylenediamine-N,N'-disuccinic acid (EDDS) or the alkali metal, alkaline earth metal, ammonium, or substituted ammonium salts thereof, or mixtures thereof. Preferred EDDS compounds are the free acid form and the sodium or magnesium salt thereof. Examples of such preferred sodium salts of EDDS include $Na_2EDDS$ and $Na_4EDDS$. Examples of such preferred magnesium salts of EDDS include MgEDDS and $Mg_2EDDS$. The magnesium salts are the most preferred for inclusion in compositions in accordance with the invention.

Preferred builder systems include a mixture of a water-insoluble aluminosilicate builder such as zeolite A, and a water soluble carboxylate chelating agent such as citric acid.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated form each other by not more than two carbon atoms.

Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in amounts of from 5% to 80 % by weight of the composition. Preferred levels of builder for liquid detergents are from 5% to 30%.

<u>Enzymes</u>

Preferred detergent compositions, in addition to the enzyme of the invention, comprise other enzyme(s) which provides cleaning performance and/or fabric care benefits. Such enzymes include proteases, lipases, cutinases, amylases, cellulases, peroxidases, oxidases (e.g. laccases).

<u>Proteases:</u> Any protease suitable for use in alkaline solutions can be used. Suitable proteases include those of animal, vegetable or microbial origin. Microbial origin is preferred. Chemically or genetically modified mutants are included. The protease may be a serine protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the <u>Fusarium</u> protease described in WO 89/06270. Preferred commercially available protease enzymes include those sold under the tradenames Alcalase, Savinase, Primase, Durazym, and Esperase by Novo Nordisk A/S (Denmark), those sold under the tradename Maxatase, Maxacal, Maxapem and Properase by Gist-Brocades/Genencor, those sold under the tradename Purafect and Purafect OXP by Genencor International, and those sold under the tradename Opticlean and Optimase by Solvay Enzymes. Protease enzymes may be incorporated into the compositions in accordance with the invention at a level of from 0.0001 % to 2 % of enzyme protein by weight of the composition, in particular at a level of from 0.001 % to 0. 1% of enzyme protein by weight of the composition.

<u>Lipases:</u> Any lipase suitable for use in alkaline solutions can be used. Suitable lipases include those of bacterial or fungal origin and chemically or genetically modified lipase mutants.

Examples of useful lipases include a Humicola lanuginosa lipase, e.g., as described in EP 258 068 and EP 305 216, and mutants thereof as described in WO 92/05249, WO 94/25577 and WO 95/22615 a Rhizomucor miehei lipase, e.g., as described in EP 238 023, a Candida lipase, such as a C. antarctica lipase, e.g., the C. antartica lipase A or B described in EP 214 761, a Pseudomonas lipase such as a P. alcaligenes and P. pseudoalcaligenes lipase, e. g., as described in EP 218 272, or any mutant of said *Pseudomonas* lipases, a P. cepacia lipase, e.g., as described in EP 331 376, a P. stutzeri lipase, e.g., as disclosed in BP 1,372,034, a P. fluorescens lipase, a Bacillus lipase, e.g., a B. subtilis lipase (Dartois et al., (1993), Biochemica et Biophysica acta 1131, 253-260), a B. stearothermophilus lipase (JP 64/744992) and a B. pumilus lipase (WO 91/16422). Furthermore, a number of cloned lipases may be useful, including the Penicillium camembertii lipase described by Yamaguchi et al., (1991), Gene 103, 61-67), the Geotricum candidum lipase (Schimada, Y. et al., (1989), J. Biochem., 106, 383-388), and various Rhizopus lipases such as a R. delemar lipase (Hass, M.J et al., (1991), Gene 109, 117-113), a R. niveus lipase (Kugimiya et al., (1992), Biosci. Biotech. Biochem. 56, 716-719) and a R. oryzae lipase.

Other types of lipolytic enzymes such as cutinases may also be useful, e.g., a cutinase derived from Pseudomonas mendocina as described in WO 88/09367, or a cutinase derived from Fusarium solani pisi (e.g. described in WO 90/09446). Especially suitable lipases are lipases such as M1 Lipase™, Luma fast™ and Lipomax™ (Gist-Brocades/Genencor), Lipolase™ and Lipolase Ultra™ (Novo Nordisk A/S), and Lipase P "Amano" (Amano Pharmaceutical Co. Ltd.).

The lipases are normally incorporated in the detergent composition at a level of from 0.0001 % to 2 % of enzyme protein by weight of the detergent composition, in particular at a level of from 0.001 % to 0.1 % of enzyme protein by weight of the composition.

Amylases: Any amylase (a and/or b) suitable for use in alkaline solutions can be used. Suitable amylases include those of bacterial or fungal origin. Chemically or genetically modified mutants are included. Amylases include, for example, a-amylases obtained from a special strain of B. licheniformis, described in more detail in GB 1,296,839. Commercially available amylases are Duramyl™, Termamyl™, Fungamyl™ and BAN™ (available from Novo Nordisk A/S) and Rapidase™ and Maxamyl P™ (available from (Gist-Brocades/Genencor).

The amylases are normally incorporated in the detergent composition at a level of from 0.0001 % to 2% of enzyme protein by weight of the detergent composition, in particular at a level of from 0.001 % to 0.1 % of enzyme protein by weight of the composition.

Cellulases: Any cellulase suitable for use in alkaline solutions can be used. Suitable cellulases include those of bacterial fungal origin. Chemically of genetically modified mutants are included. Suitable cellulases are disclosed in US 4,435,307, which discloses fungal cellulases produced from Humicola insolens. Especially suitable cellulases are the cellulases having color care benefits. Examples of such cellulases are cellulases described in published European patent application No. 0495257. Commercially available cellulases is Celluzyme™ produced by a strain of Humicola insolens. (Novo Nordisk A/S), and KAC-500(B)™ (Kao Corporation).

Said cellulases are normally incorporated in the detergent composition at a level of from 0.0001 % to 2% of enzyme protein by weight of the detergent composition, in particular at a level of from 0.001 % to 0.1 % of enzyme protein by weight of the composition.

Peroxidases/Oxidases: Peroxidase and/or oxidase enzymes are used in combination with hydrogen peroxide or oxygen sources, e.g., percarbonate, perborate, persulfate, hydrogen peroxide, oxygen, etc. They are used for "Solution bleaching", i.e. to prevent transfer of a textile dye from a dyed fabric to another fabric when said fabrics are washed together in a wash liquor, preferably together with an enhancing agent as described in e.g. WO 94/12621 and WO 95/01426. Suitable peroxidases/oxidases include those of plant, bacterial or fungal origin. Chemically or genetically modified mutants are included.

Said peroxidase and/or oxidase enzymes are normally incorporated in the detergent composition at a level of from 0.0001% to 2 % of enzyme protein by weight of the detergent composition, in particular at a level of from 0.001 % to 0.1 % of enzyme protein by weight of the composition.

Mixtures of the above mentioned enzymes are encompassed herein, in particular a mixture of a protease, an amylase, a lipase and/or a cellulase.

Optional detergent ingredients

Bleaching agents: Additional optional detergent ingredients that can be included in the detergent compositions of the present invention include bleaching agents such as PB1, PB4 and percarbonate with a particle size of 400-800 microns. These bleaching agent components can include one or more oxygen bleaching agents and, depending upon the bleaching agent chosen, one or more bleach activators. When present oxygen bleaching compounds will typically be present at levels of from about 1% about 25%.

The bleaching agent component for use herein can be any of the bleaching agents useful for detergent compositions including oxygen bleaches as well as others known in the art.

The bleaching agent suitable for the present invention can be an activated or non-activated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in US 4,483,781, US 740,446, EP 0 133 354 and US 4,412,934. Highly preferred bleaching agents also include 6-nonylamino-6-oxoperoxycaproic acid as described in US 4,634,551.

Another category of bleaching agents that can be used encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

The hydrogen peroxide releasing agents can be used in combination with bleach activators such as tetraacetylethylenediamine (TAED), nonanoyloxybenzenesulfonate (NOBS, described in US 4,412,934), 3,5-trimethylhexsanoloxybenzenesulfonate (ISONOBS, described in EP 120 591) or pentaacetylglucose (PAG), which are perhydrolyzed to form a peracid as the active bleaching species, leading to improved bleaching effect. In addition, very suitable are the bleach activators C8(6-octanamido-caproyl) oxybenzenesulfonate, C9(6-nonanamido caproyl) oxybenzenesulfonate and C10 (6-decanamido caproyl) oxybenzenesulfonate or mixtures thereof. Also suitable activators are acylated citrate esters such as disclosed in European Patent Application No. 91870207.7.

Useful bleaching agents, including peroxyacids and bleaching systems comprising bleach activators and peroxygen bleaching compounds for use in cleaning compositions according to the invention are described in application USSN 08/136,626.

Imine quaternary salts may used as a bleach catalyst together with a peroxygen compound as described WO 95/13352.

The hydrogen peroxide may also be present by adding an enzymatic system (i.e. an enzyme and a substrate therefore) which is capable of generation hydrogen peroxide at the beginning or during the washing and/or rinsing process. Such enzymatic systems are disclosed in published EP Patent Application No 0537381.

The release of peracid from a peroxygen bleach source may be activated by use of a lipase of the invention. The necessary components for the enzymatic hydrolysis system is the peracid precursor substrate: a diacyl peroxide $R_1$-CO-O-O-CO-R, where R and $R_1$ may be saturated or unsaturated alkyl, an aryl group or an alkaryl. The lipase reacts with the substrate and releases peracids in the wash.

A non-aqeous liquid detergent composition may comprise a peroxyacid material e.g peroxyacids consisting of N,N'-Di(4-Percarboxybenzoyl)ethylenediamine (PCBED), N,N'-Terephthaloyl-di(6-aminopercarboxycaproic acid) (TPCAP), N,N'-Di(4-percarboxybenzoyl)piperazine (PCBPIP), N,N'-Di(4-Percarboxybenzoyl)-1,4-diaminocyclohexane (PCBHEX), N,N'-Di(4-Percarboxybenzoyl)-I,4.butanediamine (PCBBD), N,N'-Di(4-Percarboxyaniline)-terephtalate (DPCAT), N,N,N'N'-1,2,4,5-tetra-carboxybenzoyl-di(6-aminopercarboxycaproic acid) (DiPAP), N,N'-Di(percarboxyadipoyl)phenylenediamine (DPAPD), N,N'-Succinoyl-di(4-percarboxy)aniline (SDPCA), C, analogue of N,N'-Terephaloyl-di-(8-amino peroxyoctanoic acid) (TPOCT) as described in WO 95/06104.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. These materials can be deposited upon the substrate during the washing process. Upon irradiation with light, in the presence of oxygen, such as by hanging clothes out to dry in the daylight, the sulfonated zinc phthalocynaine is activated and, consequently, the substrate is bleached. Preferred zinc phthalocyanine and a photoactivated bleaching process are described in US 4,033,718. Typically, detergent composition will contain about 0.025% to about 1.25%, by weight, of sulfonated zinc phthalocyanine.

Bleaching agents may also comprise a manganese catalyst. The manganese catalyst may, e.g., be one of the compounds described in "Efficient manganese catalysts for low-temperature bleaching", Nature 369, 1994, pp. 637-639.

Suds suppressors: Another optional ingredient is a suds suppressor, exemplified by silicones, and silica-silicone mixtures. Silicones can be generally represented by alkylated polysiloxane materials while silica is normally used in finely divided forms exemplified by silica aerogels and xerogels and hydrophobic silicas of various types. Theses materials can be incorporated as particulates in which the suds suppressor is advantageously releasably incorporated in a water-soluble or waterdispersible, substantially non surface-active detergent impermeable carrier. Alternatively the suds suppressor can be dissolved or dispersed in a liquid carrier and applied by spraying on to one or more of the other components.

A preferred silicone suds controlling agent is disclosed in US 3,933,672. Other particularly useful suds suppressors are the self-emulsifying silicone suds suppressors, described in DE 2,646,126. An example of such a compound is DC-544, commercially available form Dow Corning, which is a siloxane-glycol copolymer. Especially preferred suds controlling agent are the suds suppressor system comprising a mixture of silicone oils and 2-alkyl-alkanols. Suitable 2-alkyl-alkanols are 2-butyl-octanol which are commercially available under the trade name Isofol 12 R.

Such suds suppressor system are described in published European Patent Application No. 0593841.

Especially preferred silicone suds controlling agents are described in published European Patent Application No. 0573699. Said compositions can comprise a silicone/ silica mixture in combination with fumed nonporous silica such as Aerosil$^R$.

The suds suppressors described above are normally employed at levels of from 0.001 % to 2% by weight of the composition, preferably from 0.01% to 1% by weight.

Other components: Other components used in detergent compositions may be employed such as soil-suspending agents, soil-releasing agents, optical brighteners, abrasives, bactericides, tarnish inhibitors, coloring agents, and/or encapsulated or nonencapsulated perfumes.

Especially suitable encapsulating materials are water soluble capsules which consist of a matrix of polysaccharide and polyhydroxy compounds such as described in GB 1,464,616.

Other suitable water soluble encapsulating materials comprise dextrins derived from ungelatinized starch acid esters of substituted dicarboxylic acids such as described in US 3,455,838. These acid-ester dextrins are, preferably, prepared from such starches as waxy maize, waxy sorghum, sago, tapioca and potato. Suitable examples of said encapsulation materials include N-Lok manufactured by National Starch. The N-Lok encapsulating material consists of a modified maize starch and glucose. The starch is modified by adding monofunctional substituted groups such as octenyl succinic acid anhydride.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers, e.g Sokalan CP5, previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10% by weight, more preferably form 0.75% to 8%, most preferably from 1% to 6% by weight of the composition.

Preferred optical brighteners are anionic in character, examples of which are disodium 4,4'-bis-{2-diethanolamino-4-anilino -s- triazin-6-ylamino)stilbene-2:2' disulphonate, disodium 4, - 4'-bis-(2-morpholino-4-anilino-s-triazin-6-ylamino-sdlbene-2:2' - disulphonate, disodium 4,4' - bis-(2,4-dianilino-s-triazin-6-ylamino)stilbene-2:2' - disulphonate, monosodium 4',4" - bis-(2,4-dianilino-s-tri-azin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl)-stilbene-2,2' disulphonate, di-so-dium 4,4'bis(2-anilino-4-(1-methyl-2-hydroxyethylamino)-s-triazin-6-ylami-no)stilbene-2,2'disulphonate, sodium 2(stilbyl-4"-(naphtho-1',2':4,5)-1,2,3, - triazole-2' '-sulphonate and 4,4'-bis(2-sulphostyryl)biphenyl.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

A graft polymer as described in WO 95/22593 may also be used.

Soil release agents useful in compositions of the present invention are conventionally copolymers or terpolymers of terephthalic acid with ethylene glycol and/or propylene glycol units in various arrangements. Examples of such polymers are disclosed in US 4,116,885, US 4,711,730 and EP 0 272 033. A particular preferred polymer in accordance with EP 0 272 033 has the formula:

$$(CH_3(PEG)_{43})_{0.75}(POH)_{0.25}[T\text{-}PO)_{2.8}(T\text{-}PEG)_{0.4}]T(POH)_{0.25}((PEG)_{43}CH_3)_{0.75}$$

where PEG is -$(OC_2H_4)$0-, PO is $(OC_3H_6O)$ and T is (pc$OC_6H_4CO$).

Also very useful are modified polyesters as random copolymers of dimethyl terephthalate, dimethyl sulfoisophthalate, ethylene glycol and 1-2 propane diol, the end groups consisting primarily of sulphobenzoate and secondarily of mono esters of ethylene glycol and/or propane-diol. The target is to obtain a polymer capped at both end by sulphobenzoate groups, "primarily", in the present context most of said copolymers herein will be endcapped by sulphobenzoate groups. However, some copolymers will be less than fully capped, and therefore their end groups may consist of monoester of ethylene glycol and/or propane 1-2 diol, thereof consist "secondarily" of such species.

The selected polyesters herein contain about 46% by weight of dimethyl terephthalic acid, about 16% by weight of propane -1.2 diol, about 10% by weight ethylene gluycol about 13% by weight of dimethyl sulfobenzoic acid and about 15% by weight of sulfoisophthalic acid, and have a molecular weight of about 3.000. The polyesters and their method of preparation are described in detail in EP 311 342.

Softening agents: Fabric softening agents can also be incorporated into laundry detergent compositions in accordance with the present invention. These agents may be inorganic or organic in type. Inorganic softening agents

are exemplified by the smectite clays disclosed in GB-A-1 400898 and in US 5,019,292. Organic fabric softening agents include the water insoluble tertiary amines as disclosed in GB-A 1 514 276 and EP 0 011 340 and their combination with mono $C_{12}$-$C_{14}$ quaternary ammonium salts are disclosed in EP 026 528 and di-long-chain amides as disclosed in EP 0 242 919. Other useful organic ingredients of fabric softening systems include high molecular weight polyethylene oxide materials as disclosed in EP 0 299 575 and 0 313 146.

Levels of smectite clay are normally in the range form 5 % to 15%, more preferably form 8% to 12% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

<u>Polymeric dye transfer inhibiting agents:</u> The detergent compositions according to the present invention may also comprise from 0.001% to 10%, preferably from 0.01% to 2% , more preferably form 0.05% to 1% by weight of polymeric dye transfer inhibiting agents. Said polymeric dye transfer inhibiting agents are normally incorporated into detergent compositions in order to inhibit the transfer of dyes from colored fabrics onto fabrics washed therewith. These polymers have the ability to complex or adsorb the fugitive dyes washed out of dyed fabrics before the dyes have the opportunity to become attached to other articles in the wash.

Especially suitable polymeric dye transfer inhibiting agents are polyamine N-oxide polymers, copolymers of N-vinylpyrrolidone and N-vinylimidazole, polyvinylpyrrolidone polymers, polyvinyloxazolidones and polyvinylimidazoles or mixtures thereof. Addition of such polymers also enhances the performance of the enzymes according the invention.

The detergent composition according to the invention can be in liquid, paste, gels, bars or granular forms. Non-dusting granulates may be produced, e.g., as disclosed in US 4,106,991 and 4,661,452 (both to Novo Industri A/S) and may optionally be coated by methods known in the art. Examples of waxy coating materials are poly(ethylene oxide) products (polyethyleneglycol, PEG) with mean molecular weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GR 1483591.

Granular compositions according to the present invention can also be in "compact form", i.e. they may have a relatively higher density than conventional granular detergents, i.e. form 550 to 950 g/l; in such case, the granular detergent compositions according to the present invention will contain a lower amount of "Inorganic filler salt", compared to conventional granular detergents; typical filler salts are alkaline earth metal salts of sulphates and chlorides, typically sodium sulphate; "Compact" detergent typically comprise not more than 10% filler salt. The liquid compositions according to the present invention can also be in "concentrated form", in such case, the liquid detergent compositions according to the present invention will contain a lower amount of water, compared to conventional liquid detergents. Typically, the water content of the concentrated liquid detergent is less than 30%, more preferably less than 20%, most preferably less than 10% by weight of the detergent compositions.

The compositions of the invention may for example, be formulated as hand and machine laundry detergent compositions including laundry additive compositions and compositions suitable for use in the pretreatment of stained fabrics, rinse added fabric softener compositions, and compositions for use in general household hard surface cleaning operations and dishwashing operations.

Finally the invention relates to a process for washing or cleaning different subject matters using a composition of the invention. By using said composition it is possible more effectively to remove lipid deposits from said subject matters *e.g.* clothes, textiles or dishes.

It is also possible to reduce the amount of lipolytic enzymes which must be present in the detergent composition to obtain the same washing performance as detergent compositions comprising the parent lipolytic enzyme.

In an embodiment of the invention the process comprises the following steps:

a) soaking the subject matter in aqueous medium,
b) contacting for a period of time the subject matter to a detergent composition comprising the modified enzyme with lipolytic activity of the invention dissolved in the aqueous medium before, during or after step a),
c) rinsing the subject matter,
d) removing the rinsing water from the subject matter.

## MATERIALS AND METHODS

### MATERIALS

Plasmids:

pYES 2.0 (Invitrogen Corp., UK)
p960 *A. oryzae* expression plasmid (described in EP 305 216 from Novo Nordisk A/S)
pSX581 (*E. coli* expression plasmid) (see figure 7)
pJSO37 (*S. cerevisiae* expression plasmid) (Okkels J.S., Annals of the New York Academy of Sciences (in press 1995) (see also figure 8)
pSX167 (see figure 4)
pSX92 (WO 89/06279)
pUC19 (Yanish-Perron et al. (1985) Gene 33, 103-119)
pHD414 *(Aspergillus* expression vector being a derivative of the plasmid p775 described in EP 238 023). The construction of pHD414 is further described in WO 93/11249).
PJVi245 (See Figure 9)
pCaHj383 (see Figure 9)
pCaHj385 (see Figure 9)
pAHE2: Hobson, A. H., Buckley, C. M., Aamand, J. L., Jergensen, S. T., Diderichsen, B., and McConnell, D. J. (1993). Activation of a bacterial lipase by its chaperone. Proc. Natl. Acad. Sci. USA, 90, p. 5682-5686).

Micro-organisms:

*Sacchnrorrryces cerevisiae* YNG318: MATa Dpep4[cir$^+$] ura3-52, leu2-D2, his 4-539 *Aspergillus oryzae* IFO 4177

*A. oryzae* JaL 125: *Aspergillus oryzae* IFO 4177 available from Institute for Fermention, Osaka; 17-25 Juso Hammachi 2-Chome Yodogawa-ku, Osaka, Japan, having the alkaline protease gene named "alp" (described by Murakami K et al., (1991), Agric. Biol. Chem. 55, p. 2807-2811) deleted by a one step gene replacement method (described by G. May in "Applied Molecular Genetics of Filamentous Fungi" (1992), p. 1-25. Eds. J. R. Kinghom and G. Turner; Blackie Academic and Professional), using the *A. oryzae* pyrG gene as marker.

*E. coli* W3110 lacI$^q$ *(E. coli* W3110 is an early isolate used as ancestral stock for the K-12 strain (Bachman, (1972), Bacteriol. Rev. 36). The W3110 stain has been made lacI$^q$ in order to overproduce the Lac repressor, turning off expression from plac more completely.

*E. coli* SJ6: Diderichsen, B., Wedsted, U., Hedegaard, L., Jensen, B. R., Sjøholm, C., (1990), Cloning of aldB, which encodes alpha-acetolactate decarboxylase, an exoenzyme from *Bacillus brevis.* J. Bacteriol., 172, p. 4315-4321).

Strain SJ1503 is *E. coli* JA221 containing plasmid pAHE2:
Hobson, A. H., Buckley, C. M., Aamand, J. L., Jørgensen, S. T., Diderichsen, B., and McConnell, D. J. (1993). Activation of a bacterial lipase by its chaperone. Proc. Natl. Acad. Sci. USA, 90, p. 5682-5686.

Donor organisms:

*Humicola lanuginosa* DSM 4109 (EP 305,216)
*Humicola insolens* DSM 1800 (WO 96/13580)
*Pseudomonas cepacia* SB10, DSM 3959, is described in WO 89/01032.

Enzymes:

Bovine trypsin (Boehringer Mannheim)

The following lipases are variants of the *Humicola lanuginosa* DSM 4109 lipase (EP 305 216) which are either used as parent enzymes in the context of the present invention or which constitute modified enzymes according to the invention.

Table M1

| Lipase variants | Peptide addition | Mutations |
|---|---|---|
| HLv1s | SPIRR | D57G, N94K, D96L, L97M |
| HLv1 | - | D57G, N94K, D96L, L97M |
| HLv2s | SPIRR | D137G, D167G, E210V, W221L |
| HLv2 | - | D137G, D167G, E210V, W221L |
| HLv3s | SPIRR | N94K, F95L, D96H, N101S, F181L, D234Y, I2S2L, P256T, G263A, L264Q |
| HLv3 | - | N94K, F95L, D96H, N101S, F181L, D234Y, I252L, P256T, G263A, L264Q |
| HLv4s | SPIRR | I90F, D96L, E99K, V187A |
| HLv4 | - | I90F, D96L, E99K, V187A |
| HLv5s | SPIRR | N94K, D96A, Q249R |
| HLv5 | - | N94K, D96A, Q249R |
| HLv7s | SPIRR | D57G, G59V, N94K, D96L, L97M, S116P, S170P, Q249R |
| HLv7 | - | D57G, G59V, N94K, D96L, L97M, S116P, S170P, Q249R |
| HLv8s | SPIRR | A49P, D167G, E210V |
| HLv8 | - | A49P, D167G, E210V |
| HLv9s | SPIRPRP | D57G N94K, D96L, Q249R |
| HLv9 | - | D57G N94K, D96L, Q249R |
| HLv10s1 | GPIRPRP | D57G, N94K, D96L, L97M, Q249R |
| HLv10s2 | SHSRHNA | D57G, N94K, D96L, L97M, Q249R |
| HLv10s3 | TAIRPRK | D57G, N94K, D96L, L97M, Q249R |
| HLv10s4 | SALRRRP | D57G, N94K, D96L, L97M, Q249R |
| HLv10s5 | STRRPRP | D57G, N94K, D96L, L97M, Q249R |
| HLv10s6 | SPRRPRT | D57G, N94K, D96L, L97M, Q249R |
| HLv10s7 | SPIPPGP | D57G, N94K, D96L, L97M, Q249R |
| HLv10s8 | LPFRQRP | D57G, N94K, D96L, L97M, Q249R |
| HLv10s9 | SPFRPKL | D57G, N94K, D96L, L97M, Q249R |
| HLv10s10 | SALRRP | D57G, N94K, D96L, L97M, Q249R |
| HLv10s11 | SPIRK | D57G, N94K, D96L, L97M, Q249R |
| HLv10s12 | SPIR | D57G, N94K, D96L, L97M, Q249R |
| HLv10 | - | D57G, N94K, D96L, L97M, Q249R |
| HLv11s | SPIRP | E1P, D57G, N94K, D96L, L97M, Q249R |

The following lipases are variants of the *B. cepacia* (formerly *Pseudomonas cepacia)* lipase to which an N-terminal addition has been applied in accordance with the present invention.

Table M2

| Lipase variants | Peptide addition |
|---|---|
| SJ3708 | SPIRR |
| SJ3717 | SPIRPRP |
| SJ3718 | SPIRPRP |

Table M2 (continued)

| Lipase variants | Peptide addition |
|---|---|
| SJ3719 | TAIRPRK |
| SJ3720 | STRRPRP |
| SJ3720 | STRRPRP |
| SJ3721 | GPIRPRP |

The following lipases are variants of the *Humicola insolens* DSM 1800 lipolytic enzyme.

Table M3

| Lipase variants | Peptide addition |
|---|---|
| HILv1s | SPPRRP |
| HILV2s | SPPRP |
| HILv3s | SPIRK |
| HILv4s | PPPRRPR |

Enzyme inhibitor:

Soy bean trypsin inhibitor (Boehringer Mannheim)

Media:

YPD: 10 g yeast extract, 20 g peptone, $H_2O$ to 810 ml. Autoclaved, 90 ml 20% glucose (sterile filtered) added.
LB-medium: 10 g Bacto-tryptone, 5 g Bacto yeast extract, 10 g NaCl in 1 litre water.
FG4 medium: 3% soy meal, 3% maltodextrin, 1% peptone, pH adjusted to 7.0 with 4 M NaOH
Litex Agarose HSB 2000 (CAT NO: F90472)
BG-reagent: 4 mg/ml Brilliant Green (BG) dissolved in water
Substrate 1:

10 ml Olive oil (Sigma CAT NO. 0-1500)
20 ml 2 % polyvinyl alcohol (PVA)

The Substrate is homogenised for 15-20 minutes.

| PCS detergent | |
|---|---|
| 10 g/l: | |
| SDS | 0.52 g |
| Dobanol 25-3 | 0.60 g |
| Dobanol 25-7 | 0.58 g |
| $NaBO_3H_2O$ | 1.50 g |

Ad 1 litre 0.1 M Tris buffer (pH 9), and dilute further with the Tris buffer to the double concentration of the desired concentration of the PCS plates.

PCS-plates

Solution for making PCS plates

| | |
|---|---|
| Brilliant Green (BG-reagent) | 10 ml |

(continued)

| | |
|---|---|
| Substrate 1 | 24 ml |
| PCS detergent | 500 ml |
| 2% agarose (in TRIS buffer (pH 9) | 500 ml |

Lipase Substrate (Sigma catalogue no. 800-1)
Brilliant Green (Merck, art. No. 1.01310)

Swatches:

3.5 x 3.5 cm and 9x9 cm cotton swatches (style #400 from TestFabrics, Inc. (New Jersey) stained with lard/sudan red
Lard: Lard coloured with 0.75 mg sudan red/gram lard.

| Detergent I: | |
|---|---|
| 1.17 g/l | LAS (Nansa 1169/P, 30% a.m.) |
| 0.15 g/l | AEO (Dobanol 25-7) |
| 1.25 g/l | Sodium triphosphate |
| 1.00 g/l | Sodium sulphate |
| 0.45 g/l | Sodium carbonate |
| 0.15 g/l | Sodium silicate |
| The pH adjusted to 10 | |

Inactivated Ariel Futur (Procter and Gamble) (commercially available batch No.4279 B 23:35): The enzymes in the detergent were inactivated by heat (4 minutes at 85°C in microoven).
Water: 3.2 mM $Ca^{2++}$/$Mg^{2++}$ (5:1)
Chameleon double-stranded, site directed mutagenesis kit (cat. no. 200509) (Stratagene, Lajolle, CA)

Equipment:

473A Protein Sequencer (Applied Biosystems)
Toyopearl Butyl column (XK 16/10) (Pharmacia, Sweden)
Q-Sepharose column (HPQ XK 26/10) (Pharmacia, Sweden)
MonoQ column (1 ml) (Pharmacia, Sweden)
Highperformance Q SeparoseÔ (Pharmacia, Sweden)
Spin100 column (Clontech Lab. Inc., CA, USA)

METHODS:

Hybridization conditions

Medium to high stringency

Presoaking in 5X SSC and prehydbridizing for 1 hour at about 40°C in a solution of 20% formamide, 5X Denhardt's solution, 50 mM sodium phosphate, pH 6.8, and 50 mg denatured sonicated calf thymus DNA, followed by hybridization in the same solution supplemented with 100 mM ATP for 18 hours at about 40°C, followed by a wash in 0.4X SSC at a temperature of about 45°C.

Construction of yeast exrpession vector

The expression plasmid pJSO37, is derived from pYES 2.0. The inducible GAL1-promoter of pYES 2.0 was replaced with the constitutively expressed TPI (triose phosphate isomerase)-promoter from *Saccharomyces cerevisiae* (Albert and Karwasaki, (1982), J. Mol. Appl Genet., 1, 419-434), and the URA3 promoter has been deleted. A restriction

map of pJSO37 is shown in figure 8.

Method for constructing lipolytic variants

The peptide addition and/or mutations in the non-structural N-terminal and/or C-terminal end of the parent lipolytic enzyme to construct modified lipolytic enzymes of the invention were performed either by site-directed mutagenesis of by random mutagenesis.

Site-directed mutagenesis

For the construction of variants of a *H. lanuginosa* lipolytic enzyme the commercial kit, Chameleon double-stranded, site-directed mutagenesis kit can be used according to the manufacturer's instructions.

The gene encoding the lipolytic enzyme in question is inserted into the plasmid pHD414. In accordance with the manufacturer's instructions the ScaI site of the Ampicillin gene of pHD414 is changed to a MluI site by use of the following primer:

Primer 3: AGAAATCGGGTATCCTTTCAG (SEQ ID No. 6)

The pHD414 vector comprising the lipolytic gene in question is then used as a template for DNA polymerase and oligos 7258 and 7770. The desired mutation *(e.g.* in the N-terminal of the lipolytic gene) is introduced into the lipolytic gene in question by addition of an appropriate oligos comprising the desired mutation.

PCR reactions are performed according to the manufacturer's recomendations.

Random mutagenesis

May be performed essentially as described in WO 95/22615. More specifically, for performing random mutagenesis in short DNA stretches such as in the peptide addition, the random mutagenesis is performed by use of doped or spiked oligonucleotide probes. For larger DNA stretches PCR generated mutagenesis may be used.

Low calcium filter assay

Procedure

1) Provide SC Ura replica plates (useful for selecting strains carrying an expression vector) with a first protein binding filter (Nylon membrane) and a second low protein binding filter (Cellulose acetate) on the top.
2) Spread yeast cells containing a parent lipase gene or a mutated lipase gene on the double filter and incubate for 2 or 3 days at 30°C.
3) Keep the colonies on the top filter by transferring the topfilter to a new plate.
4) Remove the protein binding filter to an empty petri dish.
5) Pour an agarose solution comprising an olive oil emulsion (2% P.V.A.: Olive oil = 3:1), Brilliant green (indicator, 0.004%), 100 mM tris buffer pH9 and EGTA (final concentration 5mM) on the bottom filter so as to identify colonies expressing lipase activity in the form of blue-green spots.
6) Identify colonies found in step 5) having a reduced dependency for calcium as compared to the parent lipase.

Dobanol®25-7 filter assay:

The screening for an improved tolerance towards a detergent component is performed by use of a filter assay corresponding to that described above except for the fact that the solution defined in 5) further comprises 0.02% Dobanol®25-7 and optionally without any EGTA.

An alternative screening assay is the following:

Procedure

1) Provide SC Ura-plates (useful for selecting strains carrying an expression vector) with a protein binding filter (Cellulose acetate) on the top.
2) Spread yeast cells containing a parent lipase gene or a mutated lipase gene on the filter and incubate for 3 or 4 days at 30°C.
3) Keep the colonies on the top filter by transferring the topfilter to a new plate.
4) Remove the protein binding filter to a petri dish containing:
An agarose solution comprising an olive oil emulsion (2% P.V.A.:Olive oil=2:1), Brilliant green (indicator,0.004%), 100 mM tris buffer pH10 and the detergent or detergent component, e.g. PCS-plates.
5) Identify colonies expressing lipase activity in the form of blue-green spots found in step 4)

Fermentation in yeast

10 ml of SC-ura medium are inoculated with a *S. cerevisiae* colony and grown at 30°C for 2 days. The resulting 10 ml culture is used for inoculating a shake flask containing 300 ml SC-ura medium which is grown at 30°C for 3 days. The 300 ml is used for inoculation 5 litre of the following G-substrate:

| | |
|---|---|
| 400 g | Amicase |
| 6.7 g | yeast extract (Difco) |
| 12.5 g | L-Leucin (Fluka) |
| 6.7 g | $(NH_4)_2SO_4$ |
| 10 g | $MgSO_4$ $7H_2O$ |
| 17 g | $K_2SO_4$ |
| 10 ml | Tracecompounds |
| 5 ml | Vitamin solution |
| 6.7 ml | $H_3PO_4$ |
| 25 ml | 20% Pluronic (antifoam) |

In a total volume of 5000 ml:

The yeast cells are fermented for 5 days at 30°C. They are given a start dosage of 100 ml 70% glucose and added 400 ml 70% glucose/day. A pH=5.0 is kept by addition of a 10% $NH_3$ solution. Agitation is 300 rpm for the first 22 hours followed by 900 rpm for the rest of the fermentation. Air is given with 1l air/l/min for the first 22 hours followed by 1.5 l air/l/min for the rest of the fermentation.

Trace compounds: 6.8 g of $ZnCl_2$. 54.0 g of $FeCl_2 \cdot 6H_2O$, 19.1 g of $MnCl_2 \cdot 4H_2O$, 2.2 g of $CuSO_4 \cdot 5H_2O$, 2.58 g of $CaCl_2$, 0.62 g of $H_3BO_3$, 0.024 g of $(NH_4)_6Mo_7O_{24} \cdot 4H_2O$, 0.2 g of KI, 100 ml of HCl (concentrated), in a total volume of 1 l.

Vitamin solution: 250 mg of Biotin, 3 g of Thiamin, 10 g of D-Calciumpanthetonate, 100 g of Myo-Inositol. 50 g of Cholinchlorid, 1.6 g of Pyridoxin, 1.2 g of Niacinamid, 0.4 g of Folicacid, 0.4 g of Riboflavin. In a total volume of 1 litre.

Transformation of *Aspergillus oryzae* (general procedure)

100 ml of YPD (Sherman et al., (1981), Methods in Yeast Genetics, Cold Spring Harbor Laboratory) are inoculated with spores of *A. oryzae* and incubated with shaking for about 24 hours. The mycelium is harvested by filtration through miracloth and washed with 200 ml of 0.6 M $MgSO_4$. The mycelium is suspended in 15 ml of 1.2 M $MgSO_4$, 10 mM $NaH_2PO_4$, pH 5.8. The suspension is cooled on ice and 1 ml of buffer containing 120 mg of Novozym* 234, batch 1687 is added. After 5 min., 1 ml of 12 mg/ml BSA (Sigma type H25) is added and incubation with gentle agitation continued for 1.5-2.5 hours at 37°C until a large number of protoplasts is visible in a sample inspected under the microscope.

The suspension is filtered through miracloth, the filtrate transferred to a sterile tube and overlayed wit 5 ml of 0.6 M sorbitol, 100 mM Tris-HCl, pH 7.0. Centrifugation is performed for 15 min. at 1000 g and the protoplasts are collected from the top of the $MgSO_4$ cushion. 2 volumes of STC (1.2 M sorbitol, 10 mM Tris-HCl, pH 7.5, 10 mM $CaCl_2$) are added to the protoplast suspension and the mixture is centrifugated for 5 min. at 1000 g. The protoplast pellet is

resuspended in 3 ml of STC and repelleted. This is repeated. Finally, the protoplasts are resuspended in 0.2-1 ml of STC.

100 µl of protoplast suspension are mixed with 5-25 µg of p3SR2 (an *A. nidulans* amdS gene carrying plasmid described in Hynes et al., Mol. and Cel. Biol., Vol. 3, No. 8, 1430-1439, Aug. 1983) in 10 µl of STC. The mixture is left at room temperature for 25 min. 0.2 ml of 60% PEG 4000 (BDH 29576), 10 mM $CaCl_2$ and 10 mM Tris-HCl, pH 7.5 is added and carefully mixed (twice) and finally 0.85 ml of the same solution are added and carefully mixed. The mixture is left at room temperature for 25 min., spun at 2.500 g for 15 min. and the pellet is resuspended in 2 ml of 1.2M sorbitol. After one more sedimentation the protoplasts are spread on minimal plates (Cove, (1966), Biochem. Biophys. Acta 113, 51-56) containing 1.0 M sucrose, pH 7.0, 10 mM acetamide as nitrogen source and 20 mM CsCl to inhibit background growth. After incubation for 4-7 days at 37°C spores are picked, suspended in sterile water and spread for single colonies. This procedure is repeated and spores of a single colony after the second re-isolation are stored as a defined transformant.

Fed batch fermentation

Fed batch fermentation is performed in a medium comprising maltodextrin as a carbon source, urea as a nitrogen source and yeast extract. The fed batch fermentation was performed by inoculating a shake flask culture of A. *oryzae* host cells in question into a medium comprising 3.5 % of the carbon source and 0.5 % of the nitrogen source. After 24 hours of cultivation at pH 5.0 and 34°C the continuous supply of additional carbon and nitrogen sources are initiated. The carbon source is kept as the limiting factor and it is secured that oxygen is present in excess. The fed batch cultivation is continued for 4 days, after which the enzymes can be recovered by centrifugation, ultrafiltration, clear filtration and germ filtration. Further purification may be done by anionexchange chromatographic methods known in the art.

Lipase activity (LU - Lipase Units)

Lipase activity is assayed using glycerine tributyrate as a substrate and gum-arabic as an emulsifier. 1 LU (Lipase Unit) is the amount of enzyme which liberates 1 µmol titratable butyric acid per minute at 30°C, pH 7.0. The lipase activity is assayed by pH-stat using Radiometer titrator VIT90, Radiometer, Copenhagen.

Application of lard on the swatches

6 ml of stained lard heated to 74°C are applied to the canter of each swatch. After application of the stain the swatches are heated in an oven for 25 minutes at 75°C and stored overnight at room temperature prior to the first wash.

3-cycle wash performance

The 3-cycle wash performance of a modified lipolytic enzyme of the invention can be evaluated on the basis of the enzyme dosage in mg of protein (or LU) per litre compared to the parent lipolytic enzyme. Wash trials are carried out in 150 ml beakers placed in a thermostated water bath. The beakers are stirred with triangular magnetic rods.

The experimental conditions are as follows:

| | |
|---|---|
| Method | 3 cycles with overnight drying between each cycle |
| Wash liquor | 100 ml per beaker |
| Switches | 6 swatches (3.5 x 3.5 cm, stained with lard coloured with 0.75 mg sudan red/gram of lard) per beaker |
| Detergent | Detergent I, pH adjusted to 10.2 |
| Enzyme conc. | 0.075, 0.188, 0.375, 0.75 and 2.5 mg of lipase protein per litre |
| Time | 20 minutes |
| Temperature | 30°C |
| Rinse | 15 minutes in running tap water |
| Drying | overnight at room temperature (-20°C, 30-50% RH) |
| Evaluation: | after the 3rd wash, the reflectance at 460 nm was measured. |

Evaluations of wash results

Dose-response curves are compared for the modified lipolytic enzyme and the parent lipolytic enzyme. The

dose-response curves is calculated by fitting the measured data to the following equation:

$$DR = DR_{max} \frac{C^{0.5}}{K + C^{0.5}} \qquad (I)$$

where

DR is the effect expressed in reflectance units
C is the enzyme concentration (mg/l)
$DR_{max}$ is a constant expressing the maximum effect
K is a constant; $K^2$ expresses the enzyme concentration at which half of the maximum effect is obtained.

Based on the characteristic constants $DR_{max}$ and K found for each modified lipolytic enzyme as well as the parent lipolytic enzyme, improvement factors are calculated. The improvement factor, defined as

$$f_{improve} = C_{parent}/C \qquad (II)$$

expresses the amount of modified lipase protein needed to obtain the same effect as that obtained with 0.25 mg/l of the reference parent protein ($C_{parent}$).

Thus, the procedure for calculating the improvement factor is as follows:

1) The effect of the parent protein at 0.25 mg/l ($DR_{parent}$) was calculated by means of equation (I);
2) the concentration of the modified lipolytic enzyme resulting in the same effect as the parent enzyme at 0.25 mg/l was calculated by means of the following equation:

$$C = (K_{(modify)} \frac{DR_{(parent)}}{DR_{max(modify)} - DR_{(parent)}})^2 \qquad (III)$$

3) the improvement factor was calculated by means of equation (II).

<u>1 cycle wash performance</u>

1 cycle wash trials are carried out in a termostated Terg-O-to-Meter (TOM).

| | |
|---|---|
| Method: | 1 cycle wash followed by linedrying. |
| Wash liquor | 1000 ml per beaker |
| Swatches | 7 swatches (9 x 9 cm, stained with lard coloured with 0.75 mg sudan red/gram of lard) |
| Water | 3.2 mM $Ca^{2+}/Mg^{2+}$(5:1) |
| Detergent | 5 g/l inactivated Ariel Futur™. Natural pH around 10.3. (commercially available batch No. 4279 B 23:35). |
| Lipase concentrations | 0, 1250, 12500 LU/l |
| Time | 20 minutes |
| Temperature | 30°C |
| Rinse | 15 minutes in running tap water. |
| Drying | Overnight at room temperature (~ 20°C. 30-40 % RH). |
| Evaluation | The fatty matter is extracted using the soxhlet method and the amount of fany matter is gravimetrically determined. |

**EXAMPLES**

**EXAMPLE 1**

<u>Production of wildtype *Humicola lanuginosa* lipase in yeast</u>

For expression *Humicola lanuginosa* lipase in the yeast *Saccharomyces cerevisiae* YNG318 the yeast expression vector pJSO37 (see figure 8) was constructed as described in the Material and Methods section above. pJSO37 comprises the DNA sequence encoding the parent lipase and includes the DNA sequences encoding the signal peptide and propeptide (see figure 1). The plasmid was transformed into the yeast by standard methods (cf. Sambrooks et al., (1989), Molecular Cloning: A Laboratory Manual, 2nd Ed., Cold Spring Harbor). The yeast was cultivated as described in the Material and Methods section above.

Purification of *H. lanuginosa* lipase expressed in *S. cerevisiae*

Ammonium acetate (92 g) was added to the fermentation broth (1400 ml) to give a 0.8 M solution of ammonium acetate. The solution was added onto a Toyopearl Butyl column (XK 16/10). The column was washed with 0.8 M amonium acetate and the lipase eluted in $H_2O$ at a flow rate of 5 ml/min. 10 ml fractions were collected and lipase containing fractions were pooled according to activity in the standard lipase titration assay. The lipase containing pooled according to activity in the standard lipase titration assay. The lipase containing pool were filtered and the pH was adjusted to pH 7.6 and added onto a Q-Sepharose column (HPQ XK 26/10). The column was washed with 200 ml 0.1 M Tris-HCl, pH 7.25 and the lipase was eluted in a linear gradient of 0 to 0.3 M NaCl in 400 ml of 0.1 M Tris-HCl, pH 7.25 at a flow rate of 5 ml/min. 10 ml fractions were collected and the lipase containing fractions were pooled according to activity in the standard lipase titration assay. The lipase containing pool was diluted with $H_2O$ and added onto a 1 ml MonoQ column at a flow rate of 1 ml/min. The column was washed with 30 ml of $H_2O$ and the lipase was eluted in linear gradient of 0 to 0.25 M NaCl in 40 ml. The lipase was manually collected according to absorption at 280 nm.

N-terminal amino acid sequencing of *H. lanuginosa* lipase expressed in yeast

The N-terminal amino acid sequencing was conducted on the *S. cerevisiae* expressed lipase using the 473A Protein Sequencer according to the manufacturer's instructions.
When the N-terminal amino acid sequence of *S. cerevisiae* expressed lipase is compared to the N-terminal amino acid sequence of the same lipase expressed in *A. oryzae* (as described in EP 305 216) a difference was observed, as the major part of the *S. cerevisiae* expressed enzyme contains 5 amino acid residues extra (SPIRR-) at the N-terminus (see Table E1) which includes the corresponding information for the *A. oryzae* expressed lipase.

Table E1

| Expression system | Fraction containing SPIRR-EVSQ... | Fraction containing EVSQ... |
|---|---|---|
| *S. cerevisiae* | 75 % | 25 % |
| *A. oryzae* | 0 % | 100 % |

As can be seen from the table a major portion of the secreted lipase expressed in *S. cerevisiae* has been extended by the five amino acid SPIRR (from the pro-peptide). The relative amount of enzyme containing the extra amino acid residues can be established from the yields of PTH-amino acids in amino acid sequencing.

## EXAMPLE 2

Removal of the SPIRR-peptide from the N-terminus of *the H. lanuginosa* lipase expressed in *S. cerevisiae*

To 4.5 mg of the above purified modified ("SPIRR"-containing) lipase expressed in *S. cerevisiae* (in 1.8 ml 0.05 M $NH_4HCO_3$) was added 50 mg bovine trypsin (sequencing grade) and the mixture was incubated for 1 hour at 37°C. Upon incubation the tryptic digest was stopped by adding more than 50 mg soy bean trypsin inhibitor.
The removal of the N-terminal SPIRR-peptide addition was observed by N-terminal amino acid sequencing where the fraction containing SPIRR was reduced from 75 % to 13 % (See Table E2).

Table E2

| Treatnemt | Fraction containing SPIRR-EVSQ... | Fraction containing EVSQ.... |
|---|---|---|
| Untreated (*i.e.* modified lipase) | 75 % | 25 % |
| Trypsin treatment | 13 % | 87 % |

The mild uypsm treatment did not result in internal cleavages in the modified lipase as no internal amino acid sequences were observed by amino acid sequencing. Also the specific activity of the trypsin treated lipase was comparable to specific activity of the untreated lipase showing that the trypsin treatment did not affect enzyme activity in the standard assay (See Table E3).

Table E3

| Sample | $A_{280}$ | $A_{280}/A_{260}$ | Activity (LU/ml) | Specific Activity (LU/$A_{280}$) |
|---|---|---|---|---|
| Untreated (*i.e.* modified lipase) | 2.5 | 1.8 | 9725 | 3890 |
| Trypsin treated | 2.2 | 1.8 | 9163 | 4127 |

**EXAMPLE 3**

Production of wildtype *H. lanuginosa* lipase in *Aspergillus oryzae*

Cloning of *Humicola lanuginosa* lipase is described in EP 305,216, which also describes expression and characterization of the lipase in *Aspergillus oryzae* by use of the expression plasmid p960. Said plasmid comprises the *Humicola lanuginosa* lipase encoding gene and the DNA sequence encoding the SPIRR peptide addition (which is a part of the propeptide coding part of the lipase gene). The expression of the wildtype lipase in *A. oryzae* (strain IFO 4177) was done essentially as described in WO 95/22615 using an expression plasmid slightly modified as compared to p960 - cf WO 95/22615.

Purification of wildtype *H. lanuginosa* lipase expressed in *A. oryzae*

Fermentation supernatant from *Aspergillus oryzae* IFO 4177 was centrifuged and cell debris discarded. The supernatant was filtered though a 0.45 m millipore filter.

Then the supernatant was precipitated with 60% saturated ammonium sulphate. The precipitate was dissolved in water and solid ammonium acetate was added to a final concentration of 0.8 M. The solution was applied onto a Butyl Toyopearl column which was pre-equilibrated with 0.8 M ammonium acetate. The bound enzyme was eluted with gradient using water and 50% ethanol as eluent.

Fractions containing enzyme activity are then pooled and conductance was adjusted too it is lower than 5 mSi and pH is adjusted to 7.5.

The pools containing activity were then applied onto an anion exchange column (e.g. Highperformance Q Separose®) which had been pre-equilibrated with 25 mM Tris-acetate buffer, pH 7.5. The bound activity was eluted with linear salt gradient using same buffer and 0.5 M sodium chloride. Fractions containing high lipase activity were pooled.

**EXAMPLE 4**

Construction of parent *Humicola lanuginosa* lipase expression vector and expression in *E. coli*

pSX92 (see figure 4) was cut with Hind III, blunt ended with Klenow polymerase and then cut with ClaI. The large fragment was isolated (A). pHLL (see EP 305,216 figure 3 and 4) (comprising the DNA sequence encoding the parent lipase) was cut with BamHI, blunt ended, and cut with XhoII. The fragment containing the mature part of the modified lipase gene was isolated (B).

A and B were ligated together with a synthetic linker (KFN 575/576) which codes for the last 5 amino acids in the subtilisin 309 signal fused to the first four amino acids of the mature lipase. The last nucleotide "A" in the upper strand changed the XhoII site in the mature lipase gene to a Bgl II site.

Synthetic linker:

KFN 575/576: 5'- CGATCGCATCGGCTGCTGAGGTCTCGCAA-3'

3-TAGCGTAGCCGACGACTCCAGAGGCTTCTAG-5'

The resulting plasmid (pSX167) comprised the DNA sequence encoding the mature lipase. pSX167 was cut with Pme I and Bam H1 and the fragment containing the subtilisin 309 signal sequence-lipase fusion and the 5S

terminator was isolated (1769 bp). This fragment was ligated into Hinc II-Bam H1 cut pUC19 creating pSX578.

DNA coding for mature lipase down to Bst XI (from pSX167, 654 bp) was fused to the *Achronwbacter lyticus* protease I signal sequence (see figure 3) from Sph I using the PCR technique "Splicing by Overlap Extension", Horton et al., (1989), Gene).

Plasmid (pSX578) (see figure 5) was cut with Sph I and Bst XI and the above mentioned PCR DNA was inserted (figure 6). The resulting plasmid pSX581 (see figure 7) was transformed into *E. coli* W3110 lacIq. When grown in shake flasks for 72 hours in LB-medium containing 0.4% lactose at 30°C the resulting strain produces non-glycosylated lipase with the same specific activity as the normal glycosylated parent lipase enzyme.

**EXAMPLE 5**

Construction of *H. lanuginosa* lipase with peptide addition in *E. coli*

The pSX581 plasmid (see figure 7) was digested with BglII/HindIII and the vector fragment was purified from an agarose gel using standard methods.
A PCR reaction was performed with the following primers using pSX581 as template:

**SPIRR primer; Primer 1 (SEQ ID NO 3):**

**5'- AA CAG ATC TTG CGA GAC CTC TCT ACG TAT AGG GCT AGC GAG CGC GGC GCT GAT CG -3' (55-mer)**

**PCR primer; Primer 2 (SEQ ID NO 4):**

**GTTGTGTGGAATTGTGAGCGG (21-mer)**

The resulting 300 bp fragment was purified on Spin 100 columns and digested with BglII/HindIII and again spin 100 purified. This fragment was ligated to the above vector fragment. The resulting plasmid was named pJSO215 and used to transform *E.coli* W3110 lacIq. A plasmid preparation was made from a transformant and DNA sequenced to verify the introduction of the SPIRR peptide addition.

**EXAMPLE 6**

Construction and expression of modified *H. lanuginosa* lipolytic enzyme (HLv9s) in *Aspergillus oryzae* JaL125

An N-terminal peptide addition was applied to the parent *H. lanuginosa* (DSM 4109) lipolytic enzyme having the amino acid and DNA sequence, respectively, apparent from EP 305 216, and in addition carrying the following mutations D57G, N94K, D96L, Q249R in its mature part (inserted by conventional site-directed mutagenesis) in the DNA sequence (EP 305 216). The peptide addition SPIRPRP was applied to the N-terminus of the parent enzymes as follows:

Construction of pIVI220:

The plasmid was constructed using the Chamelon double stranded, site-directed mutagenesis kit from Stratagene according to the described protocol.

pHL296 was used as the plasmid template. Said plasmid contains the gene encoding the *H. lanuginosa* lipolytic enzyme with the above mentioned mutations (D57G, N94K, D96L, L97M, Q249R) cloned into pHD464.

Primer no. 7258 was used as the selection primer.

**7258: 5'p gaa tga ctt ggt tga cgc gtc acc agt cac 3' (SEQ ID NO. 79)**

(Thus changing the ScaI site found in the ampicillin resistance gene and used for cutting to a MluI site).

Primer no. 7770 was used as the selection primer.

7770: 5'p tct agc cca gaa tac tgg atc aaa tc 3' (**SEQ ID NO. 80**) (Changes the ScaI site found in the H. *lanuginosa*

lipase gene without changing the amino acid sequence).

Primer no. 8479 was used as the mutagenic primer.

8479: 5'p gcg tgg acg gcc ttg gct age cct att cgt cct cga ccg gtc tcg cag gat ctg 3 (**SEQ ID NO 81**) (replacing the propeptide and the N-terminal E1 of the parent *H. lanuginosa* enzyme (SPIRRE by SPIRPRP).

Construction of pIVI245:

The plasmid was constructed using the Chameleon double-stranded, site-directed mutagenesis kit from Stratagene (cat no. 200509) according to the described protocol.

pIVI220 was used as the plasmid templated and primer no.7887 as the selection primer (changing the introduced Mlu1 site found in the ampicillin resistance gene and used for cutting to a SeaI site). 7887: 5'p-gaa tga ctt ggt tga gta etc acc agt cac 3' (**SEQ ID NO. 77**).

Primer no. 8932 was used as the mutagenic primer (8932: 5'p-g aac tgg ata gga aat ttg aag ttc ctg ttg aaa gaa ata aat gac 3' (**SEQ ID NO. 78**) (thus changing M97 back to L97 as wildtype and still preserving the two mutations N94K and D96L)).

2. Construction of the *A. oryzae* expression plasmid pCaHj483

pCaHj483 is depicted in Figure 9. It is build from the following fragments :

a) The vector pToC65 (WO91/17243) cut with *EcoRI* and *XbaI.*

b) A 2.7 kb *XbaI* fragment from *A. nidulans* carrying the *amdS* gene (C. M. Corrick et al., (1987), Gene 53, p. 63-71). The *amdS* gene is used as a selective marker in fungal transformations. The *amdS* gene has been modified so that the *BamHI* site normally present in the gene is destroyed. This has been done by introducing a silent point mutation using Primer 3: AGAAATCGGGTATCCTTTCAG (SEQ ID No. 6)

c) A 0.6 kb *EcoRI*/*BamHI* fragment carrying the *A. niger* NA2 promoter fused to a 60bp DNA fragment of the sequence encoding the 5' untranslated end of the mRNA of the A. *nidulans tpi* gene. The NA2 promoter was isolated from the plasmid pNA2 (EP 383 779) and fused to the 60 bp *tpi* sequence by PCR. The primer (Primer 4 SEQ ID No. 14) encoding the 60 bp *tpi* sequence had the following sequence : 5'-GCTCCTCATGGTGGATCCCCAGTTGTGTATATAGAGGATTGAGGAAGGAAGAG AAGTGTGGATAGAGGTAAATTGAGTTGGAAACTCCAAGCATGGCATCCTTGC - 3'

d) A 675 bp *XbaI* fragment carrying the *A. niger* glucoamylase transcription terminator. The fragment was isolated from the plasmid pICAMG/Term (EP 238 023).

The *BamHI* site of fragment c) was connected to the *XbaI* site in front bf the transcription terminator on fragment d) via the pIC19R linker (*BamHI* to *XbaI*)

Construction of the HLv9s expression plasmid pCaHj485

The plasmid pJVi 245 was digested with BamH I and Sal I, and the resulting 904 bp fragment encoding the HLv9s lipolytic enzyme was isolated. pCaHj 483 was digested with BamH I and Sal I, and the large vector fragment (6757) was ligated to the HLv9s fragment. The ligation mixture was used to transform *E. coli* DH5$\alpha$ cells, and a transformant harbouring the expected plasmid was isolated. The plasmid was termed pCaHj485.

3. Transformation of pCaHj 485 into JaL125

*Aspergillus oryzae* JaL 125 is Aspergillus oryzae IFO 4177 deleted in the alkaline protease was transformed with pCaHj 485 using selection on acetamide as described in patent EP 0 531 372. Transformants were spore reisolated twice. Spores from second reisolation of each transformant were used to inoculate 200 µl YPM (1% yeast extract, 2% peptone, 2% maltose) in 96 well microtiter dishes. The YPM cultures were grown for 4 days at 34°C, and the higest producers were selected using a p-nitro phenylbutyrate assay:

Stock solution: 18 µl p nitro phenyl butyrate was dissolved in 1 ml isopropanol.

Working solution: 0.1 ml stock solution was mixed with 10 ml 50 mM Tris/HCl pH 7.5; 10 mM $CaCl_2$.

Assay: 1 µl of YPM supernatant was mixed with 200 µl of working solution in 96 well microtiterdishes, and the color development was measured at 450 nm using an ELISA reader.

One transformant was selected for tank fermentation.

4. Tank fermentation of JaL 125/pcaHj 485

The fermentation was carried out as a fed-batch fermentation using a constant medium temperature of 34°C and a start volume of 1.2 litre. The initial pH of the medium was set to 6.5. Once the pH had increased to 7.0 this value was maintained through addition of 10% $H_3PO_4$. The level of dissolved oxygen in the medium was controlled by varying the agitation rate and using a fixed aeration rate of 1.0 litre air per litre medium per minute. The feed addition rate was maintained at a constant level during the entire fed-batch phase.

The batch medium contained maltose syrup as carbon source, urea and yeast extract as nitrogen source and a mixture of trace metals and salts. The feed added continuously during the fed-batch phase contained maltose syrup as carbon source whereas yeast extract and urea were added in order to assure a sufficient supply of nitrogen.

5. Purification of the modified lipolytic enzyme

1) Fermentation supernatant was filtered through miliipore filter Cat. No. AP2504700 Filter type AP25.
2) Fermentation supernatant was filtered once more on through the sterile filter from Millipore membrane Type GS 0.22 micron.
3) Fermenation supernatent was then adjusted to 0.8 M ammonium acetate by adding solid ammonium acetate.
4) A Hydrophobic chromatography on TSK gel Butyl-Toyopearl 650. 50 ml column was packed with the Butyl-Toyopearl matrix. The column was washed and equilibrated with 0.8 M ammonium acetate. One liter ferementation supematent adjusted with amonium acetate was then applied on the Butyl column. The column was washed with 0.8 M ammonium acetate till all unbound material was washed out. Bound material was then eluted with water and 50 % ethanol sequentially. Fractions were collected and analyzed for lipase activity using Standard LU assay. Fractions containing lipase activity were pooled and diluted to adjust conductivity of the pool below 4 mSi and pH to 8.5.
5) Anion exchange chromatography on High Performance Q sepharose (Pharmacia, Code No. 17-1014-01). 50 ml column was packed and washed with 50 mM Borate buffer pH 8.5. Pool conatining lipase activity was then appllied on The High performance Q sepharose column. Unbound material was washed with the Borate buffer pH 8.5. Bound activity was then eluted with linear gradient using Borate buffer conatining 1 M Sodium Chloride pH 8.5. Fractions were collected and assayed for Lipase activity. Fractions containing Lipase activity with a ratio of UV absorbence at A280and A260 more than 1.7 are pooled.

**EXAMPLE 7**

3-cycle wash performance of H. lanuginosa lipase with a peptide addition

The wash performance of the *Humicola lanuginosa* lipase described in EP 305 216 and variants thereof (i.e. modified lipolytic enzymes of the invention) was tested using the 3-cycle wash performance test (described in the Materials and Methods section above) on swatches of cotton soiled with lard stained with red colour in a model wash system at 30°C, at a constant temperature for 20 minutes. The tests were performed at lipase concentrations of 0, 0.075, 0.188, 0.375, 0.750, 2.500 mg enzyme protein per litre.

4.2 g/l of a European type powder detergent composition (as described above) were used in the test. The detergent did not contain any enzymes prior to the addition of the modified lipase of the invention. The detergent was dissolved in approximately 18°dH (German Hardness) water. The pH of the wash liquor was about 10. Six swatches were washed in 100 ml of wash liquor. Subsequent to the washing, the swatches were flushed in running tap water for 15 minutes and then air-dried overnight at room temperature.

3 wash cycles were performed with overnight drying between each wash cycle.

After the third wash cycle the performance of a modified lipase of the invention and of the parent lipase expressed in *Aspergillus oryzae* was assessed. This was done by calculating the improvement factor (*fimprove*) as described above.

The results of these tests are shown in Table E4 below.

Table E4

| Lipase | N-terminal +/- SPIRR | 3-cycles $f_{improve}$ (Improvement factor) |
|---|---|---|
| Parent lipase (expressed in *A. oryzae)* | - | 1.0 (reference) |

Table E4 (continued)

| Lipase | N-terminal +/- SPIRR | 3-cycles $f_{improve}$ (Improvement factor) |
|---|---|---|
| Modified lipase (expressed in yeast) | + | 2.2 |
| Modified lipase (expressed in yeast) (treated with trypsin) | - | 0.6 |
| Variant of parent lipase (HLv1s) (expressed in yeast) | + | 9.3 |
| Variant of parent lipase (HLv1) (expressed in *A. oryzae)* | - | 1.8 |
| Parent lipase (expressed in *E. coli)* | - | 1.0 |
| Modified lipase (expressed in *E. coli)* (+SPIRR) | + | 2.0 |
| Modified lipase (expressed in *Hansenula*) | + | 2.1 |

It can be seen from Table E4 that the peptide addition (i.e. SPIRR) applied to the N-terminal of parent *Humicola lanuginosa* lipase at least doubles the wash performance.

**EXAMPLE 8**

One cycle wash performance of modified *H. lanuginosa* lipases containing an addition

The one cycle wash performance test (described above in the Materials and Methods section above) was performed of *Humicola lanuginosa* lipase variants of Table M1 with and without the SPIRR-peptide addition in 5 g/l of enzyme inactivated Ariel™ Futur (Procter and Gamble). The tests were performed at lipase concentrations of 0, 1250 12500 LU/1.

The detergent was dissolved in approximately 18°dH (German Hardness) water. The pH of the wash liquor was about 10.3.

The amount of soxhlet extracted fatty matter removed from textile are shown in the table below. Corresponding lipase variants with and without peptide addition are listed two and two.

Table M5

| Lipase variant | +/- SPIRR | low dosage | % lard removed | High dosage | % lard removed |
|---|---|---|---|---|---|
| HLv2s | SPIRR | 1250 LU/1 | 12.5 | 12500 LU/1 | nd |
| HLv2 | - | 1250 LU/1 | 1.7 | 12500 LU/1 | 6.0 |
| HLv3s | SPIRR | 1250 LU/1 | 8.9 | 12500 LU/1 | 33.9 |
| HLv3 | - | 1250 LU/1 | 4.6 | 12500 LU/1 | 6.9 |
| HLv4s | SPIRR | 2500 LU/1 | 26.5 | 12500 LU/1 | 47.6 |
| HLv4 | - | 0.25 mg/l | 1 | 12500 LU/1 | 26 |
| HLv1s | SPIRR | 1250 LU/1 | 12.8 | 12500 LU/1 | 45 |
| HLv1 | - | 1250 LU/1 | 1.8 | 12500 LU/1 | 7.2 |
| HLv5s | SPIRR | 1250 LU/1 | 11.4 | 12500 LU/1 | 36.5 |
| HLv5 | - | 1250 LU/1 | 1 | 12500 LU/1 | 10.6 |
| HLv8s | SPIRR | 1250 LU/1 | 4.5 | 12500 LU/1 | nd |
| HLv8 | - | 1250 LU/1 | 0 | 12500 LU/1 | |
| nd: not determined | | | | | |

The above results clearly shows that the lipase variants with a peptide addition have a significantly improved one cycle wash performance in comparison to the corresponding lipase variant without a peptide addition.

**EXAMPLE 9**

Site-directed mutagenesis of N-terminal addition of *H. lanuginosa* lipase

Mutations in the *Humicola lanuginosa* lipase having a SPIRR N-terminal addition was performed using the method described above in the Materials and Methods section.

First the gene encoding the lipase was inserted into the plasmid pHD414. The ScaI site of the Ampicillin gene of pHD414 was then changed to a MluI site. The unique ScaI site present in the lipase gene was then removed.

The desired mutation (*i.e.* SPIRPRP) was introduced in the N-terminal of the lipase gene by addition of the following oligo comprising the desired mutation: oligo 8479 (SEQ ID NO 5):

**5'- P GCG TGG ACG GCC TTG GCT AGC CCT ATT CGT CCT CGA CCG GTC TCG CAG GAT CTG - 3'**

This resulted in a *H. lanuginosa* lipase gene with a SPIRPRP N-terminal peptide addition.

**EXAMPLE 10**

Construction of N-terminal additions by random mutagenesis

Random mutagenesis of the part of the DNA sequence encoding the N-terminal addition SPIRPRP added to the first amino acid residue of the mature *H. lanuginosa* lipolytic enzyme (obtainable from DSM 4109) and containing the following further mutations in its mature part: D57G+N94K+D96L+L97M+Q249R was performed. The mutations in the mature part of the parent lipolytic enzyme was performed by PCR driven site-directed mutagenesis using the appropriate primer sequences using the procedures described in WO 95/26215 . The peptide addition SPIRPRP was applied as described in Example 9.

The nucleotide doping scheme of the SPIRPRP codons was as follows:

**Oligo 1: 5'-** GCG TGG ACG GCC TTG GCC 86(T/A) 66(A/T) 58(T/A) 67(T/A) 66(T/A) 575 66(T/A) GAG GTC TCG CAG GAT CTG -3' (57-mer) **(SEQ ID NO 82)**

the numbers referring to which of the following flasks to be used.

Flask 5: **80% A**; 6.66% C; 6,66% G og 6,66 % T.
Flask 6: **80% C**; 6,66% A; 6,66% G og 6,66 % T.
Flask 7: **80% G**; 6,66% A; 6,66% C og 6,66 % T.
Flask 8: **80%** T; 6,66% A; 6,66% C og 6,66 % G.

A two step PCR reaction protocol was used: The first step with the above primer as the 5' primer and with the primer 2056 (5'gca cgt aat gtt tgt acc 3') as the 3' primer conducted using pHL296 as the plasmid template. The product of the first PCR round was used in a new PCR with 4699 (5'cgg tac ccg ggg atc cac 3') as the 5' primer (to introduce the BamHI site and the first part of the coding sequence) and with the PCR product as the 3' primer using the same template. The resulting product was purified on Spin 100 (from Clonetech Lab., Inc.) and cut with BamHI and PvuII. The resulting DNA fragment was purified from the agarose gel with SpinX (Costar) and ligated into the yeast expression vector pJSO37 containing the *H. lanuginosa* lipolytic enzyme gene from pHL296 cloed as a BamHI-XbaI fragment cut with BamHI and PvuII. The resulting DNA was electrotransformed into DH10/DH12 *E. coli* cells (Gibco/BRG Lifetechnologies) using the conventional technique.

After transformation into *E. coli* and amplification the plasmid was purified and transformed into *S. cerevisiae* YNG 318. The resulting *S. cerevisiae* cells were screened for good performers in the alternative lipase filter assay containing detergent (3 g/l of PCS). The positives were sequenced and found to contain the peptide additions apparent from table 1 in the "Materials and Methods section (identified as HLv10s1-10).

The one-cycle wash performance of each of HL10s1-6 was tested as described in the Materials and methods section above (One cycle wash performance) at a temperature 30°C and using 5 g/l of inactivated Ariel Future as

detergent. The amount of fatty material removed by each of the modifed enzymes are shown below:

| Lipase variant | Low dosage | % lard removed | High dosage | % lard removed |
|---|---|---|---|---|
| HLv10s1 | 1250 LU/1 | 26 | I2500 LU/1 | 54 |
| HLv10s2 | 1250 LU/1 | 22 | 12500 LU/1 | 53 |
| HLv10s3 | 1250 LU/1 | 34 | 12500 LU/1 | 55 |
| HLv10s4 | 1250 LU/1 | 33 | 12500 LU/1 | 55 |
| HLv10s5 | 1250 LU/1 | 23 | 12500 LU/1 | 47 |
| HLv10s6 | 1250 LU/1 | 30 | 12500 LU/1 | 53 |
| HLv10s11 | 1250 LU/1 | 5 | 12500 LU/1 | 27 |
| HLv10s12 | - | - | 12500 LU/1 | 15.8 |
| HLv11s | 1250 LU/1 | 21 | 12500 LU/1 | 51 |

The tendency was that the best performers had more positive charged amino acids in the N-terminal addition.

Analogously, random mutagenesis of the N-terminal addition RPRPRPRP added to the *H. lanuginosa* lipase variant E1*, D57G, N94K, D96L, L97M, Q249R plus other variants were performed. The nucleotide doping scheme of the RPRPRPRP codons was as follows:

**Oligo 2: 5'-GTC TCT GCG TGG ACG GCC TTG GCG GCG CCA CCT CCA 67(T/A) 66(T/A) 575 66(T/A) 67(T/A) 66(T/A) 575 66(T/A) (6/7)(7/8)(C/G) 57(C/G) C57 (5/7)5(C/G) CTG TTT AAC CAG TTC AAT CTC-3' (93-mer) (SEQ ID NO 82)**

Flask 5: **80% A**; 6,66% C; 6,66% G og 6,66 % T.
Flask 6: **80% C**; 6,66% A; 6,66% G og 6,66 % T.
Flask 7: **80% G**; 6,66% A; 6,66% C og 6,66 % T.
Flask 8: **80% T**; 6,66% A; 6,66% C og 6,66 % G.

APPP is added in the N-terminal of the randomly mutagenized RPRPRPRP and prior to the signal peptide in order to protect against proteolytic degradation of the N-terminal addition. This may not be required. E1 was deleted in order to remove one negatively charged amino acid. The amino acids in position 2 to 5 of the mature *H. lanuginosa* lipase sequence were also mutagenized in order to find improved mutants in this non-structural part of the lipase. Otherwise the procedure is as stated above for the random mutagenesis of SPIRPRP. The following N-terminal peptide additions were obtained:

Ala-Pro-Pro-Pro-Arg-Pro-Arg-Leu-Leu-Pro-Ile-Ser( APPPRPRLLPIS) (in addtion to the deleted E1 residue this variant carries the additional mutation D5E in its non-structural N-terminal part of the mature enzyme).

Ala-Pro-Pro-Pro-Thr-Arg-Gln-Arg-Gln-Ser-Pro(APPPTRQRQSP) (in addtion to the deleted E1 residue this variant carries the additional mutations V2L, S3T and D5V in its non-structural N-terminal part of the mature enzyme).

Ala-Pro-Pro-Pro-Arg-Thr-Ile-Pro-Arg-Ser-Ser-Pro( APPPRTIPRSSP) (in addtion to the deleted E1 residue this variant carries the additional mutations V2L, S3R and D5E in its non-structural N-terminal part of the mature enzyme).

Ala-Pro-Pro-Pro-Arg-Pro-Arg-Pro-Arg-Pro-Arg-Pro (APPPRPRPRPRP) (in addtion to the deleted E1 residue this variant carries the additional mutations V2G and D5E in its non-structural N-terminal part of the mature enzyme).

Ala-Pro-Pro-Pro-Arg-Thr-Arg-Pro-Arg-Pro-Arg-Ser (APPPRTRPRPRS) (in addtion to the deleted E1 residue this variant carries the additional mutations V2GL, S3T, Q4P and D5E in its non-structural N-terminal part of the mature enzyme).

Ala-Pro-Pro-Pro-Lys-Ala-Ser-Pro-Arg-Gln-Arg-Pro (APPPKASPRQRP) (in addtion to the deleted E1 residue this variant carries the additional mutations V2GL, DSQ and L6M in its non-structural N-terminal part of the mature enzyme).

**EXAMPLE 11**

Construction of *Ps. cepacia* lipase variants comprising peptide additions

A lipase gene from *Pseudomonas cepacia* SB10, DSM 3959, described in WO 89/01032 (from Novo Nordisk A/S) recently reclassified as *Burkholderia cepacia* was cloned, and temperature-inducible expression of the lipase in *Escherichia coli* was obtained by use of the plasmid pAHE2. Strain SJ1503 is *E. coli* JA221 containing pAHE2.

To construct vectors expressing variant lipases with N-terminal extensions, use were made of two unique restriction sites present in pAHE2, a unique BstXI site approximately 9 codons into the lipase signal peptide coding sequence, and a unique MluI site approximately 7 codons downstream from the processing site, *i.e.* in the beginning of the sequence for the mature lipase.

PCR primers were designed to allow amplification across this region, with the primers reading upstream from the MluI site encompassing sequences encoding the N-terminal extensions. All primers had incorporated EcoRI sites in their extreme 5' ends.

The following sequences were chosen to encode N-terminal extensions:

**1) S P I R P R P**
**AGC CCG ATC CGC CCG CGC CCG**

**2) T A I R P R K**
**ACG GCG ATC CGC CCG CGC AAG**

**3) S T R R P R P**
**TCG ACG CGC CGT CCG CGC CCG**

**4) G P I R P R P**
**GGC CCG ATC CGC CCG CGC CCG**

**5) S P I R R**
**AGC CCG ATC CGC CGG**

**6) R P R P R P R P**
**CGC CCG CGT CCC AGG CCG CGT CCG**

The following primers were used:

**LWN9476 (SEQ ID No. 7)(reading downstream from the BstXI site):**
**5'-CGAATTCGATGCGTTCCAGGGTGGTGGCAGG-3'**

**LWN9472 (SEQ ID No. 8)(reading upstream from MluI, designed to incorporate SPIRPRP):**
**5'-**
**CGAATTCACGCGTCGCCGCGTAGCCAGCGGCCGGGCGCGGGCGGATCGGGC TGGGCGCGGTGGCCGCCATTGCC-3'**

**LWN9473 (SEQ ID No. 9)(reading upstream from MluI, designed to incorporate TAIRPRK):**
**5-**
**GAATTCACGCGTCGCCGCGTAGCCAGCGGCCTTGCGCGGGCGGATCGCCGT GGGCGCGGTGGCCGCCATTGCC-3'**

**LWN9471 (SEQ ID No. 10)(reading upstream from MluI, designed to incorporate STRRPRP):**
**5'-**
**CGAATTCACGCGTCGCCGCGTAGCCAGCGGCCGGGCGCGGACGGCGCGTCG AGGGCGCGGTGGCCGCCATTGCC-3'**

**LWN9474 (SEQ ID No. 11)(reading upstream from MluI, designed to incorporate GPIRPRP):**
**5'-**
**CGAATTCACGCGTCGCCGCGTAGCCAGCGGCCGGGCGCGGGCGGATCGGGC CGGGCGCGGTGGCCGCCATTGCC-3'**

LWN9475 (SEQ ID No. 12)(reading upstream from MluI, designed to incorporate SPIRR):
5'-CGAATTCACGCGTCGCCGCGTAGCCAGCGGCCCGGCGGATCGGGCT-GGGCGCGGTGGCCGCCATTGCC-3'

LWN9470 (SEQ ID No. 13)(reading upstream from MluI, designed to incorporate RPRPRPRP):
5'-
CGAATTCACGCGTCGCCGCGTAGCCAGCGGCCGGACGCGGCCTGGGACGCG GGCGGGGCGCGGTGGCCGCCATTGCC-3'

For PCR amplifications, primer LWN9476 was used in combination with each of primers LWN9470-LWN9475, with pAHE2 as template. Annealing temperature was 70°C, and reactions were performed in the presence of 2 % DMSO; otherwise using standard conditions and Taq™ polymerase.

Amplified fragments were purified from a 2% agarose gel, digested with BstXI and MluI, ligated to the 7.1 kb BstXI-MluI fragment obtained from pAHE2, and the ligation mixture used to transform, by electroporation, *E. coli* SJ6 to ampicillin resistance. Transformants were plated on LB plates with ampicillin (200 mg/ml) at 30°C.

By replica plating colonies were transferred to lipase screening plates (containing, pr. litre of agar, 20 ml of

Sigma Lipase Substrate (catalogue no. 800-1)) and 4 ml of a 1 % Brilliant Green (Merck, art. No. 1.01310) solution), which were incubated at 42°C. Eventually, green halos, indicating lipase activity, developed around several colonies from each transformation mixture.

Lipase positive colonies were re-isolated, plasmids extracted, and the BstXI-MluI region DNA sequenced. The following strains were kept:

SJ3606 (SJ6/pSJ3606); contains the SPIRPRP encoding addition, and has also the second codon in the native, mature enzyme changed from alanine to valine.
SJ3608 (SJ6/pSJ3608); contains a SPRP encoding addition (DNA sequence of insert TCT CCG CGC CCG. Obtained as a variant in attempts to produce a STRRPRP encoding addition.
SJ3708 (SJ6/pSJ3708); contains the SPIRR encoding addition.
SJ3717 (SJ6/pSJ3717); contains the SPIRPRP encoding addition.
SJ3718 (SJ6/pSJ3718); contains the SPIRPRP encoding addition.
SJ3719 (SJ6/pSJ3719); contains the TAIRPRK encoding addition.
SJ3720 (SJ6/pSJ3720); contains the STRRPRP encoding addition.
SJ3721 (SJ6/pSJ3721); contains the GPIRPRP encoding addition.

**EXAMPLE 12**

Shake Flask Fermentation of *Ps. cepacia* Lipase variants

Cultures provide in Example 11 were grown on TY-ampicillin plates (pH 7) and used to inoculate shake flasks containing 100 ml double concentrated TY-medium with ampicillin (100 mg/ml) pH 7. The inoculum was checked for lipase productivity (as described in the Materials and Methods section) by streaking on indicator plates: all cells were found to be lipase positive (plates were incubated at 30°C for 2 days, then transferred to 40°C for 1 day).

The shake flasks were incubated shaking at 275 rpm at 30°C for 6 hours until the cultures reached optical densities (578 nm) of 2.8 to 5.3. The cultures were then transferred to 40°C for another 17 hours.

Check of lipase production in a *Ps. cepacia* culture

The culture was harvested, centrifuged (20 minutes at 9000 rpm), the supernatant discarded and the pellet re-suspended in NaCl (0.5 ml 0.9% NaCl) and sonicated (2 minutes non-stop, on ice). The sonicated pellet was used to measure Lipase units (LU) using the titration method with tributyrate as substrate at pH 7.0.
All 8 strains except 1 (SJ3720) showed lipase activity as indicated in the table below.

| Strain | time (hs) | OD=578 | AmpR: cell# | OOBGAmp: :cell# | LU/ml# |
|---|---|---|---|---|---|
| SJ1503wt | t0 = 0h | 0.010 | | | |
| | t1 = 6hs | 2.89 | 7 | 7 | |
| | t2 = 17hs | 7.45 | 0 | 0 | 230.5 |
| SJ3606 | t0 = 0h | 0.006 | | | |
| | t1 = 6hs | 5.24 | 43 | 43 | |
| | t2 = 17hs | 9.15 | 0 | 0 | 244.45 |
| SJ3608 | t0 = 0h | 0.015 | | | |
| | t1 = 6hs | 4.40 | 67 | 65 | |
| | t2 = 17hs | 9.2 | 0 | 0 | 298.6 |
| SJ3708 | t0 = 0h | 0.028 | | | |
| | t1=6hs | 4.69 | 32 | 32 | |
| | t2=17hs | 11.05 | 0 | 0 | 142.2 |
| SJ3717 | t0 =0h | 0.007 | | | |
| | t1=6hs | 4.03 | 28 | 28 | |

(continued)

| Strain | time (hs) | OD=578 | AmpR: cell# | OOBGAmp: :cell# | LU/ml# |
|---|---|---|---|---|---|
| | t2=17hs | 11.2 | 15 | 15 | 163.8 |
| SJ3719 | t0=0h | 0.001 | | | |
| | t1=6hs | 4.49 | 13 | 13 | |
| | t2 =17hs | 11.7 | 0 | 0 | 33.55 |
| SJ3720 | t0=0h | 0.004 | | | |
| | t1=6hs | 3.70 | 20 | 20 | |
| | t2=17hs | 10.5 | 0 | 0 | 0 |
| SJ3721 | t0=0h | 0.016 | | | |
| | t1=6hs | 4.20 | 12 | 12 | |
| | t2= 17hs | 11.35 | 0 | 0 | 125.75 |

## EXAMPLE 13

Characterization of *Ps. cepacia* Lipase variants

The lipases produced from the strains described In Example 11 were characterized with respect to activity in the presence of detergent, using the PCS plate screening assay One set of samples was prepared from strains SJ1503, SJ3606 and SJ3608, which had been propagated as described above, cells harvested, and lysed by sonication to liberate the lipase. 15 ml of samples, containing around 230 LU/ml, were applied in wells in screening plates either without detergent, or containing 1.5 and 3.5 grams/litre of detergent, respectively. Plates were incubated at 37°C overnight, and the diameter of the green zone formed around the wells measured. The following results were obtained:

| | **STRAIN** | | |
|---|---|---|---|
| | SJ1503 | SJ3606 | SJ3608 |
| DETERGENT | | | |
| None<br>1.5 gram/l<br>3.5 gram/l | 17 mm<br>7 mm<br>0 mm | 15 mm<br>13 mm<br>8 mm | 16 mm<br>10 mm<br>6 mm |

Green zones were not observed at higher detergent concentrations.

Another set of samples were prepared by plating of the strains SJ1503, SJ3708, and SJ3717-SJ3721 on cellulose acetate filters (each filter containing all 7 strains), which were placed on LB plates with ampicillin (200 mg/ml) at 37°C overnight, these plates with filters then incubated at 42°C for 5 hours, after which the filters were transferred (colony side up) to screening plates which were incubated overnight at 37°C.

Pronounced green zones developed under all colonies on the plate without detergent; SJ3720 produced a significantly smaller zone then the rest, most likely due to reduced expression of the lipase.

Green zones were also observed under all colonies on the plate containing 1.5 gram/l of detergent. However, the zone produced from SJ1503, producing the native, unmodified lipase, was significantly reduced as compared to the zones produced from the other strains.

On the plate containing 3.5 grams/litre detergent, no green coloration developed from SJ1503, whereas a greenish stain was still discernible from some strains expressing modified B. *cepacia* lipases, in particular SJ3717, SJ3718 and SJ3721.

Thus, modification of the B. *cepacia* lipase gene to encode N-terminal additions to the native, mature lipase, as those described above, allow the production of lipases which in the presence of detergent has an improved activity as compared to the native lipase.

**EXAMPLE 14**

Fermentation of SJ1503 and SJ3717 in 10 litre tanks

The method described for shake flask was used for the fermentation in 10 litre scale. The medium used was Bacto Tryptone 400g, Bacto Yeast extract 200 g, Glucose x 2 $H_2O$ 500g, Ampicillin 1 g, Pluronic 1 ml. The pH was kept constant at pH 7.1; the temperature was 30°C for 7 hours then adjusted to 40°C. Cells were harvested after 16 hours by centrifugation and the cells were opened using a high pressure homogenizer (800 bar).

Purification of *B. cepacia* expressed *E. coli*

*E.coli* cells from 10 liter fermentation broth from SJ1503 and SJ3717 were centrifuged and the supernatant was discarded. Cells were opened using rannie homogenizer under pressure 800 bar. Homogenized cells were centrifuged at 350 x g for 60 minutes. Cell supernatant was decanted.

1. Salt precipitation

Activity containing supernatant was precipitated with addition of solid ammonium sulphate to saturation of 35 % at room temperature. Precipitation was allowed for 2 hour at room temperature and centrifuged at 350x g for 1 hour. Supernatant was decanted and discarded. Precipitate containing activity was dissolved in 30% ethanol to avoid hydrophobic biding of the lipase activity to insoluble material.

To get rid of insoluble material from the 30 % ethanol dissolved material , the solution was centrifuged. The lipase activity was recovered as supernatant and insoluble material was discarded. The supernatant containing activity was concentrated and dialyzed against 25 mM Tris-acetate pH 8, by ultra-filtration using Amicon membrane with cut-off of 10 kDa. The concentrated sample was then diluted five fold in order to reduce any leftover ethanol in the supernatant containing activity.

2. Hydrophobic chromatography

The above sample containing activity was adjusted to 0.8 M ammonium acetate by adding solid ammonium acetate. 50 ml Toyopearl Butyl column (Tosho Hass, Japan) was packed and equilibrated with 0.8 M ammonium acetate. The samples from above step containing lipase activity was then adjusted to 0.8 M ammonium acetate and applied on the Toyopearl Butyl column. All the activity binds to the matrix. Unbound material was washed with 0.8 M ammonium acetate till Uv absorbence of the effluent was under 0.05 at 280 nm. Bound activity was eluted with 25 mM Tris acetate buffer containing 50 % ethanol. Fractions containing lipase activity were pooled and dialyzed against 25 mM Tris acetate buffer pH 8.5.

3. Anion exchange chromatography

50 ml Column was packed with anion exchanger Highperformance Q-sepharose (Pharmacia). The column was washed and equilibrated with 25 mM Tris acetate buffer pH 8.5 The dialyzed sample was then applied on the column. Unbound activity was washed out by using the Tris buffer. Bound activity was eluted with a linear salt gradient from 0 to 0.5 M NaCl in the Tris buffer pH 8. Flow rate was 2 ml/min and total volume of the buffer used for elution was 10 column volumes. Fractions containing lipase activity were pooled and tested for performance in a PCS plate assay.

More specifically, 3 LU of each of the recovered modified lipases were added into holes of a PCS plate (cf. Example 15 hereinafter) and incubated overnight at 37°C. After 18 hours the following results were obtained:

| | STRAIN | |
|---|---|---|
| | SJ1503 | SJ3717 |
| DETERGENT | | |
| None | 17 mm | 13 mm |
| 0.5 gram/l | 6 mm | 10 mm |
| 1.0 gram/l | 4 mm | 7 mm |

Thus, it can be seen that the presence of a peptide addition results in a signifantly higher wash performance being obtained.

**EXAMPLE 15**

Construction of modified *H. insolens* lipolytic enzymes with an N-terminal peptide addition

The gene encoding the parent lipolytic enzyme was isolated from *Humicola insolens* DSM 1800 essentially as described in WO 96/13580. Three different peptide additions were applied to the N-terminus of the mature enzyme using the plasmid pIVI1303 as the plasmid template.

Construction of pIVI303 (encoding a *H. insolens* lipolytic enzyme variant which contains a mutation in the region 304-369 base downstream from ATG without changes in amino acid sequence and removing a possible secondary DNA structure which might otherwise have hampered the use of the chameleon double stranded kit.)

The plasmid was constructed using the Chameleon double-stranded, site-directed mutagenesis kit from Stratagene (cat no. 200509) according to the described protocol.

pIVI296 was used as the plasmid template and primer no 7258 as the selection primer.

7258: 5'p gaa tga ctt ggt tga cgc gtc acc agt cac 3'

(Thus changing the ScaI site found in the ampicillin resistance gene and used for cutting to a MluI site).

Primer no 9349 was used as the mutagenic primer:

9349: 5'p gag tcc cac atc cga aac atc tgg ata caa gga gta gga gga cct tac gac gcc gcg 3'

**1. Variant: HILv4s containing the mutation: PPRRPR (instead of PELVAR in the native *H. insolens* propeptide)**

Construction of pIVI335:

The plasmid was constructed by use of the Chameleon double-stranded, site- directed mutagenesis kit from Stratagene (cat no. 200509) according to the described protocol. pIVI303 was used as a plasmid template.

Primer no.7887 was used as a selection primer:

7887: :5'p-gaa tga ctt ggt tga gta ctc acc agt cac 3`

(changing the introduced Mlu 1 site found in the ampicillin resistance gene and used for cutting to a ScaI site).

Primer no 19473 was used as a mutagenic primer:

19473: 5'p ac cat acc ccg gcc gct cct cct agg cgt cct cgg cag ctg gga gcc 3

2. Variant: HILv1s containing the mutation SPPRRP (instead of ELVARQ in the native *H. insolens* propeptide)

Construction of pIVI359:

The plasmid was constructed by use of the Chameleon double-stranded, site-directed mutagenesis kit from Stratagene (cat no. 200509) according to the described protocol. pIVI303 was used as a plasmid template. Primer no. 7887 (cf. above) was used as a selection primer. Primer no 21992 was used as a mutagenic primer:

21992: 5'p ac cat acc ccg gcc gct cct agc cct ccg cgg cgg ccg ctg gga gcc atc gag aac ggc
3

**3. Variant: HILv2s containing the mutation SPPRP (instead of ELVARQ in the native *H. insolens* propeptide)**

Construction of pIVI360:

The plasmid was constructed using the Chameleon double-stranded, site-directed mutagenesis kit from Stratagene (cat no. 200509) according to the described protocol. pIVI303 was used as a plasmid template, and primer no. 7887 as a selection primer. The following primer was used as the selection primer:

5'p ac cat acc ccg gcc gct cct agc cct ccg cgg ccg ctg gga gcc atc gag aac ggc 3

**4. Variant: HILv3s containing the mutation: SPIRK (instead of ELVARQ in the native *H. insolens* propeptide)**

Construction of pIVI361:

The plasmid was constructed using the Chameleon double-stranded, site-directed mutagenesis kit from Stratagene (cat no. 200509) according to the described protocol. pIVI303 was used as a plasmid template and primer no 7887 as a selection primer. Primer no 21994 was used as a mutagenic primer:

21994: 5'p ac cat acc ccg gcc gct cct agc cct ata cgt aag ctg gga gcc atc gag aac ggc 3

**5. Construction of an *A. oryzae* expression vector pIVI296:**

pA2L79 is described in Example 2 of WO 96/13580. The plasmid contains the *H. insolens* lipolytic enzyme cDNA sequence inserted into the *A. oryzae* expression plasmid pD414. PA2L79 was cut with the restriction enzymes HindIII and XhoI. The fragment containing the lipolytic enzyme encoding cDNA sequence (1088 bp) was purified from agarose gel. PHD414 was cut with the restriction enzymes HindIII and XhoI and the vector purified form an agarose gel. The purified vector fragment (pHD414) and the lipase containing fragment was ligated thus creating pIVI296.

Each of the above expression vectors were transformed into *A. oryzae* IFO 4177 by use of the general transformation method disclosed in the Materials and Methods section above. One transformant of each type was isolated as HILv1-4s, respectively. The *H. insolens* transformants were grown for 3 days in shake flaks at 30°C in 500 ml YPM medium (10 g/L bacto yeast extract,20 g/L bacto peptone, 20 g/L maltose).

Fermentation supernatent was filtered as described for modified *H. lanuginosa* lipolytic enzymes.
Purification Step 1:- 1 liter of the Fermentation supematent was adjusted to pH 8 and diluted so conductance of the supematent was under 4 mSi.

Step 1: Batch treatment of the fermentation supematent on anion exchanger DE-AE A50.
DEAE-Sephadex A50 from Pharmacia was washed and equilibrated with 25 mM tris acetate buffer pH 8 using Scintered glass funnel with appropriate pore size. Fermentation supematent was then applied on the DEAE Sephadex A50 using the scintered glass funnel. The Lipolytic activity from H.insolence did not bind to anion exchanger at pH 8 and collected as effluent from DEAE Sephadex A50.

Step 2 :- pH of the efflent from DEAE Sephadex was adjusted to 4.5 by adding dilute Acetic acid. Condctance was also adjusted under 4 mSi by adding water. Cation Exchange chromatography on SP-Sepharose. 50 ml Column was packed with SP Sepaharose Fast Flow Code no 17-0729-01 Pharmacia. Column was then washed and equilibrated with 25 mM Sodium acetate buffer pH 4.5.
Sample containing Lipolytic activity adjusted to pH 4.5 and the conductance under 4 mSi was then applied on SP-Sepharose column. Unbound material was washed using 25 mM Sodium acetate buffer pH 4.5. Lipolytic activity bound to the SP-Sepharose was then eluted with linear salt gradient with 25 mM Acetate buffer pH 4.5 containing 1M Sodium Chloride. Fractions containing Lipolytic activity and ratio of the UV absorbence at A280 /A260 was higher that 1.8 were pooled. Purity of the sample was checked on SDS-PAGE.

Verification of N-terminal peptide addition

The N-terminal amino acid sequence of the HILv1s lipolytic enzyme was determined (i.e. the variant in which the last 5 amino acid residues in the propeptide and the first amino acid residue in the mature enzyme (ELVARQ) have been substituted with SPPRRP).
The N-terminal amino acid sequence found was

Arg-Arg-Pro-Leu-Gly-Ala-Ile-

corresponding to the last three amino acid residues in the substituted sequence and the first four amino acid residues following the substitution.

The N-terminal amino acid sequence of the HILv2s lipolytic enzyme was also determined (i.e. in the variant in which the last 5 amino acid residues in the propeptide and the first amino acid residue in the mature enzyme (ELVARQ) have been substituted with SPPRP). The N-terminal amino acid sequence found was

Arg-Pro-Leu-Gly-Ala-Ile-Glu-Asn

corresponding to the last two amino acid residues in the substituted sequence and the first six amino acid residues following the substitution.

**EXAMPLE 16**

Characterization of modified *Humicola insolens* Lipolytic enzymes

The modified lipolytic enzymes comprising peptide additions, produced by the strains HILv1s, HILv2s, HILv3s, respectively, (described in Example 21), and the wild-type strain HIL, were characterized with respect to lipase activity on PCS-plates containing 0.5 g/l, 1.0 g/l and 1.5 g/l PCS-detergent.

25 µl (corresponding to 5 LU) purified modified HILvs1, HILvs2 and HILvs3 lipase, and wild-type HIL lipase were entered into holes made in the PCS-plates by a pipette (4 mm) and incubated for 3 and 6 hours, respectively.

The result of the test in displayed in the tables below:

| Variant | 0.5 g/l PCS-detergent | 1.0 g/l PCs-detergent | 1.5 g/l PCS-detergent |
|---|---|---|---|
| HIL (wild-type) | 4 mm | 4 mm (weak) | 0 mm |
| HILv1s | 6 mm | 5 mm | 4 mm (weak) |
| HILv2s | 5 mm | 4 mm | 0 mm |
| HILv3s | 6 mm | 6 mm | 5 mm |
| Incubation of 3 hours on PCS-plates containing FY-detergent. | | | |

| Variant | 0.5 g/l PCS-detergent | 1.0 g/l PCS-detergent | 1.5 g/l PCS-detergent |
|---|---|---|---|
| HIL (wild-type) | 4 mm | 4 mm (weak) | 0 mm |
| HILv1s | 7 mm | 5 mm | 4 mm (weak) |
| HILv2s | 5 mm | 5 mm (weak) | 4 mm (weak) |
| HILv3s | 6 mm | 6 mm | 4 mm (weak) |

Incubation for 6 hours on PCS-plates containing PCS-detergent.

s As can be seen from the tables the modified lipase variants (*i.e.* produced by HILv1s, HILv2s and HILvs3) generally have a higher lipase activity in the presence of the PCS-detergent than the wild-type lipase.

**EXAMPLE 17**

Construction of modified *H. lanuginosa* lipolytic enzymes with a C-terminal extension

C-terminal peptide additions were applied to the *H. lanuginosa* lipolytic enzyme variant HLv12s containing the N-terminal peptide addition SPIRPRP and the internal mutations D57G,N94K,D96L,Q249R.

**1. Variant HLv13s (HLv12s with the C-terminal peptide addition: 270R,271R,272P,stop)**

Construction of plasmid pS14-1:

The plasmid was constructed using the Chameleon double-stranded, site directed mutagnenesis kit from Stratagene (cat nd. 200509) according to the descrbed protocol.
pIVI245 was used as the plasmid template (The construction of pIVI245 is described in Example 6) and primer no. 7258 as the selection primer.
7258: 5'p gaa tga ctt ggt tga cgc gtc acc agt cac 3' (Thus changing the ScaI site found in the ampicillin resistance gene and used for cutting to a MluI site).
Primer no.20694 was used as the mutagenic primer.

20694: 5'p-gg gac atg tct tcg acg acc gta gcg gct ggg tcg act c 3.

**2. Variant HLv14s (HLv12s with the mutation: 270R,271R,stop)**

Construction of plasmid pS20-2:

The plasmid was constructed using the Chameleon double-stranded, site-directed mutagenesis kit from Stratagene (cat no. 200509) according to the described protocol. pIVI245 was used as the plasmid template and primer no. 7258 as the selection primer.
7258: 5'p gaa tga ctt ggt tga cgc gtc acc agt cac 3' (Thus changing the ScaI site found in the ampicillin resistance gene used for cutting to a MluI site).
Primer no. 20695 was used as the mutagenic primer:

20695: 5'p-gg gac atg tct tcg gcg gta ggc gcg gct ggg tcg ac 3'

Production of enzyme variants

The enzymes were produced in an analogous manner to that described in Example 6 using the plasmid pToC 202 for the cotransformation step and *A. oryzae* JAL 125 as a host cell.

Verification of the presence of the C-terminal extension in HLv12s

A 1 mg sample of HLv12s containing the C-terminal extension Arg-Arg-Pro (RRP) was S-carboxamidomethylated using standard procedures before degradation with a lysyl-specific protease. The resulting peptides were separated using reversed phase HPLC and the collected fractions subjected to matrix assisted laser desorption ionization time-of-flight mass spectrometry. A fraction containing a peptide with the experimental mass of 3906.7 Da was found. This mass is within experimental error identical to the theoretical mass of the C-terminal peptide of HLv12s containing the RRP extension which is 3906.4 Da.
The amino acid sequence of the peptide in this fraction was determined to be

Ile-Glu-Gly-Ile-Asp-Ala-Thr-Gly-Gly-Asn-Asn-Arg-Pro-Asn-Ile-
Pro-Asp-Ile-Pro-Ala-His-Leu-Trp-Tyr-Phe-Gly-Leu-Ile-Gly-Thr-
Cys-Leu-Arg-Arg-Pro

which is the correct amino acid sequence of the C-terminal peptide of HLv12s and it contains the C-terminal extension Arg-Arg-Pro.

C-terminal extension:

Wash result: 5g/l inactivated Ariel Funir, 20 minutes, 1 cycle TOM, 30°C, 18°dH. The enzyme was dosed as mg Enzyme Protein per litre wash solution.

HLv13s: SPIRPR + D57G,N94K,D96L,Q249R,270R,271R,272P
HLv14s: SPIRPR + D57G,N94K,D96L,Q249R,270R.271R

| Variant | dR (0.25 mgEP/1) | dR(1 mgEP/1) |
|---|---|---|
| HLv12s | 5 | 9 |
| HLV13s | 1 | 3 |
| HLV14s | 5 | 9.5 |

**EXAMPLE 18**

A part of the N-terminal extension of HLv15s (HLv15s containing the N-terminal peptide addtion SPIRPR and the following mutations in the mature part of the *H. lanuginosa* lipolytic enzyme EP, D57G, N94K, D96L, L97M, Q249R) was cleaved off by prolonged incubation with Clostripain (EC 3.4.22.8; Sigma No. C-0888).

The incubation mixture contained: HLv15s (1 mg/ml) and Clostripain (20 μg/ml) in 25 mM sodium phosphate, pH 7.4 containing 2.5 mM DTT and 1 mM calcium chloride.

Before incubation with Clostripain 60 % of the lipase carried an intact propeptide (N-terminal amino acid sequence SPIRPRP), while 10% had lost the first Ser-residue (N-terminal amino acid sequence PIRPRPV) and 30% the first 5 amino acid residues of the propeptide (N-terminal amino acid sequence (RPVSQDL).

Following incubation for 62 h at ambient temperature (resulting in HLv15s-C) 60% of the lipase had lost the first 4 amino acid residues of the propeptide (resulting in the following peptide extension PRPVSQ), 20% were without 5 amino acid residues (thus having the peptide extension RPVSQD) while the remaining 20% had lost 6 amino acid residues (thus having the peptide extension PVSQDL).

The propeptide processing was determined using N-terminal amino acid sequence determination and it should be noted that the percentages given are approximate values.

| Variant | Peptide addition | |
|---|---|---|
| HLv15s | 60 % SPIRPRPYSQD<br>10 % PIRPRPVSQD<br>30 % RPVSQD | D57G, N94K, D96L, L97M, Q249R |
| Hlv15s-C | 60 % PRPVSQ<br>20 % RPVSQ<br>20 % PVSQDL | D57G, N94K, D96L, L97M, Q249R |

One cycle wash performance with a modified lipolytic enzyme treated with clostripain

The one cycle wash performance test (described above in Materials and Methods section above) was performed with *H. lanuginosa* lipase variant HLv15s treated with clostripain. Wash test was made both with the clostripain treated sample and the non clostripain treated variant. The wash test was carried out in 5 g/l enzyme inactivated Ariel Futur (Procter and Gamble). Lard stained swatches were washed for 20 minutes at 30°C. The tests were performed at lipase concentrations of 0, 5000 LU/1 and 12500 LU/1.

The detegent was dissolved in approx. 18°dH (German Hardness) water. The pH of the wash liquor was about 10.3. Seven swatches were washed in 1000 ml wash liquor. Subsequent to the washing, the swatches were flushed in running tap water for 15 minutes and then air-dried overnight at room temperature.

Evaluation: The reflectance of the swatches was measured at 460 nm, and the lipase performance (_R) calculated as:

_R = delta Reflectante =

($R_{swatches\ washed\ in\ detergent\ with\ lipase}$ - $R_{swatches\ washed\ in\ detergent\ without\ lipase}$)

The mutations of the lipases and the additions are described above. The_R, are shown in the table below.

| Variant | +/- treatment w. clostripain | low dosage | _R | high dosage | _R |
|---|---|---|---|---|---|
| HLv15s | no clostripain treatment | 5000 LU/1 | 10 | 12500 LU/1 | 13 |
| HLv15s-C | + clostripain treatment | 5000 LU/1 | 6 | 12500 LU/1 | 7 |

The results show that the presence of an intact peptide addition leads to the best wash performance. A reduced (but not entirely removed) peptide addition provides an improved wash performance, especially when positively charged amino acid residues are present in the addition.

**EXAMPLE 19**

Modified *H. lanuginosa* lipolytic enzyme containing an cysteine bridge (HLv16s)

The modified *H. lanuginosa* lipolytic enzyme HLv16s contains the following mutations: N94K, D96L. E239C and Q249R and the peptide addition SCIRR.
The parent enzyme HLv16 contains the following mutations: N94K, D96L, Q249R, HLv16s was constructed as follows:

1. Construction of N94K, D96L mutations in the wildtype *H. lanuginosa* lipolytic enzyme Construction of pIVI290:

The plasmid was constructed using the Chamelon double stranded, site-directed mutagenesis kit from Stratagene according to the described protocol using the pAHL (cf Fig. 6 of WO 92/05249) as the plasmid template and primers no 7258 and 7770 as the selection primers. 7258: 5'p gaa tga ctt ggt tga cgc gtc acc agt cac 3' (Thus changing the ScaI site found in the ampicillin resistans gene to a MluI site)(ScaI has been used for cutting).
7770: Sequence: 5'p tct agc cca gaa tac tgg atc aaa tc 3 (Changes the ScaI site found in the wild type *H. Lanuginosa* lipase gene).
primer no. 8932 was used as the mutagenic primer.

8932: 5'pgaac tgg ata gga aat ttg aag ttc ctg ttg aaa gaa ata aat gac 3' (Introducing N94K,D96L)

2. Construction of HLv16s (SCIRR, N94K,D96L, E239C, Q249R) Construction of pIVI319:

The plasmid was constructed using the Chameleon double-stranded,site directed mutagenesis kit from Stratagene (cat no. 200509) according to the described protocol using pIVI290 as the plasmid template and primer no 7887 as the selection primer.
7887: 5'p-gaa tga ctt ggt tga gta ctc acc agt cac 3' (changing the introduced Mlu1 site found in the ampicillin resistans gene to a ScaI site)(MluI has been used for cutting) Primers no 8829, 9639 and 9646 were used as mutagenic primers

8829: 5'p-ggc ggc aat aac cgg ccg aac att ccg gat atc cc 3' (Introducing Q249R)

9639: 5'p-at atc gtg aag ata tgc ggc att gat gcc acc 3' (Introducing E239C)

9646: 5'p-cg gcc ttg gct agc tgt att cgt cga gag gtc 3' (Modifying the propeptide from SPIRR to SCIRR )

Production of enzymes HLv16s and HLv16

The enzymes were produced in an analogous manner to that described in Example 6 using *A. oryzae* JAL 125 as a host cell. Subsequently, the one cycle wash performance of the enzymes were tested (using 5 g/l of inactivated Ariel Future as detergent and an enzymew dosage of 0.25 mg enzyme protein/1 and 1.0 mg enzyme protein/1, respectively.

The following results were obtained:

| | dR (0.25 mg EP/1) | dR (1 .0 mg EP/1) |
|---|---|---|
| HLv16s | 3 | 7 |
| HLv16 | 1 | 2 |

It is seen that a significantly improved washing performance is obtained for HLv16s containing a cystein bridge between the peptide addition and the mature part of the enzyme.

## Claims

**1.** A lipase having one of the following: N- or C-terminal sequences:

a) SPIRR or
b) RPVSQD or
c) SPIRM or
d) SPIRAR or
e) SPIRPR or
f) SPIRER or
g) SPIRK or
h) SPIKK or
i) SPIRRP or
j) SPPRR or
k) SPIPR or
l) SPRPR or
m) SPIR or
n) SCIRR or
o) SPIRPRP or
p) SCPIRPRP or
q) SPRRPRT or
r) SPFRPKL or
s) SPPRRP or
t) SPIRRE or
u) SPPRPP or
v) SPPRPR or
w) SPPWWP or
x) SPPWRP or
y) SPPRWP or
z) SPPRWP or
aa) SHWRRW or
bb) SHWRK or
cc) SHWRR or
dd) TAIRPRK or
ee) STRRPRP or
ff) GPIRPRP or
gg) LPFRQRP or
hh) SRSRHNA or
ii) IPIRPRR or
jj) STRRPRP or
kk) TAIRPRK or
II) WRWRWR or
mm) QPIRR or
nn) SHWQQ or
oo) RPRPRPRP or
pp) SSTRRASPIKK or
qq) AWWPSPIRPRP or
rr) APPPRPRPRPRP or
ss) APPPRTRPRPRS or
tt) SPKRKPRP or
uu) SQRIKQRIK or
w) SPPPRPRP or
ww) SPIRPRPRPR or
xx) SPIRKAWWP or
yy) APPPKASPRQRP or
zz) SPIRPRPSPIRPRP or
aaa) SPPRWPRR or
bbb) SPPRWPRW or
ccc) SPPRWPWR or
ddd) SPPWRPRR or
eee) SPPWWPRW or
fff) SPPWWPWR or

ggg) SPPWWPWW or
hhh) SPPWPRPRP.

**2.** The lipase of claim 1, wherein the N- or C-terminal sequence is attached to a lipase derived from *Pseudomonas* sp.

**3.** The lipase of claim 2 wherein the N- or C-terminal sequence is attached to a lipase derived from *Pseudomonas cepacia (Burkholderia cepacia).*

**4.** The lipase of claim 1, wherein the N- or C-terminal sequence is attached to a lipase derived from *Humicola* sp.,

**5.** The lipase of claim 4 wherein the N- or C-terminal sequence is attached to a lipase derived from *H. lanuginosa or H. insolens.*

**6.** The lipase of claim 1, which is derived from the amino acid sequence of Fig. 1 (SEQ ID NO: 16).

**7.** The lipase of claim 6 wherein the terminal sequence has replaced amino acid residue E23 in Fig. 1 (SEQ ID NO: 16).

**8.** The lipase of any of claims 1-7, wherein the N- or C-terminal sequence comprises a cysteine residue and the remaining part of the lipase comprises a cysteine residue such that the two cysteine residues together form a cystine bridge.

**9.** A DNA sequence encoding the lipase of any of claims 1-8 which lipase has the N- or C-terminal sequence b)-hhh).

**10.** An expression vector which comprises the DNA sequence of claim 9 and further comprises DNA sequences permitting expression of the lipase.

**11.** A transformed host cell harbouring the DNA sequence of claim 9 or the vector of claim 10.

**12.** The transformed cell of claim 11, wherein the host cell is a filamentous fungal, a yeast or a bacterial cell.

**13.** The transformed cell of claim 12, wherein the host cell is a strain of a *Aspergillus* sp.

**14.** The transformed cell of claim 13 wherein the host cell is a strain of *A. oryzae.*

**15.** The transformed cell of claim 12, wherein the host cell is a strain of *Bacillus, Streptomyces, E. coli or Pseudomonas.*

**16.** The transformed cell of claim 12, wherein the host cell is a strain of *Saccharomyces sp. or Hansenula sp.*

**17.** The transformed cell of claim 16 wherein the host cell is a strain of *Saccharomyces cerevisiae.*

**18.** A process for preparing the lipase of any of claims 1-8 comprising

a) cultivating the transformed cell of any of claims 11-17 under conditions conducive for the production of the lipase, and
b) recovering and optionally purifying the resulting lipase.

**19.** A detergent composition comprising the lipase of any of claims 1-8.

## Patentansprüche

**1.** Lipase besitzend eine der folgenden: N- oder C-terminalen Sequenzen:

a) SPIRR oder
b) RPVSQD oder
c) SPIRM oder
d) SPIRAR oder
e) SPIRPR oder

f) SPIRER oder
g) SPIRK oder
h) SPIKK oder
i) SPIRRP oder
j) SPPRR oder
k) SPIPR oder
l) SPRPR oder
m) SPIR oder
n) SCIRR oder
o) SPIRPRP oder
p) SCPIRPRP oder
q) SPRRPRT oder
r) SPFRPKL oder
s) SPPRRP oder
t) SPIRRE oder
u) SPPRPP oder
v) SPPRPR oder
w) SPPWWP oder
x) SPPWRP oder
y) SPPRWP oder
z) SPPRWP oder
aa) SHWRRW oder
bb) SHWRK oder
cc) SHWRR oder
dd) TAIRPRK oder
ee) STRRPRP oder
ff) GPIRPRP oder
gg) LPFRQRP oder
hh) SRSRHNA oder
ii) IPIRPRR oder
jj) STRRPRP oder
kk) TAIRPRK oder
ll) WRWRWR oder
mm) QPIRR oder
nn) SHWQQ oder
oo) RPRPRPRP oder
pp) SSTRRASPIKK oder
qq) AWWPSPIRPRP oder
rr) APPPRPRPRPRP oder
ss) APPPRTRPRPRS oder
tt) SPKRKPRP oder
uu) SQRIKQRIK oder
vv) SPPPRPRP oder
ww) SPIRPRPRPR oder
xx) SPIRKAWWP oder
yy) APPPKASPRQRP oder
zz) SPIRPRPSPIRPRP oder
aaa) SPPRWPRR oder
bbb) SPPRWPRW oder
ccc) SPPRWPWR oder
ddd) SPPWRPRR oder
eee) SPPWWPRW oder
fff) SPPWWPWR oder
ggg) SPPWWPWW oder
hhh) SPPWRPRPRP.

**2.** Lipase nach Anspruch 1, wobei die N- oder C-terminale Sequenz an eine Lipase aus *Pseudomonas sp.* angehängt ist.

**3.** Lipase nach Anspruch 2, wobei die N- oder C-terminale Sequenz an eine Lipase aus *Pseudomonas cepacia (Burgholderia cepacia)* angehängt ist.

**4.** Lipase nach Anspruch 1, wobei die N- oder C-terminale Sequenz an eine Lipase aus *Humicola sp.* angehängt ist.

**5.** Lipase nach Anspruch 4, wobei die N- oder C-terminale Sequenz an eine Lipase aus *H. lanuginosa* oder *H. insolens* angehängt ist.

**6.** Lipase nach Anspruch 1, welche von einer Aminosäuresequenz der Fig. 1 (SEQ ID NO: 16) abgeleitet ist.

**7.** Lipase nach Anspruch 6, wobei die terminale Sequenz einen ausgetauschten Aminosäurerest E23 in Fig. 1 (SEQ ID NO: 16) hat.

**8.** Lipase nach einem der Ansprüche 1-7, wobei die N- oder C-terminale Sequenz einen Cysteinrest umfasst und der übrige Teil der Lipase einen Cysteinrest umfasst, so dass die zwei Cysteinreste zusammen eine Cystinbrücke bilden.

**9.** DNA-Sequenz kodierend für die Lipase nach einem der Ansprüche 1-8, welche eine N- oder C-terminale Sequenz b)-hhh) besitzt.

**10.** Expressionsvektor, der die DNA-Sequenz nach Anspruch 9 umfasst und weiterhin DNA-Sequenzen umfasst, die die Expression der Lipase erlauben.

**11.** Transformierte Wirtszelle, die eine DNA-Sequenz nach Anspruch 9 oder einen Vektor nach Anspruch 10 birgt.

**12.** Transformierte Zelle nach Anspruch 11, wobei die Wirtszelle ein filamentöser Pilz, eine Hefe- oder eine bakterielle Zelle ist.

**13.** Transformierte Zelle nach Anspruch 12, wobei die Wirtszelle ein Stamm von *Aspergillus sp.* ist.

**14.** Transformierte Zelle nach Anspruch 13, wobei die Wirtszelle ein Stamm von *A. oryzae* ist.

**15.** Transformierte Zelle nach Anspruch 12, wobei die Wirtszelle ein Stamm von *Bacillus, Streptomyces, E. coli* oder *Pseudomonas* ist.

**16.** Transformierte Zelle nach Anspruch 12, wobei die Wirtszelle ein Stamm von *Saccharomyces sp.* oder *Hansenula sp.* ist.

**17.** Transformierte Zelle nach Anspruch 16, wobei die Wirtszelle ein Stamm von *Saccharomyces cerevisiae* ist.

**18.** Verfahren zum Herstellen der Lipase nach einem der Ansprüche 1-8, umfassend

a) Kultivieren der transformierten Zelle nach einem der Ansprüche 11-17 unter Bedingungen, die für die Herstellung der Lipase förderlich sind, und
b) Gewinnen und optional Reinigen der erhaltenen Lipase.

**19.** Waschmittelzusammensetzung umfassend die Lipase nach einem der Ansprüche 1-8.

## Revendications

**1.** Lipase ayant l'une des séquences N- ou C-terminales suivantes :

a) SPIRR ou
b) RPVSQD ou
c) SPIRM ou
d) SPIRAR ou
e) SPIRPR ou

f) SPIRER ou
g) SPIRK ou
h) SPIKK ou
i) SPIRRP ou
j) SPPRR ou
k) SPIPR ou
l) SPRPR ou
m) SPIR ou
n) SCIRR ou
o) SPIRPRP ou
p) SCPIRPRP ou
q) SPRRPRT ou
r) SPFRPKL ou
s) SPPRRP ou
t) SPIRRE ou
u) SPPRPP ou
v) SPPRPR ou
w) SPPWWP ou
x) SPPWRP ou
y) SPPRWP ou
z) SPPRWP ou
aa) SHWRRW ou
bb) SHWRK ou
cc) SHWRR ou
dd) TAIRPRK ou
ee) STRRPRP ou
ff) GPIRPRP ou
gg) LPFRQRP ou
hh) SRSRHNA ou
ii) IPIRPRR ou
jj) STRRPRP ou
kk) TAIRPRK ou
ll) WRWRWR ou
mm) QPIRR ou
nn) SHWQQ ou
oo) RPRPRPRP ou
pp) SSTRRASPIKK ou
qq) AWWPSPIRPRP ou
rr) APPPRPRPRPRP ou
ss) APPPRTRPRPRS ou
tt) SPKRKPRP ou
uu) SQRIKQRIK ou
vv) SPPPRPRP ou
ww) SPIRPRPRPR ou
xx) SPIRKAWWP ou
yy) APPPKASPRQRP ou
zz) SPIRPRPSPIRPRP ou
aaa) SPPRWPRR ou
bbb) SPPRWPRW ou
ccc) SPPRWPWR ou
ddd) SPPWRPRR ou
eee) SPPWWPRW ou
fff) SPPWWPWR ou
ggg) SPPWWPWW ou
hhh) SPPWPRPRP.

**2.** Lipase selon la revendication 1 où la séquence N- ou C-terminale est fixée à une lipase dérivée de *Pseudomonas* sp.

**3.** Lipase selon la revendication 2 où la séquence N- ou C-terminale est fixée à une lipase dérivée de *Pseudomonas cepacia (Burkholderia cepacia).*

**4.** Lipase selon la revendication 1 où la séquence N- ou C-terminale est fixée à une lipase dérivée de *Humicola* sp.

**5.** Lipase selon la revendication 4 où la séquence N- ou C-terminale est fixée à une lipase dérivée de *H. lanuginosa* ou *H. insolens.*

**6.** Lipase selon la revendication 1 qui est dérivée de la séquence d'aminoacides de la figure 1 (SEQ ID NO : 16).

**7.** Lipase selon la revendication 6 où la séquence terminale a le résidu d'aminoacide E23 remplacé sur la figure 1 (SEQ ID NO :16).

**8.** Lipase selon l'une quelconque des revendications 1-7 où la séquence N- ou C-terminale comprend un résidu cystéine et la partie restante de la lipase comprend un résidu cystéine de telle manière que les deux résidus cystéine forment ensemble un pont cystine.

**9.** Séquence d'ADN codant la lipase selon l'une quelconque des revendications 1-8 laquelle lipase a la séquence N- ou C-terminale b)-hhh).

**10.** Vecteur d'expression qui comprend la séquence d'ADN selon la revendication 9 et comprend en outre des séquences d'ADN permettant l'expression de la lipase.

**11.** Cellule hôte transformée contenant la séquence d'ADN selon la revendication 9 ou le vecteur selon la revendication 10.

**12.** Cellule transformée selon la revendication 11 où la cellule hôte est une cellule fongique filamenteuse, de levure ou bactérienne.

**13.** Cellule transformée selon la revendication 12 où la cellule hôte est une souche de *Aspergillus* sp.

**14.** Cellule transformée selon la revendication 13 où la cellule hôte est une souche de *A. oryzae.*

**15.** Cellule transformée selon la revendication 12 où la cellule hôte est une souche de *Bacillus, Streptomyces, E. coli* ou *Pseudomonas.*

**16.** Cellule transformée selon la revendication 12 où la cellule hôte est une souche de *Saccharomyces* sp. ou *Hansenula* sp.

**17.** Cellule transformée selon la revendication 16 où la cellule hôte est une souche de *Saccharomyces cerevisiae.*

**18.** Procédé pour préparer la lipase selon l'une quelconque des revendications 1-8 comprenant

a) la culture de la cellule transformée selon l'une quelconque des revendications 11-17 dans des conditions conduisant à la production de la lipase, et
b) la récupération et éventuellement la purification de la lipase résultante.

**19.** Composition détergente comprenant la lipase selon l'une quelconque des revendications 1-8.

```
    ATGAGGAGCTCCCTTGTGCTGTTCTTTGTCTCTGCGTGGACGGCCTTGGCCAGTCCTATT
1    M  R  S  S  L  V  L  F  F  V  S  A  W  T  A  L  A  S  P  I  -

    CGTCGAGAGGTCTCGCAGGATCTGTTTAACCAGTTCAATCTCTTTGCACAGTATTCTGCA
21   R  R  E  V  S  Q  D  L  F  N  Q  F  N  L  F  A  Q  Y  S  A  -

    GCCGCATACTGCGGAAAAAACAATGATGCCCCAGCTGGTACAAACATTACGTGCACGGGA
41   A  A  Y  C  G  K  N  N  D  A  P  A  G  T  N  I  T  C  T  G  -

    AATGCCTGCCCCGAGGTAGAGAAGGCGGATGCAACGTTTCTCTACTCGTTTGAAGACTCT
61   N  A  C  P  E  V  E  K  A  D  A  T  F  L  Y  S  F  E  D  S  -

    GGAGTGGGCGATGTCACCGGCTTCCTTGCTCTCGACAACACGAACAAATTGATCGTCCTC
81   G  V  G  D  V  T  G  F  L  A  L  D  N  T  N  K  L  I  V  L  -

    TCTTTCCGTGGCTCTCGTTCCATAGAGAACTGGATCGGGAATCTTAACTTCGACTTGAAA
101  S  F  R  G  S  R  S  I  E  N  W  I  G  N  L  N  F  D  L  K  -

    GAAATAAATGACATTTGCTCCGGCTGCAGGGGACATGACGGCTTCACTTCGTCCTGGAGG
121  E  I  N  D  I  C  S  G  C  R  G  H  D  G  F  T  S  S  W  R  -

    TCTGTAGCCGATACGTTAAGGCAGAAGGTGGAGGATGCTGTGAGGGAGCATCCCGACTAT
141  S  V  A  D  T  L  R  Q  K  V  E  D  A  V  R  E  H  P  D  Y  -

    CGCGTGGTGTTTACCGGACATAGCTTGGGTGGTGCATTGGCAACTGTTGCCGGAGCAGAC
161  R  V  V  F  T  G  H  S  L  G  G  A  L  A  T  V  A  G  A  D  -

    CTGCGTGGAAATGGGTATGATATCGACGTGTTTTCATATGGCGCCCCCCGAGTCGGAAAC
181  L  R  G  N  G  Y  D  I  D  V  F  S  Y  G  A  P  R  V  G  N

AGGGCTTTTGCAGAATTCCTGACCGTACAGACCGGCGGAACACTCTACCGCATTACCCAC
201  R  A  F  A  E  F  L  T  V  Q  T  G  G  T  L  Y  R  I  T  H  -

    ACCAATGATATTGTCCCTAGACTCCCGCCGCGCGAATTCGGTTACAGCCATTCTAGCCCA
221  T  N  D  I  V  P  R  L  P  P  R  E  F  G  Y  S  H  S  S  P  -

    GAGTACTGGATCAAATCTGGAACCCTTGTCCCCGTCACCCGAAACGATATCGTGAAGATA
241  E  Y  W  I  K  S  G  T  L  V  P  V  T  R  N  D  I  V  K  I  -

    GAAGGCATCGATGCCACCGGCGGCAATAACCAGCCTAACATTCCGGATATCCCTGCGCAC
261  E  G  I  D  A  T  G  G  N  N  Q  P  N  I  P  D  I  P  A  H  -

    CTATGGTACTTCGGGTTAATTGGGACATGTCTTTAG
281  L  W  Y  F  G  L  I  G  T  C  L  *
```

Fig. 1

ATGAAACGCATTTGTGGTTCCCTGCTGTTGCTCGGTTTGTCGATCAGCGCCGCGCTCGCT
1 M K R I C G S L L L L G L S I S A A L A

AGCCCTATACGTAGAGAGGTCTCGCAGGATCTGTTTAACCAGTTCAATCTCTTTGCACAGTATTCTGCA
21 *S P I R R* E V S Q D L F N Q F N L F A Q Y S A

GCCGCATACTGCGGAAAAAACAATGATGCCCCAGCTGGTACAAACATTACGTGCACGGGA
44 A A Y C G K N N D A P A G T N I T C T G -

AATGCCTGCCCCGAGGTAGAGAAGGCGGATGCAACGTTTCTCTACTCGTTTGAAGACTCT
64 N A C P E V E K A D A T F L Y S F E D S -

GGAGTGGGCGATGTCACCGGCTTCCTTGCTCTCGACAACACGAACAAATTGATCGTCCTC
84 G V G D V T G F L A L D N T N K L I V L -

TCTTTCCGTGGCTCTCGTTCCATAGAGAACTGGATCGGGAATCTTAACTTCGACTTGAAA
104 S F R G S R S I E N W I G N L N F D L K -

GAAATAAATGACATTTGCTCCGGCTGCAGGGGACATGACGGCTTCACTTCGTCCTGGAGG
124 E I N D I C S G C R G H D G F T S S W R -

TCTGTAGCCGATACGTTAAGGCAGAAGGTGGAGGATGCTGTGAGGGAGCATCCCGACTAT
144 S V A D T L R Q K V E D A V R E H P D Y -

CGCGTGGTGTTTACCGGACATAGCTTGGGTGGTGCATTGGCAACTGTTGCCGGAGCAGAC
164 R V V F T G H S L G G A L A T V A G A D -

CTGCGTGGAAATGGGTATGATATCGACGTGTTTTCATATGGCGCCCCCCGAGTCGGAAAC
184 L R G N G Y D I D V F S Y G A P R V G N -

AGGGCTTTTGCAGAATTCCTGACCGTACAGACCGGCGGAACACTCTACCGCATTACCCAC
204 R A F A E F L T V Q T G G T L Y R I T H -

ACCAATGATATTGTCCCTAGACTCCCGCCGCGCGAATTCGGTTACAGCCATTCTAGCCCA
224 T N D I V P R L P P R E F G Y S H S S P -

GAGTACTGGATCAAATCTGGAACCCTTGTCCCCGTCACCCGAAACGATATCGTGAAGATA
244 E Y W I K S G T L V P V T R N D I V K I -

GAAGGCATCGATGCCACCGGCGGCAATAACCAGCCTAACATTCCGGATATCCCTGCGCAC
264 E G I D A T G G N N Q P N I P D I P A H -

CTATGGTACTTCGGGTTAATTGGGACATGTCTTTAG
284 L W Y F G L I G T C L *

Fig. 2

ATGAAACGCATTTGTGGTTCCCTGCTGTTGCTCGGTTTGTCGATCAGCGCCGCGCTCGCC
1 M K R I C G S L L L L G L S I S A A L A

GAGGTCTCGCAGGATCTGTTTAACCAGTTCAATCTCTTTGCACAGTATTCTGCA
21 E V S Q D L F N Q F N L F A Q Y S A

GCCGCATACTGCGGAAAAAACAATGATGCCCCAGCTGGTACAAACATTACGTGCACGGGA
39 A A Y C G K N N D A P A G T N I T C T G -

AATGCCTGCCCCGAGGTAGAGAAGGCGGATGCAACGTTTCTCTACTCGTTTGAAGACTCT
59 N A C P E V E K A D A T F L Y S F E D S -

GGAGTGGGCGATGTCACCGGCTTCCTTGCTCTCGACAACACGAACAAATTGATCGTCCTC
79 G V G D V T G F L A L D N T N K L I V L -

TCTTTCCGTGGCTCTCGTTCCATAGAGAACTGGATCGGGAATCTTAACTTCGACTTGAAA
99 S F R G S R S I E N W I G N L N F D L K -

GAAATAAATGACATTTGCTCCGGCTGCAGGGGACATGACGGCTTCACTTCGTCCTGGAGG
119 E I N D I C S G C R G H D G F T S S W R -

TCTGTAGCCGATACGTTAAGGCAGAAGGTGGAGGATGCTGTGAGGGAGCATCCCGACTAT
139 S V A D T L R Q K V E D A V R E H P D Y -

CGCGTGGTGTTTACCGGACATAGCTTGGGTGGTGCATTGGCAACTGTTGCCGGAGCAGAC
159 R V V F T G H S L G G A L A T V A G A D -

CTGCGTGGAAATGGGTATGATATCGACGTGTTTTCATATGGCGCCCCCCGAGTCGGAAAC
179 L R G N G Y D I D V F S Y G A P R V G N -

AGGGCTTTTGCAGAATTCCTGACCGTACAGACCGGCGGAACACTCTACCGCATTACCCAC
199 R A F A E F L T V Q T G G T L Y R I T H -

ACCAATGATATTGTCCCTAGACTCCCGCCGCGCGAATTCGGTTACAGCCATTCTAGCCCA
219 T N D I V P R L P P R E F G Y S H S S P -

GAGTACTGGATCAAATCTGGAACCCTTGTCCCCGTCACCCGAAACGATATCGTGAAGATA
239 E Y W I K S G T L V P V T R N D I V K I -

GAAGGCATCGATGCCACCGGCGGCAATAACCAGCCTAACATTCCGGATATCCCTGCGCAC
259 E G I D A T G G N N Q P N I P D I P A H -

CTATGGTACTTCGGGTTAATTGGGACATGTCTTTAG
279 L W Y F G L I G T C L *

Fig. 3

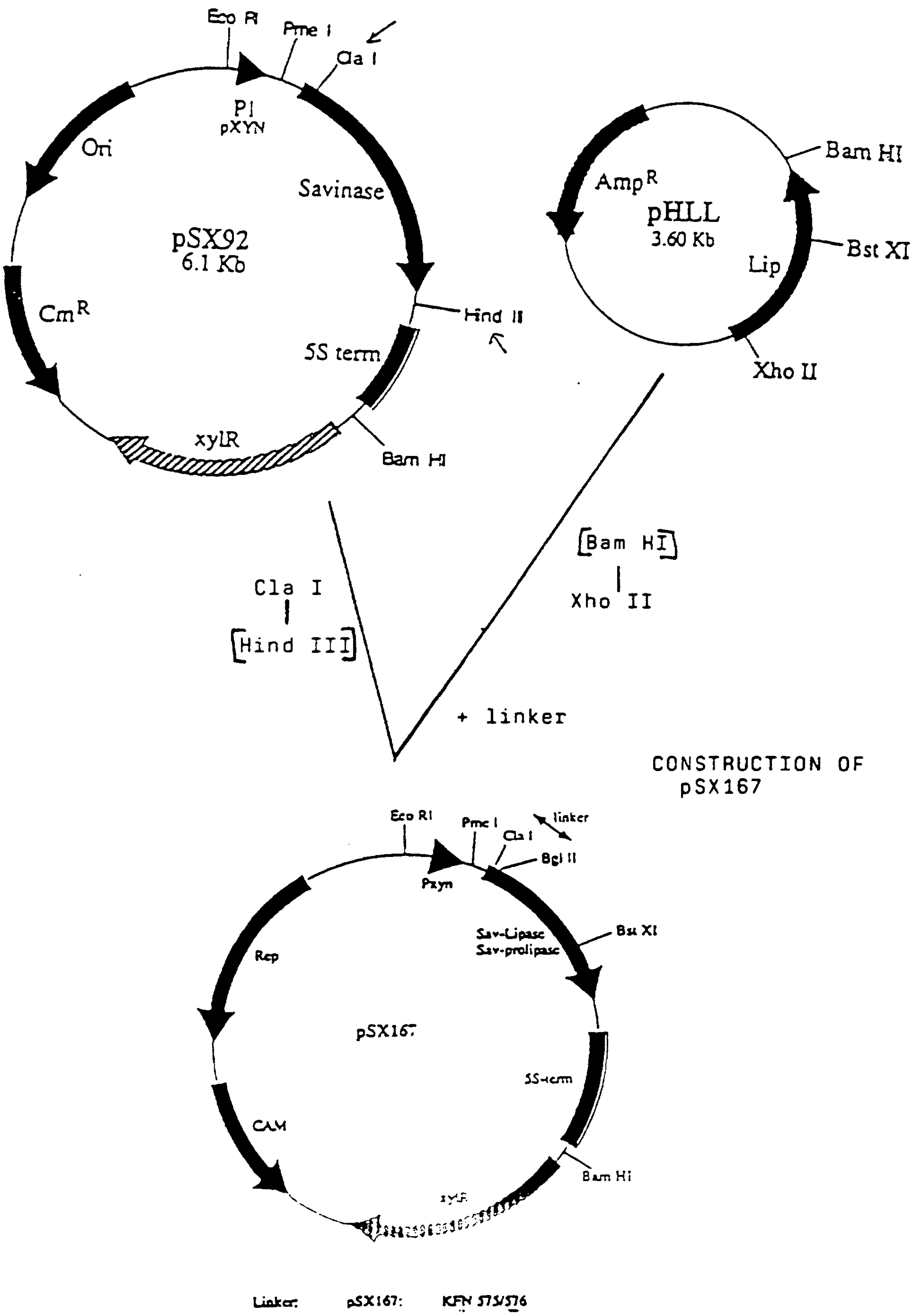
Eco RI
Pme I
Cla I
PI
pXYN
Ori
Savinase
pSX92
6.1 Kb
CmR
Hind III
5S term
xylR
Bam HI
AmpR
pHLL
3.60 Kb
Bam HI
Bst XI
Lip
Xho II
[Bam HI]
Xho II
Cla I
[Hind III]
+ linker
CONSTRUCTION OF pSX167
Eco RI
Pme I
linker
Cla I
Bgl II
Pxyn
Sav-Lipase
Sav-prolipase
Bst XI
Rep
pSX167
5S-term
CAM
Bam HI
xylR
Linker: pSX167: KFN 575/576

Fig. 4

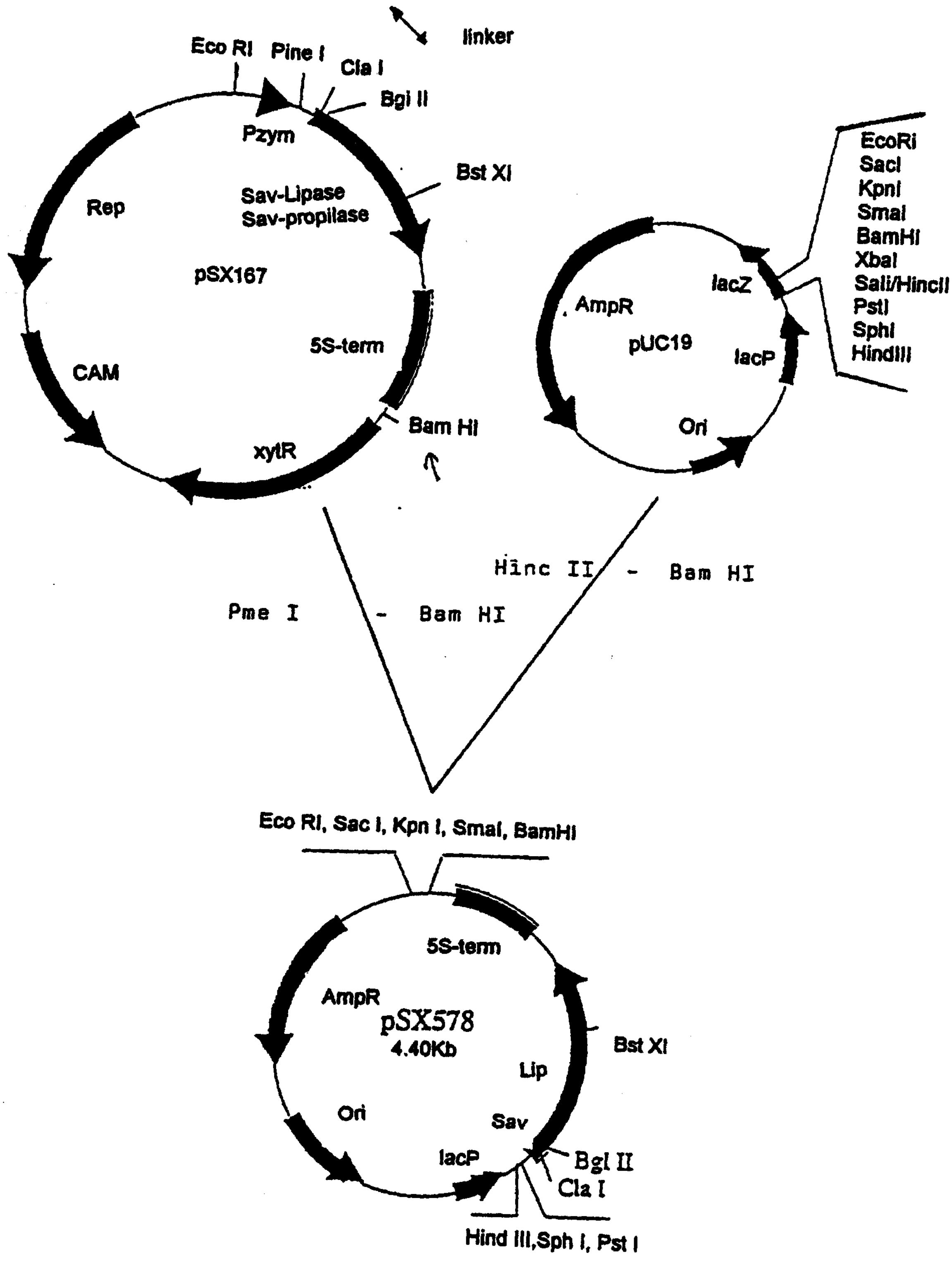


Construction of pSX578

Fig. 5

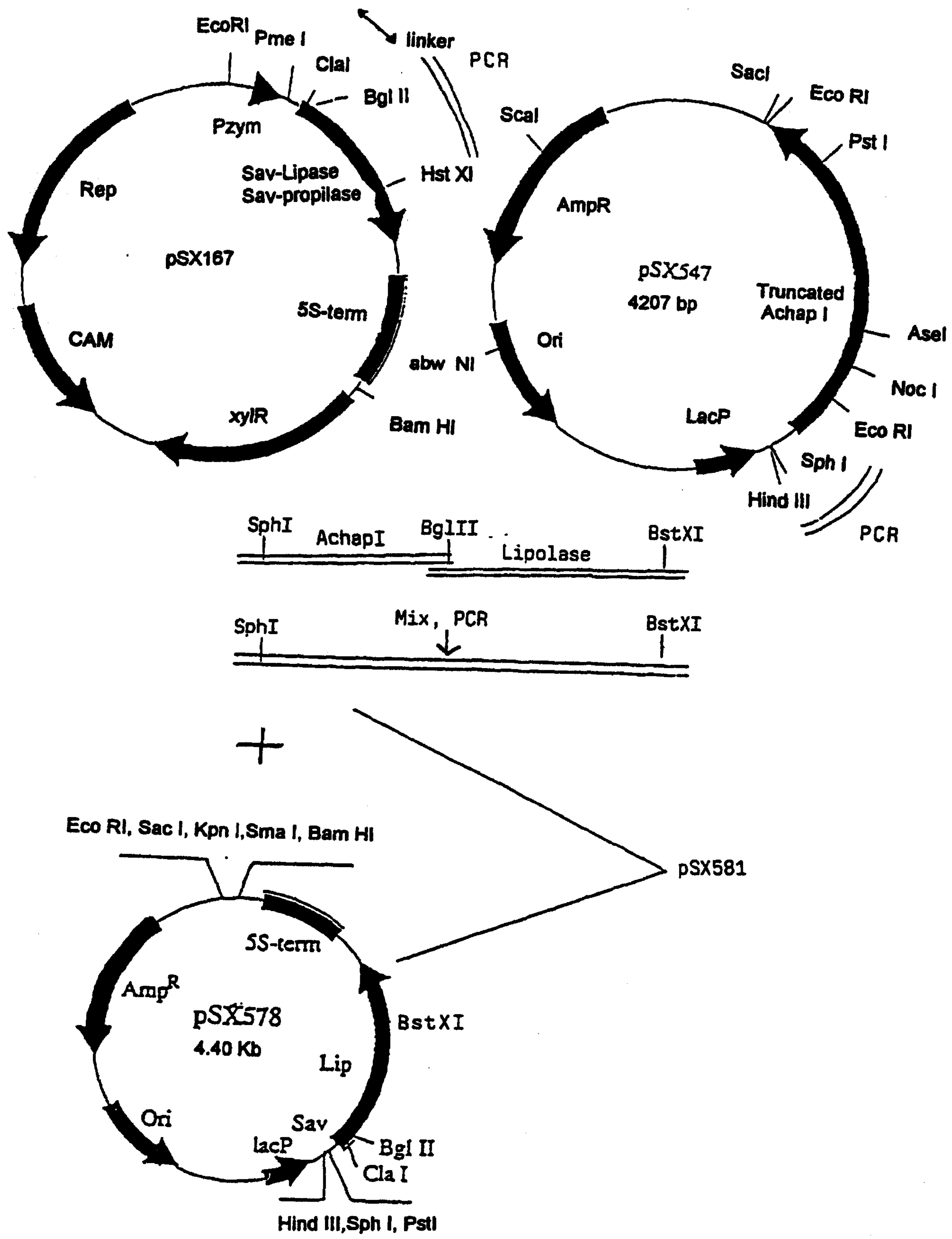


Construction of pSX578

Fig. 6

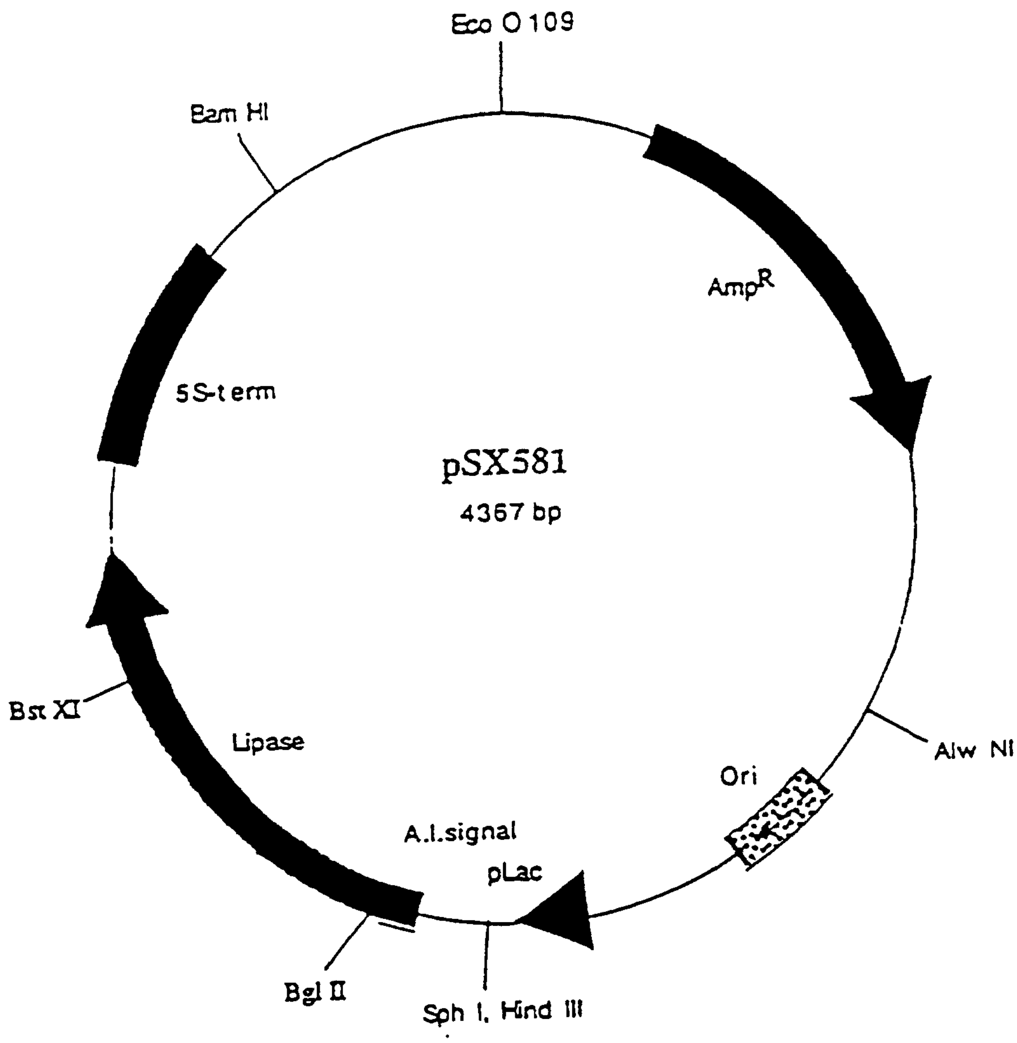
Eco O 109
Bam HI
AmpR
5S-term
pSX581
4367 bp
Bst XI
Lipase
Alw NI
Ori
A.I.signal
pLac
Bgl II
Sph I, Hind III

pSX581

Fig. 7

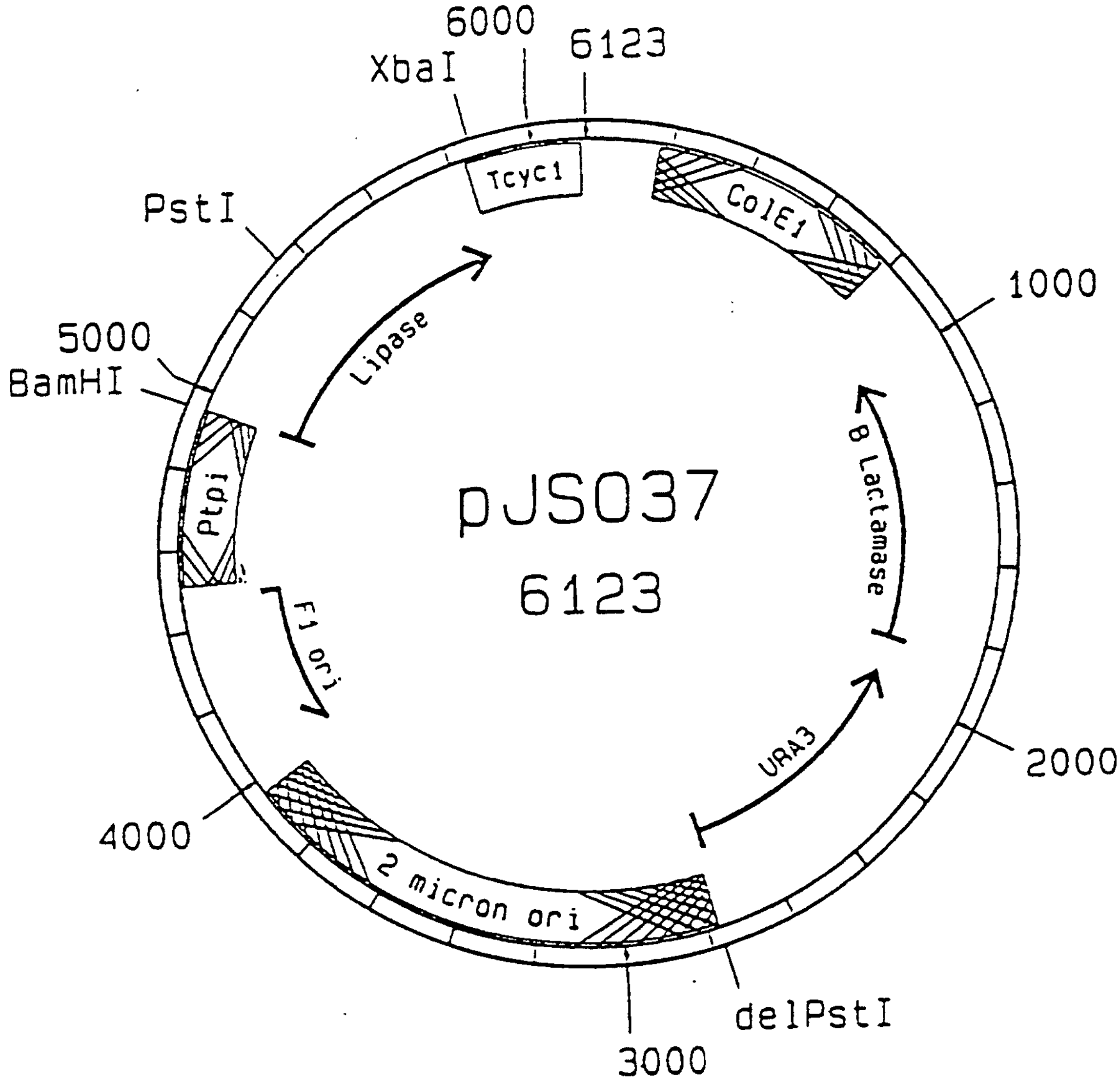
6000
6123
XbaI
Tcyc1
ColE1
PstI
1000
Lipase
5000
BamHI
B Lactamase
Ptpi
pJS037
6123
F1 ori
URA3
2000
4000
2 micron ori
delPstI
3000

pJS037

Fig. 8

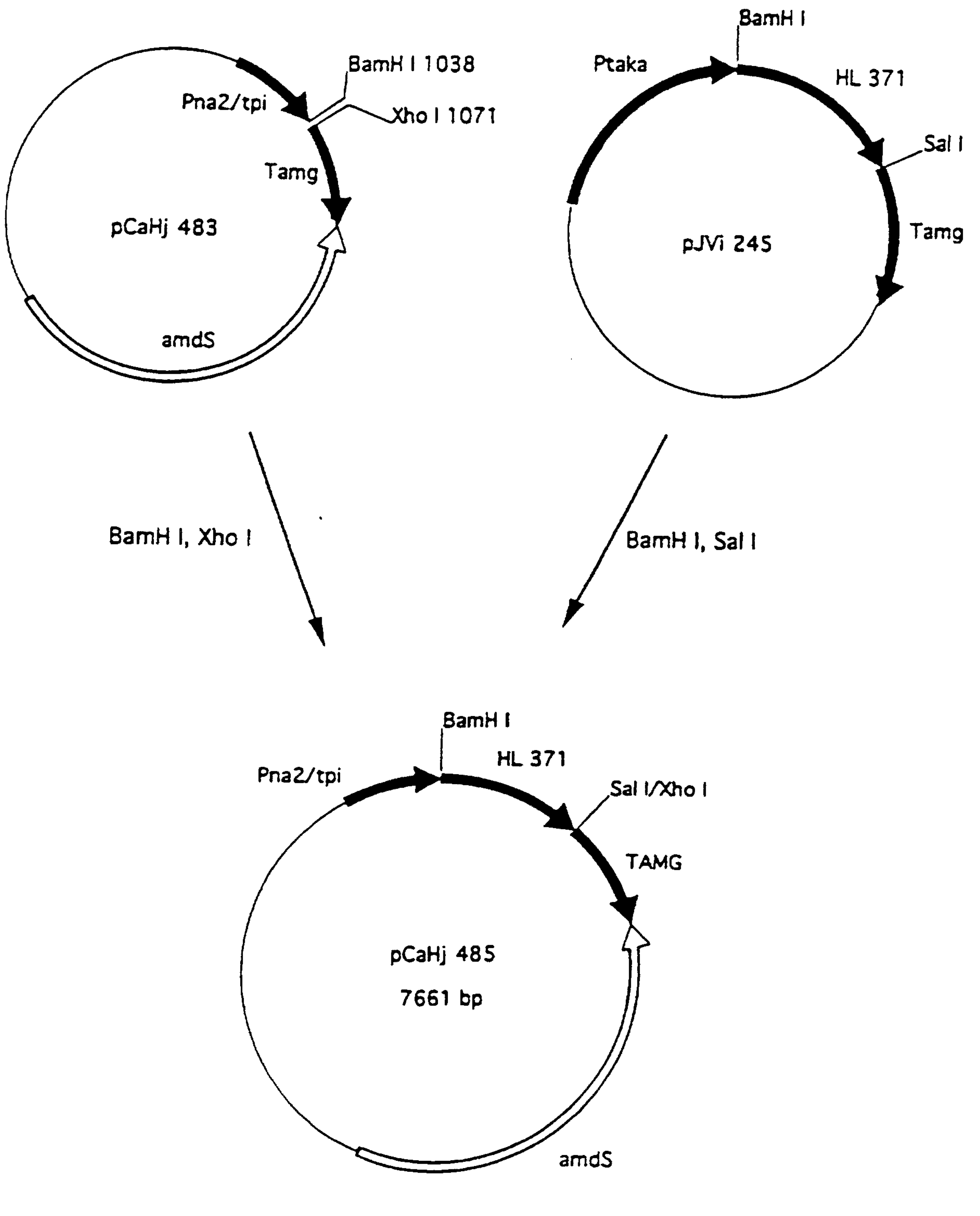
Pna2/tpi
BamH I 1038
Xho I 1071
Tamg
pCaHj 483
amdS
BamH I
Ptaka
HL 371
Sal I
Tamg
pJVi 245
BamH I, Xho I
BamH I, Sal I
BamH I
Pna2/tpi
HL 371
Sal I/Xho I
TAMG
pCaHj 485
7661 bp
amdS

Fig. 9

SEQUENCE LISTING

(1) GENERAL INFORMATION:

(i) APPLICANT:

(A) NAME: Novo Nordisk A/S
(B) STREET: Novo Alle
(C) CITY: Basvaerd
(E) COUNTRY: Denmark
(F) POSTAL CODE (ZIP): DK-2880
(G) TELEPHONE: +45 4444 8888
(H) TELEFAX: + 45 4449 3256

(ii) TITLE OF INVENTION: A modified enzyme with lipolytic activity
(iii) NUMBER OF SEQUENCES: 92
(iv) (iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "synthetic DNA"

(xi) SEQUENCE DESCRIPTION: SEQ ID No. 1:

GAATGACTTG GTTGACGCGT CACCAGTCAC 30

(2) INFORMATION FOR SEQ ID No. 2:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Synthetic DNA"

(xi) SEQUENCE DESCRIPTION: SEQ ID NO: 2:

TCTAGCCCAG AATACTGGAT CAAATC 26

(2) INFORMATION FOR SEQ ID NO: 3:

(i) SEQUENCE CHARACTERISTICS:

(A) LENGTH: 55 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 1"

(xi) SEQUENCE DESCRIPTION: SEQ ID NO: 3:

AACAGATCTT GCGAGACCTC TCTACGTATA GGGCTAGCGA
GCGCGGCGCT GATCG 55

(2) INFORMATION FOR SEQ ID NO: 4:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 2"

(xi) SEQUENCE DESCRIPTION: SEQ ID NO: 4:

GTTGTGTGGA ATTGTGAGCG G 21

(2) INFORMATION FOR SEQ ID NO: 5:

(i) SEQUENCE CHARACTERISTICS:

(A) LENGTH: 54 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = -Oligo 8479"

(xi) SEQUENCE DESCRIPTION: SEQ ID NO: 5:

**GCGTGGACGG CCTTGGCTAG CCCTATTCGT CCTCGACCGG TCTCGCAGGA**
**TCTG** **54**

(2) INFORMATION FOR SEQ ID NO: 6:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = 'Primer 3"
(xi) SEQUENCE DESCRIPTION: SEQ ID NO: 6:

**AGAAATCGGG TATCCTTTCA G** **21**

(2) INFORMATION FOR SEQ ID NO: 7:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid

(A) DESCRIPTION: /dese = *Primer LWN9476*

(xi) SEQUENCE DESCRIPTION: SEQ ID NO: 7:

**CGAATTCGAT GCGTTCCAGG GTGGTGGCAG G** **21**

(2) INFORMATION FOR SEQ ID NO: 8:

(i) SEQUENCE CHARACTERISTICS:

(A) LENGTH: 74 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer LWN9472"

(xi) SEQUENCE DESCRIPTION: SEQ ID NO: 8:

CGAATTCACG CGTCGCCGCG TAGCCAGCGG CCGGGCGCGG GCGGATCGGG
CTGGGCGCGG TGGCCGCCAT TGCC 74

(2) INFORMATION FOR SEQ ID NO: 9:

(i) SEQUENCE CHARACTERISTICS:

(A) LENGTH: 74 base pairs
(B) TYPE: nucleic acid
(C) STRANDEDNESS: single
(D) TOPOLOGY: linear

(ii) MOLECULE TYPE: other nucleic add

(A) DESCRIPTION: /desc = "Primer LWN9473"

(xi) SEQUENCE DESCRIPTION: SEQ ID NO: 9:

CGAATTCACG CGTCGCCGCG TAGCCAGCGG CCTTGCGCGG GCGGATCGCC
GTGGGCGCGG TGGCCGCCAT TGCC 74

(2) INFORMATION FOR SEQ ID NO: 10:

(i) SEQUENCE CHARACTERISTICS:

(A) LENGTH: 74 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer LWN9471"

(xi) SEQUENCE DESCRIPTION: SEQ ID NO: 10:

CGAATTCACG CGTCGCCGCG TAGCCAGCGG CCGGGCGCGG ACGGCGCGTC
GAGGGCGCGG TGGCCGCCAT TGCC 74

(2) INFORMATION FOR SEQ ID NO: 11:

(i) SEQUENCE CHARACTERISTICS:

(A) LENGTH: 74 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = Primer LWN9474"

(xi) SEQUENCE DESCRIPTION: SEQ ID NO: 11:

CGAATTCACG CGTCGCCGCG TAGCCAGCGG CCGGGCGCGG GCGGATCGGG
CCGGGCGCGG TGGCCGCCAT TGCC 74

(2) INFORMATION FOR SEQ ID NO: 12:

(i) SEQUENCE CHARACTERISTICS:

(A) LENGTH: 68 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer LWN9475"

(xi) SEQUENCE DESCRIPTION: SEQ ID NO: 12:

CGAATTCACG CGTCGCCGCG TAGCCAGCGG CCCGGCGGAT CGGGCTGGGC GCGGTGGCCG
CCATTGCC 68

(2) INFORMATION FOR SEQ ID NO: 13:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = *Primer LWN9470*

(xi) SEQUENCE DESCRIPTION: SEQ ID NO: 13:

CGAATTCACG CGTCGCCGCG TAGCCAGCGG CCGGACGCGG CCTGGGACGC
GGGCGGGGCG CGGTGGCCGC CATTGCC 77

(2) INFORMATION FOR SEQ ID NO: 14:

(i) SEQUENCE CHARACTERISTICS:

(A) LENGTH: 105 base pairs
(B) TYPE: nucleic acid
(C) STRANDEDNESS : single
(D) TOPOLOGY: linear

(ii) MOLECULE TYPE : other nucleic acid

(A) DESCRIPTION: /desc = "Primer 4"

(xi) SEQUENCE DESCRIPTION: SEQ ID NO: 14:

```
GCTCCTCATG GTGGATCCCC AGTTGTGTAT ATAGAGGATT
GAGGAAGGAA GAGAAGTGTG GATAGAGGTA AATTGAGTTG
GAAACTCCAA GCATGGCATC CTTGC                        105
```

(2) INFORMATION FOR SEQ ID NO: 15:

(i) SEQUENCE CHARACTERISTICS:

(A) LENGTH: 876 base pairs
(B) TYPE: nucleic acid
(C) STRANDEDNESS: single
(D) TOPOLOGY: linear

(ii) MOLECULE TYPE: DNA (genomic)
(vi) ORIGINAL SOURCE:

(B) STRAIN: Humicola lanuginosa DSM 4109

(ix) FEATURE:

(A) NAME/KEY: CDS
(B) LOCATION:1..876

(ix) FEATURE:

(A) NAME/KEY: sig_peptide
(B) LOCATION:1..66

(xi) SEQUENCE DESCRIPTION: SEQ ID NO: 15:

```
ATG AGG AGC TCC CTT GTG CTG TTC TTT GTC TCT GCG TGG ACG GCC TTG      48
Met Arg Ser Ser Leu Val Leu Phe Phe Val Ser Ala Trp Thr Ala Leu
  1               5                  10                  15

GCC AGT CCT ATT CGT CGA GAG GTC TCG CAG GAT CTG TTT AAC CAG TTC      96
Ala Ser Pro Ile Arg Arg Glu Val Ser Gln Asp Leu Phe Asn Gln Phe
             20                  25                  30

AAT CTC TTT GCA CAG TAT TCT GCA GCC GCA TAC TGC GGA AAA AAC AAT     144
Asn Leu Phe Ala Gln Tyr Ser Ala Ala Ala Tyr Cys Gly Lys Asn Asn
         35                  40                  45

GAT GCC CCA GCT GGT ACA AAC ATT ACG TGC ACG GGA AAT GCC TGC CCC     192
Asp Ala Pro Ala Gly Thr Asn Ile Thr Cys Thr Gly Asn Ala Cys Pro
     50                  55                  60

GAG GTA GAG AAG GCG GAT GCA ACG TTT CTC TAC TCG TTT GAA GAC TCT     240
Glu Val Glu Lys Ala Asp Ala Thr Phe Leu Tyr Ser Phe Glu Asp Ser
 65                  70                  75                  80

GGA GTG GGC GAT GTC ACC GGC TTC CTT GCT CTC GAC AAC ACG AAC AAA     288
Gly Val Gly Asp Val Thr Gly Phe Leu Ala Leu Asp Asn Thr Asn Lys
                 85                  90                  95

TTG ATC GTC CTC TCT TTC CGT GGC TCT CGT TCC ATA GAG AAC TGG ATC     336
Leu Ile Val Leu Ser Phe Arg Gly Ser Arg Ser Ile Glu Asn Trp Ile
            100                 105                 110

GGG AAT CTT AAC TTC GAC TTG AAA GAA ATA AAT GAC ATT TGC TCC GGC     384
Gly Asn Leu Asn Phe Asp Leu Lys Glu Ile Asn Asp Ile Cys Ser Gly
        115                 120                 125

TGC AGG GGA CAT GAC GGC TTC ACT TCG TCC TGG AGG TCT GTA GCC GAT     432
Cys Arg Gly His Asp Gly Phe Thr Ser Ser Trp Arg Ser Val Ala Asp
```

```
        130                 135                 140

ACG TTA AGG CAG AAG GTG GAG GAT GCT GTG AGG GAG CAT CCC GAC TAT      480
Thr Leu Arg Gln Lys Val Glu Asp Ala Val Arg Glu His Pro Asp Tyr
145                 150                 155                 160

CGC GTG GTG TTT ACC GGA CAT AGC TTG GGT GGT GCA TTG GCA ACT GTT      528
Arg Val Val Phe Thr Gly His Ser Leu Gly Gly Ala Leu Ala Thr Val
                165                 170                 175

GCC GGA GCA GAC CTG CGT GGA AAT GGG TAT GAT ATC GAC GTG TTT TCA      576
Ala Gly Ala Asp Leu Arg Gly Asn Gly Tyr Asp Ile Asp Val Phe Ser
            180                 185                 190

TAT GGC GCC CCC CGA GTC GGA AAC AGG GCT TTT GCA GAA TTC CTG ACC      624
Tyr Gly Ala Pro Arg Val Gly Asn Arg Ala Phe Ala Glu Phe Leu Thr
        195                 200                 205

GTA CAG ACC GGC GGA ACA CTC TAC CGC ATT ACC CAC ACC AAT GAT ATT      672
Val Gln Thr Gly Gly Thr Leu Tyr Arg Ile Thr His Thr Asn Asp Ile
    210                 215                 220

GTC CCT AGA CTC CCG CCG CGC GAA TTC GGT TAC AGC CAT TCT AGC CCA      720
Val Pro Arg Leu Pro Pro Arg Glu Phe Gly Tyr Ser His Ser Ser Pro
225                 230                 235                 240

GAG TAC TGG ATC AAA TCT GGA ACC CTT GTC CCC GTC ACC CGA AAC GAT      768
Glu Tyr Trp Ile Lys Ser Gly Thr Leu Val Pro Val Thr Arg Asn Asp
                245                 250                 255

ATC GTG AAG ATA GAA GGC ATC GAT GCC ACC GGC GGC AAT AAC CAG CCT      816
Ile Val Lys Ile Glu Gly Ile Asp Ala Thr Gly Gly Asn Asn Gln Pro
            260                 265                 270

AAC ATT CCG GAT ATC CCT GCG CAC CTA TGG TAC TTC GGG TTA ATT GGG      864
Asn Ile Pro Asp Ile Pro Ala His Leu Trp Tyr Phe Gly Leu Ile Gly
        275                 280                 285

ACA TGT CTT TAG                                                      876
Thr Cys Leu  *
    290
```

(2) INFORMATION FOR SEQ ID NO: 16:

(i) SEQUENCE CHARACTERISTICS:

(A) LENGTH: 292 amino acids
(B) TYPE: amino acid
(D) TOPOLOGY: linear

(ii) MOLECULE TYPE: protein
(xi) SEQUENCE DESCRIPTION: SEQ ID NO: 16:

```
Met Arg Ser Ser Leu Val Leu Phe Phe Val Ser Ala Trp Thr Ala Leu
 1               5                  10                  15

Ala Ser Pro Ile Arg Arg Glu Val Ser Gln Asp Leu Phe Asn Gln Phe
            20                  25                  30
```

```
Asn Leu Phe Ala Gln Tyr Ser Ala Ala Ala Tyr Cys Gly Lys Asn Asn
            35                  40                  45

Asp Ala Pro Ala Gly Thr Asn Ile Thr Cys Thr Gly Asn Ala Cys Pro
        50                  55                  60

Glu Val Glu Lys Ala Asp Ala Thr Phe Leu Tyr Ser Phe Glu Asp Ser
65                  70                  75                  80

Gly Val Gly Asp Val Thr Gly Phe Leu Ala Leu Asp Asn Thr Asn Lys
                85                  90                  95

Leu Ile Val Leu Ser Phe Arg Gly Ser Arg Ser Ile Glu Asn Trp Ile
            100                 105                 110

Gly Asn Leu Asn Phe Asp Leu Lys Glu Ile Asn Asp Ile Cys Ser Gly
        115                 120                 125

Cys Arg Gly His Asp Gly Phe Thr Ser Ser Trp Arg Ser Val Ala Asp
    130                 135                 140

Thr Leu Arg Gln Lys Val Glu Asp Ala Val Arg Glu His Pro Asp Tyr
145                 150                 155                 160

Arg Val Val Phe Thr Gly His Ser Leu Gly Gly Ala Leu Ala Thr Val
                165                 170                 175

Ala Gly Ala Asp Leu Arg Gly Asn Gly Tyr Asp Ile Asp Val Phe Ser
            180                 185                 190

Tyr Gly Ala Pro Arg Val Gly Asn Arg Ala Phe Ala Glu Phe Leu Thr
        195                 200                 205

Val Gln Thr Gly Gly Thr Leu Tyr Arg Ile Thr His Thr Asn Asp Ile
    210                 215                 220

Val Pro Arg Leu Pro Pro Arg Glu Phe Gly Tyr Ser His Ser Ser Pro
225                 230                 235                 240

Glu Tyr Trp Ile Lys Ser Gly Thr Leu Val Pro Val Thr Arg Asn Asp
                245                 250                 255

Ile Val Lys Ile Glu Gly Ile Asp Ala Thr Gly Gly Asn Asn Gln Pro
            260                 265                 270

Asn Ile Pro Asp Ile Pro Ala His Leu Trp Tyr Phe Gly Leu Ile Gly
        275                 280                 285

Thr Cys Leu  *
    290
```

(2) INFORMATION FOR SEQ ID NO: 17:

(i) SEQUENCE CHARACTERISTICS:

(A) LENGTH: 6 amino acids
(B) TYPE: amino acid

(D) TOPOLOGY: linear

(ii) MOLECULE TYPE: peptide addition

(xi) SEQUENCE DESCRIPTION: SEQ ID NO: 17:

Arg-Pro-Val-Ser-Gln-Asp

5

(2) INFORMATION FOR SEQ ID NO: 18:

(i) SEQUENCE CHARACTERISTICS:

(A) LENGTH: 5 amino acids

(B) TYPE: amino acid

(D) TOPOLOGY: linear

(ii) MOLECULE TYPE: peptide addition

(xi) SEQUENCE DESCRIPTION: SEQ ID NO: 18:

Ser-Pro-Ile-Arg-Met

5

(2) INFORMATION FOR SEQ ID NO: 19:

(i) SEQUENCE CHARACTERISTICS:

(A) LENGTH: 6 amino acids

(B) TYPE: amino acid

(D) TOPOLOGY: linear

(ii) MOLECULE TYPE: peptide addition

(xi) SEQUENCE DESCRIPTION: SEQ ID NO: 19:

Ser-Pro-Ile-Arg-Ala-Arg

(2) INFORMATION FOR SEQ ID NO: 20:

(i) SEQUENCE CHARACTERISTICS:

(A) LENGTH: 6 amino acids

(B) TYPE: amino acid

(D) TOPOLOGY: linear

(ii) MOLECULE TYPE: peptide addition

(xi) SEQUENCE DESCRIPTION: SEQ ID NO: 20:

Ser-Pro-Ile-Arg-Pro-Arg

5

(2) INFORMATION FOR SEQ ID NO: 21:

(i) SEQENCE CHARACTERISTICS:

(A) LENGTH : 6 amino acids
(B) TYPE: amino acid
(D) TOPOLOGY: linear

(ii) MOLECULE TYPE: peptide addition
(xi) SEQUENCE DESCRIPTION: SEQ ID NO: 21:

Ser-Pro-Ile-Arg-Glu-Arg
5

(2) INFORMATION FOR SEQ ID NO: 22:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 22:

Ser-Pro-Ile-Arg-Lys
5

(2) INFORMATION FOR SEQ ID NO: 23:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 23:

Ser-Pro-Ile-Lys-Lys
5

(2) INFORMATION FOR SEQ ID NO: 24:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 24:

Ser-Pro-Ile-Arg-Arg-Pro
5

(2) INFORMATION FOR SEQ ID NO: 25:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 25:

Ser-Pro-Pro-Arg-Arg
5

(2) INFORMATION FOR SEQ ID NO: 26:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 26:

Ser-Pro-Iso-Pro-Arg
5

(2) INFORMATION FOR SEQ ID NO: 27:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 27:

Ser-Pro-Arg-Pro-Arg
5

(2) INFORMATION FOR SEQ ID NO: 28:

(i) SEQUENCE CHARACTERISTICS:

(A) LENGTH: 4 amino acids
(B) TYPE: amino acid

(D) TOPOLOGY: linear

(ii) MOLECULE TYPE: peptide addition

(xi) SEQUENCE DESCRIPTION: SEQ ID NO: 28:

Ser-Pro-Ile-Arg

(2) INFORMATION FOR SEQ ID NO: 29:

(i) SEQUENCE CHARACTERISTICS:

(A) LENGTH: 5 amino acids
(B) TYPE: amino acid
(D) TOPOLOGY: linear

(ii) MOLECULE TYPE: peptide addition

(iii) SEQUENCE DESCRIPTION SEQ ID NO: 29:

Ser-Pro-Ile-Arg-Arg
5

(2) INFORMATION FOR SEQ ID NO: 30:

(i) SEQUENCE CHARACTERISTICS:

(A) LENGTH: 4 amino acids
(B) TYPE: amino acid
(D) TOPOLOGY: linear

(ii) MOLECULE TYPE: peptide addition

(xi) SEQUENCE DESCRIPTION: SEQ ID NO: 30:

Ser-Cys-Ile-Arg-Arg
5

(2) INFORMATION FOR SEQ ID NO: 31:

(i) SEQUENCE CHARACTERISTICS:

(A) LENGTH: 7 amino acids
(B) TYPE: amino acid
(D) TOPOLOGY: linear

(ii) MOLECULE TYPE: peptide addition

(xi) SEQUENCE DESCRIPTION: SEQ ID NO: 31:

Ser-Pro-Ile-Arg-Pro-Arg-Pro
5

(2) INFORMATION FOR SEQ ID NO: 32:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 32:

**Ser-Cys-Ile-Arg-Pro-Arg-Pro**
**5**

(2) INFORMATION FOR SEQ ID NO: 33:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 33:

**Ser-Pro-Arg-Arg-Pro-Arg-Thr**
**5**

(2) INFORMATION FOR SEQ ID NO: 34:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 34:

**Ser-Pro-Phe-Arg-Pro-Lys-Leu**
**5**

(2) INFORMATION FOR SEQ ID NO: 35:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 35:

Ser-Pro-Pro-Arg-Arg-Pro
5

(2) INFORMATION FOR SEQ ID NO: 36:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 36:

Ser-Pro-Ile-Arg-Arg-Glu
5

(2) INFORMATION FOR SEQ ID NO: 37:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 37:

Ser-Pro-Pro-Arg-Pro-Pro
5

(2) INFORMATION FOR SEQ ID NO: 38:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 38:

Ser-Pro-Pro-Arg-Pro-Arg
5

(2) INFORMATION FOR SEQ ID NO: 39:

(i) SEQUENCE CHARACTERISTICS:

(A) LENGTH: 6 amino acids

(B) TYPE: amino acid
(D) TOPOLOGY: linear

(ii) MOLECULE TYPE: peptide addition
(xi) SEQUENCE DESCRIPTION: SEQ ID NO: 39:

Ser-Pro-Pro-Trp-Trp-Pro
5

(2) INFORMATION FOR SEQ ID NO: 40:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 40:

Ser-Pro-Pro-Trp-Arg-Pro
5

(2) INFORMATION FOR SEQ ID NO: 41:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 41:

Ser-Pro-Pro-Arg-Trp-Pro
5

(2) INFORMATION FOR SEQ. ID NO: 42:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 42:

Ser-Pro-Pro-Arg-Trp-Pro
5

(2) INFORMATION FOR SEQ ID NO: 43:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 43:

Ser-His-Trp-Arg-Arg-Trp
5

(2) INFORMATION FOR SEQ ID NO: 44:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 44:

Ser-His-Trp-Arg-Lys
5

(2) INFORMATION FOR SEQ ID NO: 45:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 45:

Ser-His-Trp-Arg-Arg
5

(2) INFORMATION FOR SEQ ID NO: 46:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 46:

Thr-Ala-Ile-Arg-Pro-Arg-Lys

5

(2) INFORMATION FOR SEQ ID NO: 47:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 47:

Ser-Thr-Arg-Arg-Pro-Arg-Pro

5

(2) INFORMATION FOR SEQ ID NO: 48:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 48:

Gly-Pro-Ile-Arg-Pro-Arg-Pro

5

(2) INFORMATION FOR SEQ ID NO: 49:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 49:

Leu-Pro-Phe-Arg-Glu-Arg-Pro

5

(2) INFORMATION FOR SEQ ID NO: 50:

(i) SEQUENCE CHARACTERISTICS:

(A) LENGTH: 7 amino acids

(B) TYPE: amino acid
(D) TOPOLOGY: linear

(ii) MOLECULE TYPE: peptide addition
(xi) SEQUENCE DESCRIPTION: SEQ ID NO: 50:

Ser-Arg-Ser-Arg-His-Asp-Ala
5

(2) INFORMATION FOR SEQ ID NO: 51:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 51:

Ile-Pro-Ile-Arg-Pro-Arg-Arg
5

(2) INFORMATION FOR SEQ ID NO: 52:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 52:

Ser-Thr-Arg-Arg-Pro-Arg-Pro
5

(2) INFORMATION FOR SEQ ID NO: 53:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 53:

Thr-Ala-Ile-Arg-Pro-Arg-Lys
5

(2) INFORMATION FOR SEQ ID NO: 54:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 54:

Trp-Arg-Trp-Arg-Trp-Arg
5

(2) INFORMATION FOR SEQ ID NO: 55:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 55:

Glu-Pro-Ile-Arg-Arg
5

(2) INFORMATION FOR SEQ ID NO: 56:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 56:

Ser-His-Trp-Glu-Glu
5

(2) INFORMATION FOR SEQ ID NO: 57:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 57:

Arg-Pro-Arg-Pro-Arg-Pro-Arg-Pro
5

(2) INFORMATION FOR SEQ ID NO: 58:

(i) SEQUENCE CHARACTERISTICS:

(A) LENGTH: 11 amino acids
(B) TYPE: amino acid
(D) TOPOLOGY: linear

(ii) MOLECULE TYPE: peptide addition
(xi) SEQUENCE DESCRIPTION: SEQ ID NO: 58:

Ser-Ser-Thr-Arg-Arg-Ala-Ser-Pro-Ile-Lys-Lys
5 10

(2) INFORMATION FOR SEQ ID NO: 59:

(i) SEQUENCE CHARACTERISTICS:

(A) LENGTH: 11 amino acids
(B) TYPE: amino acid
(D) TOPOLOGY: linear

(ii) MOLECULE TYPE: peptide addition
(xi) SEQUENCE DESCRIPTION: SEQ ID NO: 59:

Ala-Trp-Trp-Pro-Ser-Pro-Ile-Arg-Pro-Arg-Pro
5 10

(2) INFORMATION FOR SEQ ID NO: 60:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 60:

Ala-Pro-Pro-Pro-Arg-Pro-Arg-Pro-Arg-Pro-Arg-Pro
5 10

(2) INFORMATION FOR SEQ ID NO: 61:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 61:

Ala-Pro-Pro-Pro-Arg-Thr-Arg-Pro-Arg-Pro-Arg-Ser
5 10

(2) INFORMATION FOR SEO ID NO: 62:

(i) SEQUENCE CHARACTERISTICS:

(A) LENGHT: 8 amino acids
(B) TYPE: amino acid
(D) TOPOLOGY: linear

(ii) MOLECULE TYPE: peptide addition
(xi) SEQUENCE DESCRIPTION: SEQ ID NO: 62:

Ser-Pro-Lys-Arg-Lys-Pro-Arg-Pro
5

(2) INFORMATION FOR SEQ ID NO: 63:

(i) SEQUENCE CHARACTERISTICS:

(A) LENGTH: 9 amino acids
(B) TYPE: amino acid
(D) TOPOLOGY: linear

(ii) MOLECULE TYPE: peptide addition
(xi) SEQUENCE DESCRIPTION: SEQ ID NO: 63:

Ser-Gln-Arg-Ile-Lys-Gln-Arg-Ile-Lys
5

(2) INFORMATION FOR SEQ ID NO: 64:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 64:

Ser-Pro-Pro-Pro-Arg-Pro-Arg-Pro
5

(2) INFORMATION FOR SEQ ID NO: 65:

(i) SEQUENCE CHARACTERISTICS:

(A) LENGTH: 10 amino acids
(B) TYPE: amino acid
(D) TOPOLOGY: linear

(ii) MOLECULE TYPE: peptide addition
(xi) SEQUENCE DESCRIPTION: SEQ ID NO: 65:

Ser-Pro-Ile-Arg-Pro-Arg-Pro-Arg-Pro-Arg
5 10

(2) INFORMATION FOR SEQ ID NO: 66:

(i) SEQUENCE CHARACTERISTICS:

(A) LENGTH: 9 amino acids
(B) TYPE: amino acid
(D) TOPOLOGY: linear

(ii) MOLECULE TYPE: peptide addition
(xi) SEQUENCE DESCRIPTION: SEQ ID NO: 66:

Ser-Pro-Ile-Arg-Lys-Ala-Trp-Trp-Pro
5

(2) INFORMATION FOR SEQ ID NO: 67:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 67:

Ala-Pro-Pro-Pro-Lys-Ala-Ser-Pro-Arg-Gln-Arg-Pro
5 10

(2) INFORMATION FOR SEQ ID NO:68:

(i) SEQUENCE CHARACTERISTICS:

(A) LENGTH: 15 amino acids
(B) TYPE: amino acid
(D) TOPOLOGY: linear

(ii) MOLECULE TYPE: peptide addition
(xi) SEQUENCE DESCRIPTION: SEQ ID NO: 68:

Ser-Pro-Ile-Arg-Pro-Arg-Pro-Ser-Pro-Ile-Arg-Pro-Arg-Pro-Arg
5 10 15

(2) INFORMATION FOR SEQ ID NO: 69:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 69:

Ser-Pro-Pro-Arg-Trp-Pro-Arg-Arg
5

(2) INFORMATfON FOR SEO ID NO: 70:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 70:

Ser-Pro-Pro-Arg-Trp-Pro-Arg-Trp
5

(2) INFORMATION FOR SEQ ID NO: 71:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 71:

Ser-Pro-Pro-Arg-Trp-Pro-Trp-Arg

5

(2) INFORMATION FOR SEQ ID NO: 72:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 72:

Ser-Pro-Pro-Trp-Arg-Pro-Arg-Arg

5

(2) INFORMATION FOR SEQ ID NO: 73:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 73:

Ser-Pro-Pro-Trp-Trp-Pro-Arg-Trp

5

(2) INFORMATION FOR SEQ ID NO: 74:

(i) SEQUENCE CHARACTERISTICS :

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 74:

Ser-Pro-Pro-Trp-Trp-Pro-Trp-Arg

5

(2) INFORMATION FOR SEQ ID NO: 75:

(i) SEQUENCE CHARACTERISTICS:

(A) LENGTH: 8 amino acids

(B) TYPE: amino acid
(D) TOPOLOGY: linear

(ii) MOLECULE TYPE: peptide addition
(xi) SEQUENCE DESCRIPTION: SEQ ID NO: 75:

Ser-Pro-Pro-Trp-Trp-Pro-Trp-Trp
5

(2) INFORMATION FOR SEQ ID NO: 76:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 76:

Ser-Pro-Pro-Trp-Pro-Arg-Pro-Arg-Pro
5

(2) DEFORMATION FOR SEQ ID NO: 77:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7887"

(xi) SEQUENCE DESCRIPTION: SEQ ID NO: 77:

gaatgacttg gttgagtact caccagtcac 30

(2) INFORMATION FOR SEQ ID NO: 78:

(i) SEQUENCE CHARACTERISTICS:

(A) LENGTH: 46 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 8932"

(xi) SEQUENCE DESCRIPTION: SEQ ID NO: 78:

gaactggata ggaaatttga agttcctgtt gaaagaaata aatgac 46

(2) INFORMATION FOR SEQ ID NO: 79:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7258"

(xi) SEQUENCE DESCRIPTION: SEQ ID NO: 79:

gaatgacttg gttgacgcgt caccagtcac 30

(2) INFORMATION FOR SEQ ID NO: 80:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7770"

(xi) SEQUENCE DESCRIPTION: SEQ ID NO: 80:

tctagcccag aatactggat caaatc 26

(2) INFORMATION FOR SEQ ID NO: 81:

(i) SEQUENCE CHARACTERISTICS:

(A) LENGTH: 54 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = *Primer 8479*

(xi) SEQUENCE DESCRIPTION: SEQ ID NO: 81:

gcgtggacgg ccttggctag ccctattcgt cctcgaccgg tctcgcagga tctg 54

(2) INFORMATION FOR SEQ ID NO: 82:

(i) SEQUENCE CHARACTERISTICS:

(A) LENGTH: 57 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Oligo 1"

(xi) SEQUENCE DESCRIPTION: SEQ ID NO: 82:

GCGTGGACGG CCTTGGCC86(T/A) 66(A/T) 58(T/A) 67(T/A) 66(T/A) 575 66(T/A) GAGGTC TCG CAGGATC TG

(2) INFORMATION FOR SEQ ID NO: 83:

(i) SEQUENCE CHARACTERISTICS:

(A) LENGTH: 93 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Oligo 2"

(xi) SEQUENCE DESCRIPTION: SEQ ID NO: 83:

GTCTCTGCGT GGACGGCCTT GGCGGCGCCA CCTCCA67(T/A) 66(T/A) 575 66(T/A) 67(T/A) 66(T/A) 575 66(T/A) (6/7)(7/8)(C/G) 57(C/G) C57 (5/7)5(C/G) CTGT TTAACCAGTT CAATCTC

(2) INFORMATION FOR SEQ ID NO: 84:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7887"

(xi) SEQUENCE DESCRIPTION: SEQ ID NO: 84:

gaatgacttg gttgagtact caccagtcac 30

(2) INFORMATION FOR SEQ ID NO: 85:

(i) SEQUENCE CHARACTERISTICS:

(A) LENGTH: 47 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 19473"

(xi) SEQUENCE DESCRIPTION: SEQ ID NO: 85:

accatacccc ggccgctcct cctaggcgtc ctcggcagct gggagcc 47

(2)INFORMATION FOR SEQ ID NO: 86:

(i) SEQUENCE CHARACTERISTICS:

(A) LENGTH: 59 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 21992"

(xi) SEQUENCE DESCRIPTION: SEQ ID NO: 86:

accatacccc ggccgctcct agccctccgc ggcggccgct gggagccatc gagaacggc 59

(2) INFORMATION FOR SEQ ID NO: 87:

(i) SEQUENCE CHARACTERISTICS:

(A) LENGTH: 21 base pain;
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 21994"

(xi) SEQUENCE DESCRIPTION: SEQ ID NO: 87:

accatacccc ggccgctcct agccctatac gtaagctggg agccatcgag aacggc 56

(2) INFORMATION FOR SEQ ID NO: 88:

(i) SEQUENCE CHARACTERISTICS:

(A) LENGTH: 56 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 9349"

(xi) SEQUENCE DESCRIPTION: SEQ ID NO: 88:

gagtcccaca tccgaaacat ctggatacaa ggagtaggag gaccttacgac gccgcg 56

(2) INFORMATION FOR SEQ ID NO: 89:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION; SEQ ID NO: 89:

Ser- Ala-Leu-Arg-Pro-Arg-Lys
5

(2) INFORMATION FOR SEQ ID NO: 90:

(i) SEQUENCE CHARACTERISTICS:

(A) LENGTH : 12 amino acids
(B) TYPE: amino acid
(D) TOPOLOGY: linear

(ii) MOLECULE TYPE: peptide addition
(xi) SEQUENCE DESCRIPTION: SEQ ID NO: 90:

Ala-Pro-Pro-Pro-Arg-Pro-Arg-Leu-Leu-Pro-Ile-Ser
5 10

(2) INFORMATION FOR SEQ ID NO: 91:

(i) SEQUENCE CHARACTERISTICS:

(A) LENGTH: 11 amino acids
(B) TYPE: amino acid
(D) TOPOLOGY: linear

(ii) MOLECULE TYPE: peptide addition
(xi) SEQUENCE DESCRIPTION: SEQ ID NO: 91:

Ala-Pro-Pro-Pro-Thr-Arg-Gln-Arg-Gln-Ser-Pro
5 10

(2) INFORMATION FOR SEQ ID NO: 92:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 92:

Ala-Pro-Pro-Pro-Arg-Thr-Ile-Pro-Arg-Ser-Ser-Pro
5 10

(2) INFORMATION FOR SEQ ID NO: 93:

(i) SEQUENCE CHARACTERISTICS:

(A) LENGTH: 288 amino acids
(B) TYPE: amino acid
(C) STRANDEDNESS: single
(D) TOPOLOGY: linear

(ii) MOLECULE TYPE: protein
(vi) ORIGINAL SOURCE:

(B) STRAIN: Peudomonas sp.

(xi) SEQUENCE DESCRIPTION: SEQ. ID NO: 93:

Phe Gly Ser Ser Asn Tyr Thr Lys Thr Gln Tyr Pro Ile Val Leu Thr
1 5 10 15

His Gly Met Leu Gly Phe Asp Ser Leu Leu Gly Val Asp Tyr Trp Tyr
20 25 30

Gly Ile Pro Ser Ala Leu Arg Lys Asp Gly Ala Thr Val Tyr Val Thr
35 40 45

Glu Val Ser Gln Leu Asp Thr Ser Glu Ala Arg Gly Glu Gln Leu Leu
50 55 60

Thr Gln Val Glu Glu Ile Val Ala Ile Ser Gly Lys Pro Lys Val Asn
65 70 75 80

Leu Phe Gly His Ser His Gly Gly Pro Thr Ile Arg Tyr Val Ala Ala
85 90 95

Val Arg Pro Asp Leu Val Ala Ser Val Thr Ser Ile Gly Ala Pro His
100 105 110

Lys Gly Ser Ala Thr Ala Asp Phe Ile Arg Gln Val Pro Glu Gly Ser
115 120 125

Ala Ser Glu Ala Ile Leu Ala Gly Ile Val Asn Gly Leu Gly Ala Leu
130 135 140

Ile Asn Phe Leu Ser Gly Ser Ser Ser Asp Thr Pro Gln Asn Ser Leu
145 150 155 160

Gly Thr Leu Glu Ser Leu Asn Ser Glu Gly Ala Ala Arg Phe Asn Ala
165 170 175

Arg Phe Pro Gln Gly Val Pro Thr Ser Ala Cys Gly Glu Gly Asp Tyr
180 185 190

Val Val Asn Gly Val Arg Tyr Tyr Ser Trp Ser Gly Thr Ser Pro Leu
195 200 205

Thr Asn Val Leu Asp Pro Ser Asp Leu Leu Leu Gly Ala Thr Ser Leu
210 215 220

Thr Phe Gly Phe Glu Ala Asn Asp Gly Leu Val Gly Arg Cys Ser Ser
225 230 235 240

Arg Leu Gly Met Val Ile Arg Asp Asn Tyr Arg Met Asn His Leu Asp
245 250 255

Glu Val Asn Gln Thr Phe Gly Leu Thr Ser Ile Phe Glu Thr Ser Pro
260 265 270

Val Ser Val Tyr Arg Gln Gln Ala Asn Arg Leu Lys Asn Ala Gly Leu
275 280 285